(19)

Europäisches
Patentamt
European
Patent Office
Office européen
des brevets

(11) **EP 3 635 114 B1**

(12) **EUROPEAN PATENT SPECIFICATION**

(45) Date of publication and mention
of the grant of the patent:
**06.11.2024   Bulletin 2024/45**

(21) Application number: **18813261.7**

(22) Date of filing: **07.06.2018**

(51) International Patent Classification (IPC):
**C12N 15/11** (2006.01)     **C12N 15/10** (2006.01)
**C12N 15/113** (2010.01)     **C12N 15/63** (2006.01)

(52) Cooperative Patent Classification (CPC):
(C-Sets available)
**C12N 15/11; C12N 15/1093; C12N 15/1096;**
C12N 2310/20; C12N 2320/12; C12N 2330/31

(Cont.)

(86) International application number:
**PCT/US2018/036563**

(87) International publication number:
**WO 2018/227025 (13.12.2018 Gazette 2018/50)**

(54) **CREATION AND USE OF GUIDE NUCLEIC ACIDS**

HERSTELLUNG UND VERWENDUNG VON GUIDE-NUKLEINSÄUREN

CRÉATION ET UTILISATION D'ACIDES NUCLÉIQUES GUIDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.06.2017   US 201762516619 P
21.08.2017   US 201762548036 P**

(43) Date of publication of application:
**15.04.2020   Bulletin 2020/16**

(73) Proprietor: **Arc Bio, LLC
Cambridge, Massachusetts 02139 (US)**

(72) Inventors:
• **GOURGUECHON, Stephane B.
San Mateo
California 94402 (US)**
• **CARPENTER, Meredith L.
San Mateo
California 94401 (US)**
• **RASMUSSEN, Morten
Mountain View
California 94040 (US)**
• **RADHAKRISHNAN, Srihari
Mountain View
California 94043 (US)**

• **ELMER, Anna Katharina
Palo Alto
California 94306 (US)**

(74) Representative: **Boult Wade Tennant LLP
Salisbury Square House
8 Salisbury Square
London EC4Y 8AP (GB)**

(56) References cited:
WO-A1-2016/077350     WO-A1-2016/130697
WO-A1-2017/031360     WO-A1-2017/031360
WO-A1-2017/062723     WO-A1-2017/081097
WO-A1-2017/081097     WO-A1-2017/100343
WO-A2-2009/133466     US-A1- 2010 159 561
US-A1- 2014 030 704     US-A1- 2015 094 212
US-A1- 2015 211 002     US-A1- 2015 284 789
US-A1- 2016 115 530     US-A1- 2016 237 488
US-A1- 2016 354 752     US-A1- 2016 362 680
US-A1- 2017 121 694     US-B2- 9 102 944

• **LANE ANDREW B ET AL: "Enzymatically
Generated CRISPR Libraries for Genome
Labeling and Screening", DEVELOPMENTAL
CELL, CELL PRESS, US, vol. 34, no. 3, 23 July
2015 (2015-07-23), pages 373 - 378, XP029258679,
ISSN: 1534-5807, DOI:
10.1016/J.DEVCEL.2015.06.003**

EP 3 635 114 B1

- **TOBIAS SCHMIDT ET AL: "Synthesis of an arrayed sgRNA library targeting the human genome", SCIENTIFIC REPORTS, vol. 5, no. 1, 8 October 2015 (2015-10-08), XP055406733, DOI: 10.1038/srep14987**

(52) Cooperative Patent Classification (CPC): (Cont.)

C-Sets
**C12N 15/1093, C12Q 2521/301, C12Q 2521/501, C12Q 2525/113, C12Q 2525/186, C12Q 2525/191, C12Q 2533/101, C12Q 2535/122, C12Q 2539/101**

**Description**

**CROSS-REFERENCE**

This application claims the benefit of priority to US Provisional Patent Application Serial No. 62/516,619 filed on June 7, 2017 and US Provisional Patent Application Serial No. 62/548,036 filed on August 21, 2017.

**BACKGROUND**

Human clinical DNA samples and sample libraries such as cDNA libraries derived from RNA contain highly abundant sequences that have little informative value and increase the cost of sequencing. While methods have been developed to deplete these unwanted sequences (e.g., via hybridization capture), these methods are often time-consuming and can be inefficient.

Although a guide nucleic acid (gNA) mediated nuclease systems (such as guide RNA (gRNA)-mediated Cas systems) can efficiently deplete any target DNA, targeted depletion of very high numbers of unique DNA molecules is not feasible. For example, a sequencing library derived from human blood may contain >99% human genomic DNA. Using a gRNA-mediated Cas9 system-based method to deplete this genomic DNA to detect an infectious agent circulating in the human blood would require extremely high numbers of gRNAs (about 10-100 million gRNAs), in order to ensure that a gRNA will be present every 30-50 base pairs (bp), and that no target DNA will be missed. Very large numbers of gRNAs can be predicted computationally and then synthesized chemically, but at a prohibitively expensive cost.

Therefore, there is a need in the art to provide a cost-effective method of converting any DNA into a gNA (e.g., gRNA) library to enable, for example, genome-wide depletion of unwanted DNA sequences from those of interest, without prior knowledge about their sequences. Provided herein are methods and compositions that address this need. WO2017/081097 describes a method for obtaining a CRISPR-Cas system sgRNA library. WO2009/133466 describes methods and compositions for asymmetrically tagging a nucleic acid fragment using asymmetric adapters. WO2017/031360 describes methods and compositions for the capture of nucleic acids using a nucleic acid-guided nuclease-based system.

**SUMMARY**

The invention is defined in the appended claims. Provided herein are collections of guide nucleic acids, methods of making the same, and methods of using the same.

The invention provides methods of making a collection of nucleic acids, comprising:

(a) obtaining target nucleic acids, each comprising a PAM site of a nucleic acid-guided nuclease;
(b) hybridizing first primers to the PAM sites of the target nucleic acids, wherein the first primers comprise (i) a MAP site that is complementary to the PAM site, (ii) a complementary recognition site that is complementary to a recognition site of the nucleic acid guided nuclease, and (iii) a complementary promoter site that is complementary to a promoter site; (c) extending the first primers using the target nucleic acids as template, thereby producing first extension products comprising sequence of the first primer and sequence complementary to the target nucleic acids; (d) hybridizing second primers to the first extension products; and (e) extending the second primers using the first extension products as template, thereby producing second extension products comprising the PAM site, the recognition site, and the promoter site.

Although not encompassed by the wording of the claims the following additional methods are provided herein as they are considered useful for understanding the invention.

Provided herein are methods of making a collection of nucleic acids, comprising: (a) obtaining target nucleic acids, each comprising a PAM site of a nucleic acid-guided nuclease; (b) hybridizing primers to the PAM sites of the target nucleic acids, wherein the primers comprise (i) a MAP site that is complementary to the PAM site, (ii) a complementary recognition site that is complementary to a recognition site of the nucleic acid guided nuclease, and (iii) a complementary promoter site that is complementary to a promoter site; (c) extending the primers using the target nucleic acids as template, thereby producing extension products comprising the PAM site, the recognition site, and the promoter site; (d) nicking the target nucleic acids; and (e) digesting the nicked target nucleic acids.

Provided herein are methods of making a collection of nucleic acids, comprising: (a) obtaining target nucleic acids, each comprising a PAM site of a nucleic acid-guided nuclease; (b) ligating first loop adapters to both ends of the target nucleic acids, wherein the first loop adapters comprise a promoter site; (c) cleaving the target nucleic acids at the PAM site, thereby producing DNA cleavage products each comprising one of the first loop adapters at a first end and a PAM site at a second end; (d) ligating second loop adapters to the second end of the cleavage products, wherein the second loop adapters comprise a complementary stem loop sequence that is complementary to a stem loop sequence of the nucleic acid-guided nuclease; and (e) amplifying the cleavage products, thereby producing amplification products comprising the promoter site, a recognition site, and the stem loop sequence, wherein the recognition site comprises a sequence that was adjacent to the PAM site in one of the target nucleic acids.

Provided herein are methods of making a collection of nucleic acids, comprising: (a) obtaining sequence reads of target nucleic acids; (b) mapping the sequence reads to at least one reference sequence; (c) determining abundance values of the sequence reads; (d) identifying recognition sites from the sequence reads, wherein the recognition sites are adjacent to PAM sites of a nucleic acid-guided nuclease; and (e) sorting the recognition sites based on the abundance values.

Provided herein are methods of making a collection of guide nucleic acids, comprising: (a) obtaining sequence reads of target nucleic acids; (b) determining the most frequent recognition site from the sequence reads, wherein recognition sites are adjacent to PAM sites of a nucleic acid-guided nuclease; (c) determining the next most frequent recognition site from the sequence reads; and (d) repeating step c until a condition is met, wherein the condition is selected from the group consisting of (i) a set number of recognition sites are determined, (ii) no further recognition sites can be determined, (iii) a set percentage of the target nucleic acids is covered by the recognition sites, and (iv) cleavage of the target nucleic acids at or near the recognition sites yields a maximum fragment size below a set size.

Provided herein are compositions comprising a collection of guide nucleic acids,

wherein each guide nucleic acid comprises a recognition site and a stem loop sequence of a nucleic acid-guided nuclease, wherein each recognition site is complementary to a target site of a target nucleic acid that is adjacent to a PAM site of the nucleic acid-guided nuclease, and

wherein the target sites to which the recognition sites of the collection of guide nucleic acids are complementary are distributed within the target nucleic acids at an average spacing of less than about 10,000 base pairs.

Provided herein are methods of depleting target nucleic acids, comprising: (a) obtaining nucleic acids comprising target nucleic acids and non-target nucleic acids; (b) contacting the nucleic acids with nucleic acid-guided nickase protein-gNA complex, such that the target nucleic acids are nicked at nick sites, and wherein the gNA comprises a 5' stem-loop sequence and a 3' targeting sequence; (c) conducting nick translation at the nick sites, wherein the nick translation is conducted with labeled nucleotides; (d) capturing the target nucleic acids with the labeled nucleotides; and (e) separating the target nucleic acids from the non-target nucleic acids.

Provided herein are methods of depleting target nucleic acids, comprising: (a) obtaining nucleic acids comprising target nucleic acids and non-target nucleic acids, wherein the nucleic acids comprise hairpin loops at a first end; (b) hybridizing loop adapters to a second end of the nucleic acids; (c) contacting the nucleic acids with nucleic acid-guided nickase proteins, such that the target nucleic acids are nicked; and (d) digesting nicked target nucleic acids.

Provided herein are methods of preparing a sequencing library, comprising: (a) providing a DNA molecule comprising a site of interest obtained after undergoing a depletion or capture method of the disclosure; (b) blocking 3' ends of the DNA molecule such that the 3' ends cannot be extended by a polymerase; (c) hybridizing a first primer to the DNA molecule; (d) extending the first primer to yield an extension product comprising sequence of the first primer and sequence of the site of interest; (e) hybridizing a second primer to the extension product; and (f) amplifying the extension product using the second primer.

Provided herein are methods of preparing a sequencing library, comprising: (a) providing an RNA molecule resulting from a gNA depletion or capture method; (b) attaching a first hybridization site to the RNA molecule; (c) hybridizing a first oligonucleotide to the first hybridization site; (d) reverse transcribing at least a portion of the RNA molecule using the first oligonucleotide as a primer, thereby generating cDNA; (e) hybridizing a second oligonucleotide to a tail of the cDNA; and (f) amplifying the cDNA using the second oligonucleotide and/or the first oligonucleotide as a primer.

Provided herein are methods of making a collection of nucleic acids, comprising: (a) digesting a DNA sample with a restriction endonuclease to produce a collection of DNA fragments; (b) treating the collection of DNA fragments with a nuclease; (c) ligating a first adapter to the collection of DNA fragments to produce a collection of first-adapter DNA fragments; wherein the sequence encoding the first adapter comprises an MmeI restriction site and a FokI restriction site; and wherein the MmeI site is positioned between the FokI site and the DNA fragment following ligation; (d) digesting the collection first-adapter DNA fragments first with MmeI and second with FokI to produce a collection of N20 DNA fragments; and (e) ligating a second adapter to the collection of N20 DNA fragments; wherein the sequence encoding the second adapter comprises a promoter sequence and a nucleic acid guided nuclease system protein binding sequence; and wherein the nucleic acid guided nuclease system protein binding sequence is positioned between the N20 sequence and the promoter following ligation of the second adapter.

Provided herein are methods of making a collection of nucleic acids, comprising: (a) replacing at least two consecutive adenosines in a DNA sample with inosines; (b) treating the DNA sample with human alkyladenine DNA Glycosylase (hAAG); (c) treating the DNA sample with an endonuclease to produce a collection of DNA fragments; (d) ligating a first adapter to the collection of DNA fragments to generate a collection of first-adapter DNA fragments in a first ligation step; wherein the first adapter comprises a double stranded DNA molecule and a single stranded DNA overhang of 5' NAA 3' at the 5' end of the double stranded DNA molecule; wherein the first adapter comprises an MmeI site and a FokI site; and wherein the MmeI site is positioned between the FokI site and the DNA fragment following ligation of the first adapter; (e) digesting the collection first-adapter ligated fragments first with MmeI and second with FokI to produce a collection of N20 DNA fragments; and (f) ligating a second adapter to the collection of N20 DNA fragments in a second ligation step; wherein the sequence encoding the second adapter comprises a promoter sequence and a nucleic acid guided nuclease system protein binding sequence; and wherein the nucleic acid guided nuclease system protein binding sequence is positioned between the N20 sequence and the promoter following ligation of the second adapter.

Provided herein are methods of making a collection of nucleic acids, comprising: (a) replacing at least one thymidine in a DNA sample with a uracil to produce a DNA sample comprising at least one base pair mismatch; (b) excising the at least one uracil with at least one DNA repair enzyme to produce a DNA sample with at least one single stranded region of at least one base pair; (c) treating the DNA sample with a nuclease to produce a collection of DNA fragments; (d) ligating to the collection of DNA fragments a first adapter in a first ligation step to produce a collection of first-adapter DNA fragments; wherein the first adapter comprises an MmeI site and a FokI site; wherein the MmeI site is positioned between the FokI site and the DNA fragment following ligation; (e) digesting the collection of first-adapter DNA fragments first with MmeI and second with FokI to produce a collection of N20 DNA fragments; and (f) ligating a second adapter to the collection of N20 DNA fragments in a second ligation step; wherein the sequence encoding the second adapter comprises a promoter sequence and a nucleic acid guided nuclease system protein binding sequence; and wherein the nucleic acid guided nuclease system protein binding sequence is positioned between the N20 sequence and the promoter following ligation.

Provided herein are methods of making a collection of nucleic acids, comprising: (a) randomly fragmenting a DNA sample to produce a collection of DNA fragments; (b) ligating a first adapter to the collection of DNA fragments in a first ligation step; wherein the first adapter is comprises a double stranded DNA molecule and a single stranded DNA overhang of 5' NAA 3' at the 5' end of the double stranded DNA molecule; wherein the first adapter comprises a FokI site and a MmeI site; and wherein the MmeI site is positioned between the FokI site and the DNA fragment following ligation; (c) digesting the collection first-adapter ligated fragments first with MmeI and second with FokI to produce a collection of N20 DNA fragments; and (d) ligating a second adapter to the collection of N20 DNA fragments in a second ligation step; wherein the sequence encoding the second adapter comprises a promoter sequence and a nucleic acid guided nuclease system protein binding sequence; and wherein the nucleic acid guided nuclease system protein binding sequence is positioned between the N20 sequence and the promoter following ligation.

Provided herein are methods of making a collection of nucleic acids, comprising: (a) randomly shearing a DNA sample to produce a collection of DNA fragments; (b) methylating the DNA fragments with a methylase; (c) end repairing the collection of DNA fragments to produce a collection of blunt ended DNA fragments; (d) ligating a first adapter to the collection of blunt ended DNA fragments to produce a collection of first-adapter DNA fragments in a first ligation step; wherein the first adapter comprises, 5' to 3', an NtBstNBI restriction site, a modified cleavage resistant bond in the phosphate backbone of the first adapter, and a sequence complementary to a PAM sequence; (e) digesting the first-adapter DNA fragments with a restriction enzyme and NtBstNBI; (f) ligating a second adapter to the digested first adapter DNA fragments in a second ligation step to produce a collection of second-adapter DNA fragments; wherein the second adapter comprises a FokI site and a MmeI site; and wherein the MmeI site is positioned between the FokI site and the DNA fragment following ligation; (g) digesting the collection second-adapter ligated fragments first with MmeI and second with FokI to produce a collection of N20 DNA fragments; and (h) ligating a third adapter to the collection of N20 DNA fragments in a third ligation reaction; wherein the sequence encoding the third adapter comprises a sequence encoding a promoter sequence and a nucleic acid guided nuclease system protein binding sequence; and wherein the nucleic acid guided nuclease system protein binding sequence is positioned between the N20 sequence and the promoter following ligation.

Provided herein are methods of making a collection of nucleic acids, comprising: (a) randomly shearing a DNA sample to produce a collection of DNA fragments; (b) end repairing the collection of DNA fragments to produce blunt ended DNA fragments; (c) ligating a first adapter to the blunt ended DNA fragments to produce a collection of first-adapter DNA fragments in a first ligation step; wherein the first adapter comprises, 5' to 3', an Nt.BstNBI restriction site and a sequence complementary to a PAM sequence; (d) nicking the first-adapter DNA fragments with Nt.BstNBI; (e) degrading the top strand of the first-adapter DNA fragments from the nick to the 5' end in a 3' to 5' direction; (f) ligating a second adapter to the degraded first-adapter DNA fragments to produce a collection second-adapter DNA fragments in a second ligation step; wherein the second adapter comprises, in a 5' to 3' orientation, an MlyI sequence, a sequence complementary to the PAM sequence and the PAM sequence; (g) digesting the second-adapter fragments with MlyI; (h) ligating a third adapter to the MlyI digested second-adapter ligated fragments in a third ligation step to produce a collection of third-adapter DNA fragments; wherein the third adapter comprises a FokI site and a MmeI site; and wherein the MmeI site is positioned between the FokI site and the DNA fragment following ligation; (i) digesting the collection of third-adapter DNA fragments first with MmeI and second with FokI to produce a collection of N20 DNA fragments; and (j) ligating a fourth adapter to the collection of N20 DNA fragments in a fourth ligation reaction; wherein the sequence encoding the fourth adapter comprises a promoter sequence and a nucleic acid guided nuclease system protein binding sequence; and wherein the nucleic acid guided nuclease system protein binding sequence is positioned between the N20 sequence and the promoter following ligation.

Provided herein are methods of making a collection of nucleic acids, comprising: (a) randomly shearing a DNA sample to produce a collection of DNA fragments; (b) ligating a circular adapter to the collection of DNA fragments in a first ligation reaction to produce a collection of circular-adapter DNA fragments; wherein the circular adapter comprises a sequence complementary to a PAM sequence; (c) methylating the collection of circular-adapter DNA fragments with a methylase; (d) digesting the collection of circular-adapter DNA fragments with an exonuclease; (e) digesting the collection of circular-adapter DNA fragments with a restriction enzyme; (f) ligating a second adapter to the collection of circular-adapter DNA fragments to produce a collection of second-adapter DNA fragments in a second ligation reaction; wherein the second adapter comprises, from 5' to 3', a sequence complementary to a PAM site, a PAM site and an MlyI site; (g) PCR amplifying the collection of second-adapter DNA fragments; wherein PCR primers comprise a sequence of the second adapter or a sequence complementary to a sequence of the second adapter to produce a collection of PCR amplified second-adapter DNA fragments; (h) digesting the collection of PCR amplified second-adapter DNA fragments with MlyI; (i) ligating a third adapter to the collection of PCR amplified second-adapter DNA fragments to produce a collection of third-adapter DNA fragments in a third ligation reaction; wherein the third adapter comprises a FokI site and a MmeI site; and wherein the MmeI site is positioned between the FokI site and the DNA fragment following ligation; (j) digesting the collection third-adapter ligated fragments first with MmeI and second with FokI to produce a collection of N20 DNA fragments; and (k) ligating a fourth adapter to the collection of N20 DNA fragments in a fourth ligation reaction; wherein the sequence encoding the fourth adapter comprises a promoter sequence and a nucleic acid guided nuclease system protein binding sequence; and wherein the nucleic acid guided nuclease system protein binding sequence is positioned between the N20 sequence and the promoter following ligation.

In some embodiments, the target nucleic acids comprise genomic DNA or cDNA. In some embodiments, the target nucleic acids comprise human DNA. In some embodiments, the target nucleic acids comprise eukaryotic DNA.

## BRIEF DESCRIPTION OF THE DRAWINGS

FIG. 1 illustrates an exemplary scheme for producing a collection of gRNAs (a gRNA library) from genomic DNA.

FIG. 2 illustrates another exemplary scheme for producing a collection of gRNAs (a gRNA library) from genomic DNA.

FIG. 3 illustrates an exemplary scheme for nicking of DNA and subsequent treatment with polymerase to generate blunt ends.

FIG. 4 illustrates an exemplary scheme for sequential production of a library of gNAs using three adapters.

FIG. 5 illustrates how an exemplary scheme for sequential production of a library of gNAs using one adapter and one oligo.

FIG. 6 illustrates how an exemplary scheme for generation of a large pool of DNA fragments with blunt ends using Nicking Enzyme Mediated DNA Amplification (NEMDA).

FIG. 7 illustrates an exemplary scheme for generation of nucleic acid fragments.

FIG. 8A illustrates an exemplary scheme for constructing a guide nucleic acid library from input nucleic acids.

FIG. 8B illustrates an exemplary scheme for constructing a guide nucleic acid library from input nucleic acids.

FIG. 8C illustrates an exemplary scheme for constructing a guide nucleic acid library from input nucleic acids.

FIG. 8D illustrates an exemplary scheme for constructing a guide nucleic acid library from input nucleic acids.

**FIG. 9A** and **FIG. 9B** illustrate an exemplary scheme for constructing a guide nucleic acid library from input nucleic acids.

**FIG. 10** illustrates an exemplary scheme for designing collections of guide nucleic acids.

**FIG. 11** illustrates an exemplary scheme for designing collections of guide nucleic acids.

**FIG. 12** illustrates an exemplary scheme for depleting, partitioning, or capturing targeted nucleic acids.

**FIG. 13** illustrates an exemplary schematic of a strand-switching method.

**FIG. 14** illustrates an exemplary scheme for the library generation and enrichment in a single workflow.

**FIG. 15** illustrates an exemplary scheme for a guide nucleic acid library from a DNA source that has been cut with either MseI or MluCI and treated with mung bean nuclease to degrade single stranded overhangs.

**FIG. 16A** and **FIG. 16B** illustrate an exemplary scheme for a guide nucleic acid library from a DNA source in which adenosines have been replaced with inosines.

**FIG. 17A** and **FIG. 17B** illustrate an exemplary scheme for a guide nucleic acid library from a DNA source in which thymidines have been replaced with uracils.

**FIG. 18** illustrates an exemplary scheme for a guide nucleic acid library from a DNA source that has been randomly fragmented with a non-specific nickase and T7 endonuclease I (fragmentase).

**FIG. 19A** and **FIG. 19B** illustrate an exemplary scheme for a guide nucleic acid library from a DNA source that has been randomly sheared and methylated.

**FIG. 20A, FIG. 20B** and **FIG. 20C** illustrate an exemplary scheme for a guide nucleic acid library from a randomly sheared DNA source.

**FIG. 21A** and **FIG. 21B** illustrate an exemplary scheme for a guide nucleic acid library from a randomly sheared DNA source using the ligation of a circular adapter.

**FIG. 22A, FIG. 22B, FIG. 22C** and **FIG. 22D** illustrate an exemplary scheme for a guide nucleic acid library from a randomly sheared DNA source that has been blunt end repaired.

**FIG. 23A, FIG. 23B** and **FIG. 23C** illustrate an exemplary scheme for a guide nucleic acid library from a randomly sheared DNA source that has been blunt end repaired.

**FIG. 24** illustrates an exemplary scheme for a guide nucleic acid library from a randomly sheared DNA source that has been circularized.

## DETAILED DESCRIPTION

There is a need in the art for a scalable, low-cost approach to generate large numbers of diverse guide nucleic acids (gNAs) (e.g., gRNAs, gDNAs) for a variety of downstream applications.

Unless defined otherwise herein, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

Numeric ranges are inclusive of the numbers defining the range.

For purposes of interpreting this specification, the following definitions will apply and whenever appropriate, terms used in the singular will also include the plural and vice versa. In the event that any definition set forth below conflicts with any document, the definition set forth shall control.

As used herein, the singular form "a", "an", and "the" includes plural references unless indicated otherwise.

It is understood that aspects and embodiments of the invention described herein include "comprising," "consisting," and "consisting essentially of' aspects and embodiments.

The term "about" as used herein refers to the usual error range for the respective value readily known to the skilled person in this technical field. Reference to "about" a value or parameter herein includes (and describes) embodiments that are directed to that value or parameter per se.

The term "nucleic acid," as used herein, refers to a molecule comprising one or more nucleic acid subunits. A nucleic acid can include one or more subunits selected from adenosine (A), cytosine (C), guanine (G), thymine (T) and uracil (U), and modified versions of the same. A nucleic acid comprises deoxyribonucleic acid (DNA), ribonucleic acid (RNA), combinations, or derivatives thereof. A nucleic acid may be single-stranded and/or double-stranded.

The nucleic acids comprise "nucleotides", which, as used herein, is intended to include those moieties that contain purine and pyrimidine bases, and modified versions of the same. Such modifications include methylated purines or pyrimidines, acylated purines or pyrimidines, alkylated riboses or other heterocycles. In addition, the term "nucleotide" or "polynucleotide" includes those moieties that contain hapten or fluorescent labels and may contain not only conventional ribose and deoxyribose sugars, but other sugars as well. Modified nucleosides, nucleotides or polynucleotides also include modifications on the sugar moiety, e.g., wherein one or more of the hydroxyl groups are replaced with halogen atoms or aliphatic groups, or are functionalized as ethers, amines, or the like.

The term "nucleic acids" and "polynucleotides" are used interchangeably herein. Polynucleotide is used to describe a nucleic acid polymer of any length, e.g., greater than about 2 bases, greater than about 10 bases, greater than about 100 bases, greater than about 500 bases, greater than 1000 bases, up to about 10,000 or more bases composed of nucleotides, e.g., deoxyribonucleotides or ribonucleotides, and may be produced enzymatically or synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine and thymine (G, C, A and T, respectively). DNA and RNA have a deoxyribose and ribose sugar backbones, respectively, whereas PNA's backbone is composed of repeating N-(2-aminoethyl)-glycine units linked by peptide bonds. In PNA various purine and pyrimidine bases are linked to the backbone by methylene carbonyl bonds. A locked nucleic acid (LNA), often referred to as inaccessible RNA, is a modified RNA nucleotide. The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. The term "unstructured nucleic acid," or "UNA," is a nucleic acid containing non-natural nucleotides that bind to each other with reduced stability. For example, an unstructured nucleic acid may contain a G' residue and a C' residue, where these residues correspond to non-naturally occurring forms, i.e., analogs, of G and C that base pair with each other with reduced stability, but retain an ability to base pair with naturally occurring C and G residues, respectively. Unstructured nucleic acid is described in US20050233340.

The term "oligonucleotide" as used herein denotes a single-stranded multimer of nucleotides.

Unless otherwise indicated, nucleic acids are written left to right in 5' to 3' orientation; amino acid sequences are written left to right in amino to carboxy orientation, respectively.

The term "cleaving," as used herein, refers to a reaction that breaks the phosphodiester bonds between two adjacent nucleotides in both strands of a double-stranded DNA molecule, thereby resulting in a double-stranded break in the DNA molecule.

The term "nicking" as used herein, refers to a reaction that breaks the phosphodiester bond between two adjacent nucleotides in only one strand of a double-stranded DNA molecule, thereby resulting in a break in one strand of the DNA molecule.

The term "cleavage site, as used herein, refers to the site at which a double-stranded DNA molecule has been cleaved.

The "nucleic acid-guided nuclease-gNA complex" refers to a complex comprising a nucleic acid-guided nuclease protein and a guide nucleic acid (gNA, for example a gRNA or a gDNA). For example the "Cas9-gRNA complex" refers to a complex comprising a Cas9 protein and a guide RNA (gRNA). The nucleic acid-guided nuclease may be any type of nucleic acid-guided nuclease, including but not limited to wild type nucleic acid-guided nuclease, a catalytically dead nucleic acid-guided nuclease, or a nucleic acid-guided nuclease-nickase.

The term "nucleic acid-guided nuclease-associated guide NA" refers to a guide nucleic acid (guide NA). The nucleic acid-guided nuclease-associated guide NA may exist as an isolated nucleic acid, or as part of a nucleic acid-guided nuclease-gNA complex, for example a Cas9-gRNA complex.

The terms "capture" and "enrichment" are used interchangeably herein, and refer to the process of selectively isolating a nucleic acid region containing: sequences of interest, targeted sites of interest, sequences not of interest, or targeted sites not of interest.

The term "hybridization" refers to the process by which a strand of nucleic acid joins with a complementary strand through base pairing as known in the art. A nucleic acid is considered to be "selectively hybridizable" to a reference nucleic acid sequence if the two sequences specifically hybridize to one another under moderate to high stringency hybridization and wash conditions. Moderate and high stringency hybridization conditions are known (see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.). One example of high stringency conditions includes hybridization at about 42 °C in 50% formamide, 5X SSC, 5X Denhardt's solution, 0.5% SDS and 100 $\mu$g/ml denatured carrier DNA followed by washing two times in 2X SSC and 0.5% SDS at room temperature and two additional times in 0.1 X SSC and 0.5% SDS at 42 oC.

The term "duplex," or "duplexed," as used herein, describes two complementary polynucleotides that are base-paired, i.e., hybridized together.

The term "amplifying" as used herein refers to generating one or more copies of a target nucleic acid, using the target nucleic acid as a template.

The term "genomic region," as used herein, refers to a region of a genome, e.g., an animal or plant genome such as the genome of a human, monkey, rat, fish or insect or plant. In certain cases, an oligonucleotide used in the method described herein may be designed using a reference genomic region, i.e., a genomic region of known nucleotide sequence, e.g., a chromosomal region whose sequence is deposited at NCBI's Genbank database or other databases, for example.

The term "genomic sequence," as used herein, refers to a sequence that occurs in a genome. Because RNAs are transcribed from a genome, this term encompasses sequence that exist in the nuclear genome of an organism, as well as sequences that are present in a cDNA copy of an RNA (e.g., an mRNA) transcribed from such a genome.

The term "genomic fragment," as used herein, refers to a region of a genome, e.g., an animal or plant genome such as the genome of a human, monkey, rat, fish or insect or plant. A genomic fragment may be an entire chromosome, or a fragment of a chromosome. A genomic fragment may be adapter ligated (in which case it has an adapter ligated to one or both ends of the fragment, or to at least the 5' end of a molecule), or may not be adapter ligated.

In certain cases, an oligonucleotide used in the method described herein may be designed using a reference genomic region, i.e., a genomic region of known nucleotide sequence, e.g., a chromosomal region whose sequence is deposited at NCBI's Genbank database or other databases, for example. Such an oligonucleotide may be employed in an assay that uses a sample containing a test genome, where the test genome contains a binding site for the oligonucleotide.

The term "ligating," as used herein, refers to the enzymatically catalyzed joining of the terminal nucleotide at the 5' end of a first DNA molecule to the terminal nucleotide at the 3' end of a second DNA molecule.

If two nucleic acids are "complementary," each base of one of the nucleic acids base pairs with corresponding nucleotides in the other nucleic acid. The term "complementary" and "perfectly complementary" are used synonymously herein.

The term "separating," as used herein, refers to physical separation of two elements (e.g., by size or affinity, etc.) as well as degradation of one element, leaving the other intact. For example, size exclusion can be employed to separate nucleic acids, including cleaved targeted sequences.

In a cell, DNA usually exists in a double-stranded form, and as such, has two complementary strands of nucleic acid referred to herein as the "top" and "bottom" strands. In certain cases, complementary strands of a chromosomal region may be referred to as "plus" and "minus" strands, the "first" and "second" strands, the "coding" and "noncoding" strands, the "Watson" and "Crick" strands or the "sense" and "antisense" strands. The assignment of a strand as being a top or bottom strand is arbitrary and does not imply any particular orientation, function or structure. Until they become covalently linked, the first and second strands are distinct molecules. For ease of description, the "top" and "bottom" strands of a double-stranded nucleic acid in which the top and bottom strands have been covalently linked will still be described as the "top" and "bottom" strands. In other words, for the purposes of this disclosure, the top and bottom strands of a double-stranded DNA do not need to be separated molecules. The nucleotide sequences of the first strand of several exemplary mammalian chromosomal regions (e.g., BACs, assemblies, chromosomes, etc.) is known, and may be found in NCBI's Genbank database, for example.

The term "top strand," as used herein, refers to either strand of a nucleic acid but not both strands of a nucleic acid. When an oligonucleotide or a primer binds or anneals "only to a top strand," it binds to only one strand but not the other. The term "bottom strand," as used herein, refers to the strand that is complementary to the "top strand." When an oligonucleotide binds or anneals "only to one strand," it binds to only one strand, e.g., the first or second strand, but not the other strand. If an oligonucleotide binds or anneals to both strands of a double-stranded DNA, the oligonucleotide may have two regions, a first region that hybridizes with the top strand of the double-stranded DNA, and a second region that hybridizes with the bottom strand of the double-stranded DNA.

The term "double-stranded DNA molecule" refers to both double-stranded DNA molecules in which the top and bottom strands are not covalently linked, as well as double-stranded DNA molecules in which the top and bottom stands are covalently linked. The top and bottom strands of a double-stranded DNA are base paired with one other by Watson-Crick interactions.

The term "denaturing," as used herein, refers to the separation of at least a portion of the base pairs of a nucleic acid duplex by placing the duplex in suitable denaturing conditions. Denaturing conditions are well known in the art. In one embodiment, in order to denature a nucleic acid duplex, the duplex may be exposed to a temperature that is above the Tm of the duplex, thereby releasing one strand of the duplex from the other. In certain embodiments, a nucleic acid may be denatured by exposing it to a temperature of at least 90 oC for a suitable amount of time (e.g., at least 30 seconds, up to 30 mins). In certain embodiments, fully denaturing conditions may be used to completely separate the base pairs of the duplex. In other embodiments, partially denaturing conditions (e.g., with a lower temperature than fully denaturing conditions) may be used to separate the base pairs of certain parts of the duplex (e.g., regions enriched for A-T base pairs may separate while regions enriched for G-C base pairs may remain paired). Nucleic acid may also be denatured chemically (e.g., using urea or NaOH).

The term "genotyping," as used herein, refers to any type of analysis of a nucleic acid sequence, and includes sequencing, polymorphism (SNP) analysis, and analysis to identify rearrangements.

The term "sequencing," as used herein, refers to a method by which the identity of consecutive nucleotides of a polynucleotide are obtained.

The term "next-generation sequencing" refers to the so-called parallelized sequencing-by-synthesis or sequencing-by-ligation platforms, for example, those currently employed by Illumina, Life Technologies, and Roche, etc. Next-generation sequencing methods may also include nanopore sequencing methods or electronic-detection based methods such as Ion Torrent technology commercialized by Life Technologies.

The term "complementary DNA" or cDNA refers to a double-stranded DNA sample that was produced from an RNA sample by reverse transcription of RNA (using primers such as random hexamers or oligo-dT primers) followed by second-strand synthesis by digestion of the RNA with RNaseH and synthesis by DNA polymerase.

The term "RNA promoter adapter" is an adapter that contains a promoter for a bacteriophage RNA polymerase, e.g., the RNA polymerase from bacteriophage T3, T7, SP6 or the like.

Other definitions of terms may appear throughout the specification.

For any of the structural and functional characteristics described herein, methods of determining these characteristics are known in the art.

## *Guide Nucleic Acids (gNAs)*

Provided herein are guide nucleic acids (gNAs) derivable from any nucleic acid source. The gNAs can be guide RNAs (gRNAs) or guide DNAs (gDNAs). The nucleic acid source can be DNA or RNA. Provided herein are methods to generate gNAs from any source nucleic acid, including DNA from a single organism, or mixtures of DNA from multiple organisms, or mixtures of DNA from multiple species, or DNA from clinical samples, or DNA from forensic samples, or DNA from environmental samples, or DNA from metagenomic DNA samples (for example a sample that contains more than one species of organism). Examples of any source DNA include, but are not limited to any genome, any genome fragment, cDNA, synthetic DNA, or a DNA collection (e.g. a SNP collection, DNA libraries). The gNAs provided herein can be used for genome-wide applications.

In some embodiments, the gNAs are derived from genomic sequences (e.g., genomic DNA). In some embodiments, the gNAs are derived from mammalian genomic sequences. In some embodiments, the gNAs are derived from eukaryotic genomic sequences. In some embodiments, the gNAs are derived from prokaryotic genomic sequences. In some embodiments, the gNAs are derived from viral genomic sequences. In some embodiments, the gNAs are derived from bacterial genomic sequences. In some embodiments, the gNAs are derived from plant genomic sequences. In some embodiments, the gNAs are derived from microbial genomic sequences. In some embodiments, the gNAs are derived from genomic sequences from a parasite, for example a eukaryotic parasite.

In some embodiments, the gNAs are derived from repetitive DNA. In some embodiments, the gNAs are derived from abundant DNA. In some embodiments, the gNAs are derived from mitochondrial DNA. In some embodiments, the gNAs are derived from ribosomal DNA. In some embodiments, the gNAs are derived from centromeric DNA. In some embodiments, the gNAs are derived from DNA comprising Alu elements (Alu DNA). In some embodiments, the gNAs are derived from DNA comprising long interspersed nuclear elements (LINE DNA). In some embodiments, the gNAs are derived from DNA comprising short interspersed nuclear elements (SINE DNA). In some embodiments the abundant DNA comprises ribosomal DNA. In some embodiments, the abundant DNA comprises host DNA (e.g., host genomic DNA or all host DNA). In an example, the gNAs can be derived from host DNA (e.g., human, animal, plant) for the depletion of host DNA to allow for easier analysis of other DNA that is present (e.g., bacterial, viral, or other metagenomic DNA). In another example, the gNAs can be derived from the one or more most abundant types (e.g., species) in a mixed sample, such as the one or more most abundant bacteria species in a metagenomic sample. The one or more most abundant types (e.g., species) can comprise the two, three, four, five, six, seven, eight, nine, ten, or more than ten most abundant types (e.g., species). The most abundant types can be the most abundant kingdoms, phyla or divisions, classes, orders, families, genuses, species, or other classifications. The most abundant types can be the most abundant cell types, such as epithelial cells, bone cells, muscle cells, blood cells, adipose cells, or other cell types. The most abundant types can be non-cancerous cells. The most abundant types can be cancerous cells. The most abundant types can be animal, human, plant, fungal, bacterial, or viral. gNAs can be derived from both a host and the one or more most abundant non-host types (e.g., species) in a sample, such as from both human DNA and the DNA of the one or more most abundant bacterial species. In some embodiments, the abundant DNA comprises DNA from the more abundant or most abundant cells in a sample. For example, for a specific sample, the highly abundant cells can be extracted and their DNA can be used to produce gNAs; these gNAs can be used to produce depletion library and applied to original sample to enable or enhance sequencing or detection of low abundance targets.

In some embodiments, the gNAs are derived from DNA comprising short terminal repeats (STRs).

In some embodiments, the gNAs are derived from a genomic fragment, comprising a region of the genome, or the whole genome itself. In one embodiment, the genome is a DNA genome. In another embodiment, the genome is a RNA genome.

In some embodiments, the gNAs are derived from a eukaryotic or prokaryotic organism; from a mammalian organism or a non-mammalian organism; from an animal or a plant; from a bacteria or virus; from an animal parasite; from a pathogen.

In some embodiments, the gNAs are derived from any mammalian organism. In one embodiment the mammal is a human. In another embodiment the mammal is a livestock animal, for example a horse, a sheep, a cow, a pig, or a donkey. In another embodiment, a mammalian organism is a domestic pet, for example a cat, a dog, a gerbil, a mouse, a rat. In another embodiment the mammal is a type of a monkey.

In some embodiments, the gNAs are derived from any bird or avian organism. An avian organism includes but is not limited to chicken, turkey, duck and goose.

In some embodiments, the sequences of interest are from an insect. Insects include, but are not limited to honeybees, solitary bees, ants, flies, wasps or mosquitoes.

In some embodiments, the gNAs are derived from a plant. In one embodiment, the plant is rice, maize, wheat, rose, grape, coffee, fruit, tomato, potato, or cotton.

In some embodiments, the gNAs are derived from a species of bacteria. In one embodiment, the bacteria are tuberculosis-causing bacteria.

In some embodiments, the gNAs are derived from a virus.

In some embodiments, the gNAs are derived from a species of fungi.

In some embodiments, the gNAs are derived from a species of algae.

In some embodiments, the gNAs are derived from any mammalian parasite.

In some embodiments, the gNAs are derived from any mammalian parasite. In one embodiment, the parasite is a worm. In another embodiment, the parasite is a malaria-causing parasite. In another embodiment, the parasite is a Leishmaniasis-causing parasite. In another embodiment, the parasite is an amoeba.

In some embodiments, the gNAs are derived from a nucleic acid target. Contemplated targets include, but are not limited to, pathogens; single nucleotide polymorphisms (SNPs), insertions, deletions, tandem repeats, or translocations; human SNPs or STRs; potential toxins; or animals, fungi, and plants. In some embodiments, the gRNAs are derived from pathogens, and are pathogen-specific gNAs.

In some embodiments, a guide NA comprises a first NA segment comprising a targeting sequence, wherein the targeting sequence is 15-250 bp; and a second NA segment comprising a nucleic acid guided nuclease system (e.g., CRISPR/Cas system) protein-binding sequence. In some embodiments, the targeting sequence is greater than 21 bp, greater than 22 bp, greater than 23 bp, greater than 24 bp, greater than 25 bp, greater than 26 bp, greater than 27 bp, greater than 28 bp, greater than 29 bp, greater than 30 bp, greater than 40 bp, greater than 50 bp, greater than 60 bp, greater than 70 bp, greater than 80 bp, greater than 90 bp, greater than 100 bp, greater than 110 bp, greater than 120 bp, greater than 130 bp, greater than 140 bp, or even greater than 150 bp. In an exemplary embodiment, the targeting sequence is greater than 30bp. In some embodiments, the targeting sequences range in size from 30-50 bp. In some embodiments, targeting sequences range in size from 30-75 bp. In some embodiments, targeting sequences range in size from 30-100 bp. For example, a targeting sequence can be at least 15 bp, 20 bp, 25 bp, 30 bp, 35 bp, 40 bp, 45 bp, 50 bp, 55 bp, 60 bp, 65 bp, 70 bp, 75 bp, 80 bp, 85 bp, 90 bp, 95 bp, 100 bp, 110 bp, 120 bp, 130 bp, 140 bp, 150 bp, 160 bp, 170 bp, 180 bp, 190 bp, 200 bp, 210 bp, 220 bp, 230 bp, 240 bp, or 250 bp. In specific embodiments, the targeting sequence is at least 20 bp. In specific embodiments, the targeting sequence is at least 22 bp. In specific embodiments, the targeting sequence is at least 30 bp.

In some embodiments, target-specific gNAs can comprise a nucleic acid sequence that is complementary to a region on the opposite strand of the targeted nucleic acid sequence 5' to a PAM sequence, which can be recognized by a nucleic acid-guided nuclease system (e.g., CRISPR/Cas system) protein. In some embodiments the targeted nucleic acid sequence is immediately 5' to a PAM sequence. In specific embodiments, the nucleic acid sequence of the gNA that is complementary to a region in a target nucleic acid is 15-250 bp. In specific embodiments, the nucleic acid sequence of the gNA that is complementary to a region in a target nucleic acid is 20, 22, 23, 24, 25, 30, 35, 40, 45, 50, 60, 70, 75, 80, 90, or 100 bp.

In some particular embodiments, the targeting sequence is not 20 bp. In some particular embodiments, the targeting sequence is not 21 bp.

In some embodiments, the gNAs comprise any purines or pyrimidines (and/or modified versions of the same). In some embodiments, the gNAs comprise adenine, uracil, guanine, and cytosine (and/or modified versions of the same). In some embodiments, the gNAs comprise adenine, thymine, guanine, and cytosine (and/or modified versions of the same). In some embodiments, the gNAs comprise adenine, thymine, guanine, cytosine and uracil (and/or modified versions of the same).

In some embodiments, the gNAs comprise a label, are attached to a label, or are capable of being labeled. In some embodiments, the gNA comprises a moiety that is further capable of being attached to a label. A label includes, but is not limited to, an enzyme, an enzyme substrate, an antibody, an antigen binding fragment, a peptide, a chromophore, a lumiphore, a fluorophore, a chromogen, a hapten, an antigen, a radioactive isotope, a magnetic particle, a metal nanoparticle, a redox active marker group (capable of undergoing a redox reaction), an aptamer, one member of a binding pair, a member of a FRET pair (either a donor or acceptor fluorophore), and combinations thereof.

In some embodiments, the gNAs are attached to a substrate. The substrate can be made of glass, plastic, silicon, silica-based materials, functionalized polystyrene, functionalized polyethyleneglycol, functionalized organic polymers, nitrocellulose or nylon membranes, paper, cotton, and materials suitable for synthesis. Substrates need not be flat. In some embodiments, the substrate is a 2-dimensional array. In some embodiments, the 2-dimensional array is flat. In some embodiments, the 2-dimensional array is not flat, for example, the array is a wave-like array. Substrates include any type of shape including spherical shapes (e.g., beads). Materials attached to substrates may be attached to any portion of the substrates (e.g., may be attached to an interior portion of a porous substrates material). In some embodiments, the substrate is a 3-dimensional array, for example, a microsphere. In some embodiments, the microsphere is magnetic. In some embodiments, the microsphere is glass. In some embodiments, the microsphere is made of polystyrene. In some embodiments, the microsphere is silica-based. In some embodiments, the substrate is an array with interior surface, for example, is a straw, tube, capillary, cylindrical, or microfluidic chamber array. In some embodiments, the substrate comprises multiple straws, capillaries, tubes, cylinders, or chambers.

### Nucleic Acids Encoding gNAs

Also provided herein are nucleic acids encoding for gNAs (e.g., gRNAs or gDNAs). In some embodiments, by encoding it is meant that a gNA results from the transcription of a nucleic acid encoding for a gNA (e.g., gRNA). T7 promoters are discussed in this disclosure, though the use of other appropriate promoters is also contemplated. In some embodiments, by encoding, it is meant that the nucleic acid is a template for the transcription of a gNA (e.g., gRNA). In some embodiments, by encoding, it is meant that a gNA results from the reverse transcription of a nucleic acid encoding for a gNA. In some embodiments, by encoding, it is meant that the nucleic acid is a template for the reverse transcription of a gNA. In some embodiments, by encoding, it is meant that a gNA results from the amplification of a nucleic acid encoding for a gNA. In some embodiments, by encoding, it is meant that the nucleic acid is a template for the amplification of a gNA.

In some embodiments the nucleic acid encoding for a gNA comprises a first segment comprising a regulatory region; a second segment comprising targeting sequence, wherein the second segment can range from 15 bp - 250 bp; and a third segment comprising a nucleic acid encoding a nucleic acid-guided nuclease system (e.g., CRISPR/Cas system) protein-binding sequence.

In some embodiments, the nucleic acids encoding for gNAs comprise DNA. In some embodiments, the first segment is double stranded DNA. In some embodiments, the first segment is single stranded DNA. In some embodiments, the second segment is single stranded DNA. In some embodiments, the third segment is single stranded DNA. In some embodiments, the second segment is double stranded DNA. In some embodiments, the third segment is double stranded DNA.

In some embodiments, the nucleic acids encoding for gNAs comprise RNA.

In some embodiments the nucleic acids encoding for gNAs comprise DNA and RNA.

In some embodiments, the regulatory region is a region capable of binding a transcription factor. In some embodiments, the regulatory region comprises a promoter. In some embodiments, the promoter is selected from the group consisting of T7, SP6, and T3.

### Collections of gNAs

Provided herein are collections (interchangeably referred to as libraries) of gNAs.

As used herein, a collection of gNAs denotes a mixture of gNAs containing at least $10^2$ unique gNAs. In some embodiments a collection of gNAs contains at least $10^2$, at least $10^3$, at least $10^4$, at least $10^5$, at least $10^6$, at least $10^7$, at least $10^8$, at least $10^9$, at least $10^{10}$ unique gNAs. In some embodiments a collection of gNAs contains a total of at least $10^2$, at least $10^3$, at least $10^4$, at least $10^5$, at least $10^6$, at least $10^7$, at least $10^8$, at least $10^9$, at least $10^{10}$ gNAs.

In some embodiments, a collection of gNAs comprises a first NA segment comprising a targeting sequence; and a second NA segment comprising a nucleic acid-guided nuclease system (e.g., CRISPR/Cas system) protein-binding sequence, wherein at least 10% of the gNAs in the collection vary in size. In some embodiments, the first and second segments are in 5'- to 3'-order'. In some embodiments, the first and second segments are in 3'- to 5'-order'.

In some embodiments, the size of the first segment varies from 15-250 bp, or 20bp, or 30-100 bp, or 20-30bp, or 22-30 bp, or 15-50bp, or 15-75 bp, or 15-100 bp, or 15-125 bp, or 15-150 bp, or 15-175 bp, or 15-200 bp, or 15-225 bp, or 15-250 bp, or 22-50 bp, or 22-75 bp, or 22-100 bp, or 22-125 bp, or 22-150 bp, or 22-175 bp, or 22-200 bp, or 22-225 bp, or 22-250 bp across the collection of gNAs.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are greather than or equal to to 20 bp. In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are equal to 20 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are greater than 21 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are greater than 25 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are greater than 30 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are 15-50 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the first segments in the collection are 30-100 bp.

In some particular embodiments, the size of the first segment is not 20 bp.

In some particular embodiments, the size of the first segment is not 21 bp.

In some embodiments, the gNAs and/or the targeting sequence of the gNAs in the collection of gRNAs comprise unique 5' ends. In some embodiments, the collection of gNAs exhibit variability in sequence of the 5' end of the targeting sequence, across the members of the collection. In some embodiments, the collection of gNAs exhibit variability at least 5%, or at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75% variability in the sequence of the 5' end of the targeting sequence, across the members of the collection.

In some embodiments, the 3' end of the gNA targeting sequence can be any purine or pyrimidine (and/or modified versions of the same). In some embodiments, the 3' end of the gNA targeting sequence is an adenine. In some embodiments, the 3' end of the gNA targeting sequence is a guanine. In some embodiments, the 3' end of the gNA targeting sequence is a cytosine. In some embodiments, the 3' end of the gNA targeting sequence is a uracil. In some embodiments, the 3' end of the gNA targeting sequence is a thymine. In some embodiments, the 3' end of the gNA targeting sequence is not cytosine.

In some embodiments, the collection of gNAs comprises targeting sequences which can base-pair with the targeted DNA, wherein the target of interest is spaced at least every 1 bp, at least every 2 bp, at least every 3 bp, at least every 4 bp, at least every 5 bp, at least every 6 bp, at least every 7 bp, at least every 8 bp, at least every 9 bp, at least every 10 bp, at least every 11 bp, at least every 12 bp, at least every 13 bp, at least every 14 bp, at least every 15 bp, at least every 16 bp, at least every 17 bp, at least every 18 bp, at least every 19 bp, 20 bp, at least every 25 bp, at least every 30 bp, at least every 40 bp, at least every 50 bp, at least every 100 bp, at least every 200 bp, at least every 300 bp, at least every 400 bp, at least every 500 bp, at least every 600 bp, at least every 700 bp, at least every 800 bp, at least every 900 bp, at least every 1000 bp, at least every 2500 bp, at least every 5000 bp, at least every 10,000 bp, at least every 15,000 bp, at least every 20,000 bp, at least every 25,000 bp, at least every 50,000 bp, at least every 100,000 bp, at least every 250,000 bp, at least every 500,000 bp, at least every 750,000bp, or even at least every 1,000,000 bp across a genome of interest.

In some embodiments, the collection of gNAs comprises a first NA segment comprising a targeting sequence; and a second NA segment comprising a nucleic acid-guided nuclease system (e.g., CRISPR/Cas system) protein-binding sequence, wherein the gNAs in the collection can have a variety of second NA segments with various specificities for protein members of the nucleic acid-guided nuclease system (e.g., CRISPR/Cas system). For example a collection of gNAs as provided herein, can comprise members whose second segment comprises a nucleic acid-guided nuclease system (e.g., CRISPR/Cas system) protein-binding sequence specific for a first nucleic acid-guided nuclease system (e.g., CRISPR/Cas system) protein; and also comprises members whose second segment comprises a nucleic acid-guided nuclease system (e.g., CRISPR/Cas system) protein-binding sequence specific for a second nucleic acid-guided nuclease system (e.g., CRISPR/Cas system) protein, wherein the first and second nucleic acid-guided nuclease system (e.g., CRISPR/Cas system) proteins are not the same. In some embodiments a collection of gNAs as provided herein comprises members that exhibit specificity to at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or even at least 20 nucleic acid-guided nuclease system (e.g., CRISPR/Cas system) proteins. In one specific embodiment, a collection of gNAs as provided herein comprises members that exhibit specificity for a Cas9 protein and another protein selected from the group consisting of Cpfl, Cas3, Cas8a-c, Cas10, Cse1, Csy1, Csn2, Cas4, Csm2, and Cm5. In some embodiments, the nucleic acid-guided nuclease system protein-binding sequences specific for the first and second nucleic acid-guided nuclease system proteins are both 5' of the first NA segment comprising a targeting sequence. In some embodiments, the nucleic acid-guided nuclease system protein-binding sequences specific for the first and second nucleic acid-guided nuclease system proteins are both 3' of the first NA segment comprising a targeting sequence. In some embodiments, the nucleic acid-guided nuclease system protein-binding sequence specific for the first nucleic acid-guided nuclease system (e.g., CRISPR/Cas system) protein is 5' of the first NA segment comprising a targeting sequence and the second nucleic acid-guided nuclease system protein-binding sequences specific for the second nucleic acid-guided nuclease system protein is 3' of the first NA segment comprising a targeting sequence. The order of the first NA segment comprising a targeting sequence and the second NA segment comprising a nucleic acid-guided nuclease system protein-binding sequence will depend on the nucleic acid-guided nuclease system protein. The appropriate 5' to 3' arrangement of the first and second NA segments and choice of nucleic acid-guided nuclease system proteins will be apparent to one of ordinary skill in the art.

In some embodiments, a plurality of the gNA members of the collection are attached to a label, comprise a label or are capable of being labeled. In some embodiments, the gNA comprises a moiety that is further capable of being attached to a label. Exemplary but nonlimiting moieties comprise digoxigenin (DIG) and fluorescein (FITC). A label includes, but is not limited to, enzyme, an enzyme substrate, an antibody, an antigen binding fragment, a peptide, a chromophore, a lumiphore, a fluorophore, a chromogen, a hapten, an antigen, a radioactive isotope, a magnetic particle, a metal nanoparticle, a redox active marker group (capable of undergoing a redox reaction), an aptamer, one member of a binding pair, a member of a FRET pair (either a donor or acceptor fluorophore), and combinations thereof.

In some embodiments, a plurality of the gNA members of the collection are attached to a substrate. The substrate can be made of glass, plastic, silicon, silica-based materials, functionalized polystyrene, functionalized polyethyleneglycol, functionalized organic polymers, nitrocellulose or nylon membranes, paper, cotton, and materials suitable for synthesis. Substrates need not be flat. In some embodiments, the substrate is a 2-dimensional array. In some embodiments, the 2-dimensional array is flat. In some embodiments, the 2-dimensional array is not flat, for example, the array is a wave-like array. Substrates include any type of shape including spherical shapes (e.g., beads). Materials attached to substrates may be attached to any portion of the substrates (e.g., may be attached to an interior portion of a porous substrates material). In some embodiments, the substrate is a 3-dimensional array, for example, a microsphere. In some embodiments, the microsphere is magnetic. In some embodiments, the microsphere is glass. In some embodiments, the microsphere is made of polystyrene. In some embodiments, the microsphere is silica-based. In some embodiments, the substrate is an array with interior surface, for example, is a straw, tube, capillary, cylindrical, or microfluidic chamber array. In some embodiments, the substrate comprises multiple straws, capillaries, tubes, cylinders, or chambers.

### Collections of Nucleic Acids Encoding gNAs

Provided herein are collections (interchangeably referred to as libraries) of nucleic acids encoding for gNAs (e.g., gRNAs or gDNAs). In some embodiments, by encoding it is meant that a gNA results from the transcription of a nucleic acid encoding for a gNA. In some embodiments, by encoding, it is meant that the nucleic acid is a template for the transcription of a gNA.

As used herein, a collection of nucleic acids encoding for gNAs denotes a mixture of nucleic acids containing at least $10^2$ unique nucleic acids. In some embodiments a collection of nucleic acids encoding for gNAs contains at least $10^2$, at least $10^3$, at least $10^4$, at least $10^5$, at least $10^6$, at least $10^7$, at least $10^8$, at least $10^9$, at least $10^{10}$

unique nucleic acids encoding for gNAs. In some embodiments a collection of nucleic acids encoding for gNAs contains a total of at least 10², at least 10³, at least 10⁴, at least 10⁵, at least 10⁶, at least 10⁷, at least 10⁸, at least 10⁹, at least 10¹⁰ nucleic acids encoding for gNAs.

In some embodiments, a collection of nucleic acids encoding for gNAs comprises a first segment comprising a regulatory region; a second segment comprising a targeting sequence; and a third segment comprising a nucleic acid encoding a nucleic acid-guided nuclease system (e.g., CRISPR/Cas system) protein-binding sequence, wherein at least 10% of the nucleic acids in the collection vary in size.

In some embodiments, the first, second, and third segments are in 5'- to 3'-order'.

In some embodiments, the first second and third segments are arranged, in order from 5' to 3', first segment, third segment and then second segment.

In some embodiments, the nucleic acids encoding for gNAs comprise DNA. In some embodiments, the first segment is single stranded DNA. In some embodiments, the first segment is double stranded DNA. In some embodiments, the second segment is single stranded DNA. In some embodiments, the third segment is single stranded DNA. In some embodiments, the second segment is double stranded DNA. In some embodiments, the third segment is double stranded DNA.

In some embodiments, the nucleic acids encoding for gNAs comprise RNA.

In some embodiments the nucleic acids encoding for gNAs comprise DNA and RNA.

In some embodiments, the regulatory region is a region capable of binding a transcription factor. In some embodiments, the regulatory region comprises a promoter. In some embodiments, the promoter is selected from the group consisting of T7, SP6, and T3.

In some embodiments, the size of the second segments (targeting sequence) in the collection varies from 15-250 bp, or 30-100 bp, or 22-30 bp, or 15-50 bp, or 15-75 bp, or 15-100 bp, or 15-125 bp, or 15-150 bp, or 15-175 bp, or 15-200 bp, or 15-225 bp, or 15-250 bp, or 22-50 bp, or 22-75 bp, or 22-100 bp, or 22-125 bp, or 22-150 bp, or 22-175 bp, or 22-200 bp, or 22-225 bp, or 22-250 bp across the collection of gNAs.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the second segments in the collection are greater than or equal to 20 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the second segments in the collection are greater than 21 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the second segments in the collection are greater than 25 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the second segments in the collection are greater than 30 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the second segments in the collection are 15-50 bp.

In some embodiments, at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100% of the second segments in the collection are 30-100 bp.

In some particular embodiments, the size of the second segment is not 20 bp.

In some particular embodiments, the size of the second segment is not 21 bp.

In some embodiments, the gNAs and/or the targeting sequence of the gNAs in the collection of gNAs comprise unique 5' ends. In some embodiments, the collection of gNAs exhibit variability in sequence of the 5' end of the targeting sequence, across the members of the collection. In some embodiments, the collection of gNAs exhibit variability at least 5%, or at least 10%, or at least 15%, or at last 20%, or at least 25%, or at least 30%, or at least 35%, or at least 40%, or at least 45%, or at least 50%, or at least 55%, or at least 60%, or at least 65%, or at least 70%, or at least 75% variability in the sequence of the 5' end of the targeting sequence, across the members of the collection.

In some embodiments, the collection of nucleic acids comprises targeting sequences, wherein the target of interest is spaced at least every 1 bp, at least every 2 bp, at least every 3 bp, at least every 4 bp, at least every 5 bp, at least every 6 bp, at least every 7 bp, at least every 8 bp, at least every 9 bp, at least every 10 bp, at least every 11 bp, at least every 12 bp, at least every 13 bp, at least every 14 bp, at least every 15 bp, at least every 16 bp, at least every 17 bp, at least every 18 bp, at least every 19 bp, 20 bp, at least every 25 bp, at least every 30 bp, at least every 40 bp, at least every 50 bp, at least every 100 bp, at least every 200 bp, at least every 300 bp, at least every 400 bp, at least every 500 bp, at least every 600 bp, at least every 700 bp, at least every 800 bp, at least every 900 bp, at least every 1000 bp, at least every 2500 bp, at least every 5000 bp, at least every 10,000 bp, at least every 15,000 bp, at least every 20,000 bp, at least every 25,000 bp, at least every 50,000 bp, at least every 100,000 bp, at least every 250,000 bp, at least every 500,000 bp, at least every 750,000 bp, or even at least every 1,000,000 bp across a genome of interest.

In some embodiments, the collection of nucleic acids encoding for gNAs comprise a third segment encoding for a nucleic acid-guided nuclease system (e.g., CRISPR/Cas system) protein-binding sequence, wherein the segments in the collection vary in their specificity for protein members of the nucleic acid-guided nuclease system (e.g., CRISPR/Cas system). For example, a collection of nucleic acids encoding for gNAs as provided herein, can comprise members whose third segment encode for a nucleic acid-guided nuclease system (e.g., CRISPR/Cas system) protein-binding sequence specific for a first nucleic acid-guided nuclease system (e.g., CRISPR/Cas system) protein; and also comprises members whose third segment encodes for a nucleic acid-guided nuclease system (e.g., CRISPR/Cas system) protein-binding sequence specific for a second nucleic acid-guided nuclease system (e.g., CRISPR/Cas system) protein, wherein the first and second nucleic acid-guided nuclease system (e.g., CRISPR/Cas system) proteins are not the same. In some embodiments, a collection of nucleic acids encoding for gNAs as provided herein comprises members that exhibit specificity to at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or even at least 20 nucleic acid-guided nuclease system (e.g., CRISPR/Cas system) proteins. In one specific embodiment, a collection of nucleic acids encoding for gNAs as provided herein comprises members that exhibit specificity for a Cas9 protein and another protein selected from the group consisting of Cpfl, Cas3, Cas8a-c, Cas10, Cse1, Csy1, Csn2, Cas4, Csm2, and Cm5. In some embodiments, the nucleic acid-guided nuclease system protein-binding sequences specific for the first and second nucleic acid-guided nuclease system proteins are both 5' of the first NA segment comprising a targeting sequence. In some embodiments, the nucleic acid-guided nuclease system protein-binding sequences specific for the first and second nucleic acid-guided nuclease system proteins are both 3' of the first NA segment comprising a targeting sequence. In some embodiments, the nucleic acid-guided nuclease system protein-binding sequence specific for the first nucleic acid-guided nuclease system (e.g., CRISPR/Cas system) protein is 5' of the first NA segment comprising a targeting sequence and the second nucleic acid-guided nuclease system protein-binding sequences specific for the second nucleic acid-guided nuclease system protein is 3' of the first NA segment comprising a targeting sequence. The order of the first NA segment comprising a targeting sequence and the second NA segment comprising a nucleic acid-guided nuclease system protein-binding sequence will depend on the nucleic acid-guided nuclease system protein. The appropriate 5' to 3' arrangement of the first and second NA segments and choice of nucleic acid-guided nuclease system proteins will be apparent to one of ordinary skill in the art.

### *Sequences of Interest*

Provided herein are gNAs and collections of gNAs, derived from any source DNA (for example from genomic DNA, cDNA, artificial DNA, DNA libraries), that can be used to target sequences of interest in a sample for a variety of applications including, but not limited to, enrichment, depletion, capture, partitioning, labeling, regulation, and editing. The gNAs comprise a targeting sequence, directed at sequences of interest.

In some embodiments, the sequences of interest are genomic sequences (genomic DNA). In some embodiments, the sequences of interest are mammalian genomic sequences. In some embodiments, the sequences of interest are eukaryotic genomic sequences. In some embodiments, the sequences of interest are prokaryotic genomic sequences. In some embodiments, the sequences of interest are viral genomic sequences. In some embodiments, the sequences of interest are bacterial genomic sequences. In some embodiments, the sequences of interest are plant genomic sequences. In some embodiments, the sequences of interest are microbial genomic sequences. In some embodiments, the sequences of interest are genomic sequences from a parasite, for example a eukaryotic parasite. In some embodiments, the sequences of interest are host genomic sequences (e.g., the host organism of a microbiome, a parasite, or a pathogen). In some embodiments, the sequences of interest are abundant genomic sequences, such as sequences from the genome or genomes of the most abundant species in a sample.

In some embodiments, the sequences of interest comprise repetitive DNA. In some embodiments, the sequences of interest comprise abundant DNA. In some embodiments, the sequences of interest comprise mitochondrial DNA. In some embodiments, the sequences of interest comprise ribosomal DNA. In some embodiments, the sequences of interest comprise centromeric DNA. In some embodiments, the sequences of interest comprise DNA comprising Alu elements (Alu DNA). In some embodiments, the sequences of interest comprise long interspersed nuclear elements (LINE DNA). In some embodiments, the sequences of interest comprise short interspersed nuclear elements (SINE DNA). In some embodiments, the abundant DNA comprises ribosomal DNA.

In some embodiments, the sequences of interest comprise single nucleotide polymorphisms (SNPs), short tandem repeats (STRs), cancer genes, inserts, deletions, structural variations, exons, genetic mutations, or regulatory regions.

In some embodiments, the sequences of interest can be a genomic fragment, comprising a region of the genome, or the whole genome itself. In one embodiment, the genome is a DNA genome. In another embodiment, the genome is a RNA genome.

In some embodiments, the sequences of interest are from a eukaryotic or prokaryotic organism; from a mammalian organism or a non-mammalian organism; from an animal or a plant; from a bacteria or virus; from an animal parasite; from a pathogen.

In some embodiments, the sequences of interest are from any mammalian organism. In one embodiment, the mammal is a human. In another embodiment, the mammal is a livestock animal, for example a horse, a sheep, a cow, a pig, or a donkey. In another embodiment, a mammalian organism is a domestic pet, for example a cat, a dog, a gerbil, a mouse, a rat. In another embodiment, the mammal is a type of a monkey.

In some embodiments, the sequences of interest are from any bird or avian organism. An avian organism includes but is not limited to chicken, turkey, duck and goose.

In some embodiments, the sequences of interest are from an insect. Insects include, but are not limited to honeybees, solitary bees, ants, flies, wasps or mosquitoes.

In some embodiments, the sequences of interest are from a plant. In one embodiment, the plant is rice, maize, wheat, rose, grape, coffee, fruit, tomato, potato, or cotton.

In some embodiments, the sequences of interest are from a species of bacteria. In one embodiment, the bacteria are tuberculosis-causing bacteria.

In some embodiments, the sequences of interest are from a virus.

In some embodiments, the sequences of interest are from a species of fungi.

In some embodiments, the sequences of interest are from a species of algae.

In some embodiments, the sequences of interest are from any mammalian parasite.

In some embodiments, the sequences of interest are obtained from any mammalian parasite. In one embodiment, the parasite is a worm. In another embodiment, the parasite is a malaria-causing parasite. In another embodiment, the parasite is a Leishmaniasis-causing parasite. In another embodiment, the parasite is an amoeba.

In some embodiments, the sequences of interest are from a pathogen.

Targeting Sequences

As used herein, a targeting sequence is one that directs the gNA to the sequences of interest in a sample. For example, a targeting sequence targets a particular sequence of interest, for example the targeting sequence targets a genomic sequence of interest.

Provided herein are gNAs and collections of gNAs that comprise a segment that comprises a targeting sequence. Also provided herein, are nucleic acids encoding for gNAs, and collections of nucleic acids encoding for gNAs that comprise a segment encoding for a targeting sequence.

In some embodiments, the targeting sequence comprises DNA.

In some embodiments, the targeting sequence comprises RNA.

In some embodiments, the targeting sequence comprises RNA, and shares at least 70% sequence identity, at least 75% sequence identity, at least 80% sequence identity, at least 85% sequence identity, at least 90% sequence identity, at least 95% sequence identity, or shares 100% sequence identity to a sequence 5' to a PAM sequence on a sequence of interest, except that the RNA comprises uracils instead of thymines. In some embodiments, the targeting sequence comprises RNA, and shares at least 70% sequence identity, at least 75% sequence identity, at least 80% sequence identity, at least 85% sequence identity, at least 90% sequence identity, at least 95% sequence identity, or shares 100% sequence identity to a sequence 3' to a PAM sequence on a sequence of interest, except that the RNA comprises uracils instead of thymines. In some embodiments, the PAM sequence is AGG, CGG, TGG, GGG or NAG. In some embodiments, the PAM sequence is TTN, TCN or TGN.

In some embodiments, the targeting sequence comprises DNA, and shares at least 70% sequence identity, at least 75% sequence identity, at least 80% sequence identity, at least 85% sequence identity, at least 90% sequence identity, at least 95% sequence identity, or shares 100% sequence identity to a sequence 5' to a PAM sequence on a sequence of interest. In some embodiments, the targeting sequence comprises DNA, and shares at least 70% sequence identity, at least 75% sequence identity, at least 80% sequence identity, at least 85% sequence identity, at least 90% sequence identity, at least 95% sequence identity, or shares 100% sequence identity to a sequence 3' to a PAM sequence on a sequence of interest.

In some embodiments, the targeting sequence comprises RNA and is complementary to the strand opposite to a sequence of nucleotides 5' to a PAM sequence. In some embodiments, the targeting sequence is at least 70% complementary, at least 75% complementary, at least 80% complementary, at least 85% complementary, at least 90% complementary, at least 95% complementary, or is 100% complementary to the strand opposite to a sequence of nucleotides 5' to a PAM sequence. In some embodiments, the targeting sequence comprises RNA and is complementary to the strand opposite to a sequence of nucleotides 3' to a PAM sequence. In some embodiments, the targeting sequence is at least 70% complementary, at least 75% complementary, at least 80% complementary, at least 85% complementary, at least 90% complementary, at least 95% complementary, or is 100% complementary to the strand opposite to a sequence of nucleotides 3' to a PAM sequence. In some embodiments, the PAM sequence is AGG, CGG, TGG, GGG or NAG. In some embodiments, the PAM sequence is TTN, TCN or TGN.

In some embodiments, the targeting sequence comprises DNA and is complementary to the strand opposite to a sequence of nucleotides 5' to a PAM sequence. In some embodiments, the targeting sequence is at least 70% complementary, at least 75% complementary, at least 80% complementary, at least 85% complementary, at least 90% complementary, at least 95% complementary, or is 100% complementary to the strand opposite to a sequence of nucleotides 5' to a PAM sequence. In some embodiments, the targeting sequence comprises DNA and is complementary to the strand opposite to a sequence of nucleotides 3' to a PAM sequence. In some embodiments, the targeting sequence is at least 70% complementary, at least 75% complementary, at least 80% complementary, at least 85% complementary, at least 90% complementary, at least 95% complementary, or is 100% complementary to the strand opposite to a sequence of nucleotides 3' to a PAM sequence. In some embodiments, the PAM sequence is AGG, CGG, TGG, GGG or NAG. In some embodiments, the PAM sequence is TTN, TCN or TGN.

In some embodiments, a DNA encoding for a targeting sequence of a gRNA shares at least 70% sequence identity, at least 75% sequence identity, at least 80% sequence identity, at least 85% sequence identity, at least 90% sequence identity, at least 95% sequence identity, or shares 100% sequence identity to the strand opposite to a sequence of nucleotides 5' to a PAM sequence. In some embodiments, a DNA encoding for a targeting sequence of a gRNA shares at least 70% sequence identity, at least 75% sequence identity, at least 80% sequence identity, at least 85% sequence identity, at least 90% sequence identity, at least 95% sequence identity, or shares 100% sequence identity to the strand opposite to a sequence of nucleotides 3' to a PAM sequence. In some embodiments, the PAM sequence is AGG, CGG, TGG, GGG or NAG. In some embodiments, the PAM sequence is TTN, TCN or TGN.

In some embodiments, a DNA encoding for a targeting sequence of a gRNA is complementary to the strand opposite to a sequence of nucleotides 5' to a PAM sequence and is at least 70% complementary, at least 75% complementary, at least 80% complementary, at least 85% complementary, at least 90% complementary, at least 95% complementary, or is 100% complementary to a sequence 5' to a PAM sequence on a sequence of interest. In some embodiments, a DNA encoding for a targeting sequence of a gRNA is complementary to the strand opposite to a sequence of nucleotides 5' to a PAM sequence and is at least 70% complementary, at least 75% complementary, at least 80% complementary, at least 85% complementary, at least 90% complementary, at least 95% complementary, or is 100% complementary to a sequence 3' to a PAM sequence on a sequence of interest. In some embodiments, the PAM sequence is AGG, CGG, TGG, GGG or NAG. In some embodiments, the PAM sequence is TTN, TCN or TGN.

Different CRISPR/Cas system proteins recognize different PAM sequences. PAM sequences can be located 5' or 3' of a targeting sequence. For example, Cas9 can recognize an NGG PAM located on the immediate 3' end of a targeting sequence. Cpfl can recognize a TTN PAM located on the immediate 5' end of a targeting sequence. All PAM sequences recognized by all CRISPR/Cas system proteins are envisaged as being within the scope of the invention. It will be readily apparent to one of ordinary skill in the art which PAM sequences are compatible with a particular CRISPR/Cas system protein.

### *Nucleic Acid-Guided Nuclease System Proteins*

Provided herein are gNAs and collections of gNAs comprising a segment that comprises a nucleic acid-guided nuclease system (e.g., CRISPR/Cas system) protein-binding sequence. Also provided herein, are nucleic acids encoding for gNAs, and collections of nucleic acids encoding for gNAs that comprise a segment encoding a nucleic acid-guided nuclease system (e.g., CRISPR/Cas system) protein-binding sequence. A nucleic acid-guided nuclease system can be an RNA-guided nuclease system. A nucleic acid-guided nuclease system can be a DNA-guided nuclease system.

Methods of the present disclosure can utilize nucleic acid-guided nucleases. As used herein, a "nucleic acid-guided nuclease" is any nuclease that cleaves DNA, RNA or DNA/RNA hybrids, and which uses one or more nucleic acid guide nucleic acids (gNAs) to confer specificity. Nucleic acid-guided nucleases include CRISPR/Cas system proteins as well as non-CRISPR/Cas system proteins.

The nucleic acid-guided nucleases provided herein can be DNA guided DNA nucleases; DNA guided RNA nucleases; RNA guided DNA nucleases; or RNA guided RNA nucleases. The nucleases can be endonucleases. The nucleases can be exonucleases. In one embodiment, the nucleic acid-guided nuclease is a nucleic acid-guided-DNA endonuclease. In one embodiment, the nucleic acid-guided nuclease is a nucleic acid-guided-RNA endonuclease.

A nucleic acid-guided nuclease system protein-binding sequence is a nucleic acid sequence that binds any protein member of a nucleic acid-guided nuclease system. For example, a CRISPR/Cas system protein-binding sequence is a nucleic acid sequence that binds any protein member of a CRISPR/Cas system.

In some embodiments, the nucleic acid-guided nuclease is selected from the group consisting of CAS Class I Type I, CAS Class I Type III, CAS Class I Type IV, CAS Class II Type II, and CAS Class II Type V. In some embodiments, CRISPR/Cas system proteins include proteins from CRISPR Type I systems, CRISPR Type II systems, and CRISPR Type III systems. In some embodiments, the nucleic acid-guided nuclease is selected from the group consisting of Cas9, Cpfl, Cas3, Cas8a-c, Cas10, Cse1, Csy1, Csn2, Cas4, Csm2, Cm5, Csf1, C2c2, and NgAgo.

In some embodiments, nucleic acid-guided nuclease system proteins (e.g., CRISPR/Cas system proteins) can be from any bacterial or archaeal species.

In some embodiments, the nucleic acid-guided nuclease system proteins (e.g., CRISPR/Cas system proteins) are from, or are derived from nucleic acid-guided nuclease system proteins (e.g., CRISPR/Cas system proteins) from *Streptococcus pyogenes, Staphylococcus aureus, Neisseria meningitidis, Streptococcus thermophiles, Treponema denticola, Francisella tularensis, Pasteurella multocida, Campylobacter jejuni, Campylobacter lari, Mycoplasma gallisepticum, Nitratifractor salsuginis, Parvibaculum lavamentivorans, Roseburia intestinalis, Neisseria cinerea, Gluconacetobacter diazotrophicus, Azospirillum, Sphaerochaeta globus, Flavobacterium columnare, Fluviicola taffensis, Bacteroides coprophilus, Mycoplasma mobile, Lactobacillus farciminis, Streptococcus pasteurianus, Lactobacillus johnsonii, Staphylococcus pseudintermedius, Filifactor alocis, Legionella pneumophila, Suterella wadsworthensis Corynebacter diphtheria, Acidaminococcus, Lachnospiraceae bacterium* or *Prevotella.*

In some embodiments, examples of nucleic acid-guided nuclease system (e.g., CRISPR/Cas system) proteins can be naturally occurring or engineered versions.

In some embodiments, naturally occurring nucleic acid-guided nuclease system (e.g., CRISPR/Cas system) proteins include Cas9, Cpfl, Cas3, Cas8a-c, Cas10, Cse1, Csy1, Csn2, Cas4, Csm2, and Cm5. Engineered versions of such proteins can also be employed.

In some embodiments, engineered examples of nucleic acid-guided nuclease system (e.g., CRISPR/Cas system) proteins include catalytically dead nucleic acid-guided nuclease system proteins. The term "catalytically dead" generally refers to a nucleic acid-guided nuclease system protein that has inactivated nucleases (e.g., HNH and RuvC nucleases). Such a protein can bind to a target site in any nucleic acid (where the target site is determined by the guide NA), but the protein is unable to cleave or nick the target nucleic acid (e.g., double-stranded DNA). In some embodiments, the nucleic acid-guided nuclease system catalytically dead protein is a catalytically dead CRISPR/Cas system protein, such as catalytically dead Cas9 (dCas9). Accordingly, the dCas9 allows separation of the mixture into unbound nucleic acids and dCas9-bound fragments. In one embodiment, a dCas9/gRNA complex binds to targets determined by the gRNA sequence. The dCas9 bound can prevent cutting by Cas9 while other manipulations proceed. In another embodiment, the dCas9 can be fused to another enzyme, such as a transposase, to target that enzyme's activity to a specific site. Naturally occurring catalytically dead nucleic acid-guided nuclease system proteins can also be employed.

In some embodiments, engineered examples of nucleic acid-guided nuclease (e.g., CRISPR/Cas) system proteins also include nucleic acid-guided nickases (e.g., Cas nickases). A nucleic acid-guided nickase refers to a modified version of a nucleic acid-guided nuclease system protein, containing a single inactive catalytic domain. In one embodiment, the nucleic acid-guided nickase is a Cas nickase, such as Cas9 nickase. A Cas9 nickase may contain a single inactive catalytic domain, for example, either the RuvC- or the HNH-domain. With only one active nuclease domain, the Cas9 nickase cuts only one strand of the target DNA, creating a single-strand break or "nick". Depending on which mutant is used, the guide NA-hybridized strand or the non-hybridized strand may be cleaved. Nucleic acid-guided nickases bound to 2 gNAs that target opposite strands will create a double-strand break in a target double-stranded DNA. This "dual nickase" strategy can increase the specificity of cutting because it requires that both nucleic acid-guided nuclease/gNA (e.g., Cas9/gRNA) complexes be specifically bound at a site before a double-strand break is formed. Naturally occurring nickase nucleic acid-guided nuclease system proteins can also be employed.

In some embodiments, engineered examples of nucleic acid-guided nuclease system proteins also include nucleic acid-guided nuclease system fusion proteins. For example, a nucleic acid-guided nuclease (e.g., CRISPR/Cas) system protein may be fused to another protein, for example an activator, a repressor, a nuclease, a fluorescent molecule, a radioactive tag, or a transposase.

In some embodiments, the nucleic acid-guided nuclease system protein-binding sequence comprises a gNA (e.g., gRNA) stem-loop sequence.

Different CRISPR/Cas system proteins are compatible with different nucleic acid-guided nuclease system protein-binding sequences. It will be readily apparent to one of ordinary skill in the art which CRISPR/Cas system proteins are compatible with which nucleic acid-guided nuclease system protein-binding sequences.

In some embodiments, a double-stranded DNA sequence encoding the gNA (e.g., gRNA) stem-loop sequence comprises the following DNA sequence on one strand (5'>3', GTTTTAGAGCTAGAAATAGCAAGTTAAAATAAGGCTAGTCCGTTATCAACTTGAAA AAGTGGCACCGAGTCGGTGCTTTTTTT), and its reverse-complementary DNA on the other strand (5'>3', AAAAAAAGCACCGACTCGGTGCCACTTTTTCAAGTTGATAACGGACTAGCCTTATTT TAACTTGCTATTTCTAGCTCTAAAAC).

In some embodiments, a single-stranded DNA sequence encoding the gNA (e.g., gRNA) stem-loop sequence comprises the following DNA sequence: (5'>3', AAAAAAAGCACCGACTCGGTGCCACTTTTTCAAGTTGATAACGGACTAGCCTTATTT TAACTTGCTATTTCTAGCTCTAAAAC), wherein the single-stranded DNA serves as a transcription template.

In some embodiments, the gNA (e.g., gRNA) stem-loop sequence comprises the following RNA sequence: (5'>3', GUUUUAGAGCUAGAAAUAGCAAGUUAAAAUAAGGCUAGUCCGUUAUCAACUUGA AAAAGUGGCACCGAGUCGGUGCUUUUUUU).

In some embodiments, a double-stranded DNA sequence encoding the gNA (e.g., gRNA) stem-loop sequence comprises the following DNA sequence on one strand (5'>3', GTTTTAGAGCTATGCTGGAAACAGCATAGCAAGTTAAAATAAGGCTAGTCCGTTAT CAACTTGAAAAAGTGGCACCGAGTCGGTGCTTTTTTTC), and its reverse-complementary DNA on the other strand (5'>3', GAAAAAAAGCACCGACTCGGTGCCACTTTTTCAAGTTGATAACGGACTAGCCTTATT TTAACTTGCTATGCTGTTTCCAGCATAGCTCTAAAAC).

In some embodiments, a single-stranded DNA sequence encoding the gNA (e.g., gRNA) stem-loop sequence comprises the following DNA sequence: (5'>3', GAAAAAAAGCACCGACTCGGTGCCACTTTTTCAAGTTGATAACGGACTAGCCTTATT TTAACTTGCTATGCTGTTTCCAGCATAGCTCTAAAAC), wherein the single-stranded DNA serves as a transcription template.

In some embodiments, the gNA (e.g., gRNA) stem-loop sequence comprises the following RNA sequence: (5'>3', GUUUUAGAGCUAUGCUGGAAACAGCAUAGCAAGUUAAAAUAAGGCUAGUCCGUU AUCAACUUGAAAAAGUGGCACCGAGUCGGUGCUUUUUUUC).

In some embodiments, the CRISPR/Cas system protein is a Cpfl protein. In some embodiments, the Cpfl protein is isolated or derived from Franciscella species or Acidaminococcus species. In some embodiments, the gNA (e.g., gRNA) CRISPR/Cas system protein-binding sequence comprises the following RNA sequence: (5'>3', AAUUUCUACUGUUGUAGAU).

In some embodiments, the CRISPR/Cas system protein is a Cpfl protein. In some embodiments, the Cpfl protein is isolated or derived from Franciscella species or Acidaminococcus species. In some embodiments, a DNA sequence encoding the gNA (e.g., gRNA) CRISPR/Cas system protein-binding sequence comprises the following DNA sequence: (5'>3', AATTTCTACTGTTGTAGAT). In some embodiments, the DNA is single stranded. In some embodiments, the DNA is double stranded.

In some embodiments, provided herein is a nucleic acid encoding for a gNA (e.g., gRNA) comprising a first segment comprising a regulatory region; a second segment encoding a targeting sequence; and a third segment comprising a nucleic acid encoding a nucleic acid-guided nuclease (e.g., CRISPR/Cas) system protein-binding sequence. In some embodiments, the third segment comprises a single transcribed component, which upon transcription yields a NA (e.g., RNA) stem-loop sequence. In some embodiments, the third segment comprising a single transcribed component that encodes for the gNA (e.g., gRNA) stem-loop sequence is double-stranded, comprises the following DNA sequence on one strand (5'>3',

GTTTTAGAGCTAGAAATAGCAAGTTAAAATAAGGCTAGTCCGTTATCAACTTGAAA AAGTGGCACCGAGTCGGTGCTTTTTTT), and its reverse-complementary DNA on the other strand (5'>3', AAAAAAAGCACCGACTCGGTGCCACTTTTTCAAGTTGATAACGGACTAGCCTTATTT TAACTTGCTATTTCTAGCTCTAAAAC). In some embodiments, the third segment comprising a single transcribed component that encodes for the gNA (e.g., gRNA) stem-loop sequence is single-stranded, and comprises the following DNA sequence: (5'>3', AAAAAAAGCACCGACTCGGTGCCACTTTTTCAAGTTGATAACGGACTAGCCTTATTT TAACTTGCTATTTCTAGCTCTAAAAC), wherein the single-stranded DNA serves as a transcription template. In some embodiments, upon transcription from the single transcribed component, the resulting gNA (e.g., gRNA) stem-loop sequence comprises the following RNA sequence: (5'>3', GUUUUAGAGCUAGAAAUAGCAAGUUAAAAUAAGGCUAGUCCGUUAUCAACUUGA AAAAGUGGCACCGAGUCGGUGCUUUUUUU). In some embodiments, the third segment comprising a single transcribed component that encodes for the gNA (e.g., gRNA) stem-loop sequence is double-stranded, comprises the following DNA sequence on one strand (5'>3', GTTTTAGAGCTATGCTGGAAACAGCATAGCAAGTTAAAATAAGGCTAGTCCGTTAT CAACTTGAAAAAGTGGCACCGAGTCGGTGCTTTTTTTC), and its reverse-complementary DNA on the other strand (5'>3', GAAAAAAAGCACCGACTCGGTGCCACTTTTTCAAGTTGATAACGGACTAGCCTTATT TTAACTTGCTATGCTGTTTCCAGCATAGCTCTAAAAC). In some embodiments, the third segment comprising a single transcribed component that encodes for the gNA (e.g., gRNA) stem-loop sequence is single-stranded, and comprises the following DNA sequence: (5'>3',

GAAAAAAAGCACCGACTCGGTGCCACTTTTTCAAGTTGATAACGGACTAGCCTTATT TTAACTTGCTATGCTGTTTCCAGCATAGCTCTAAAAC), wherein the single-stranded DNA serves as a transcription template. In some embodiments, upon transcription from the single transcribed component, the yielded gRNA stem-loop sequence comprises the following RNA sequence: (5'>3', GUUUUAGAGCUAUGCUGGAAACAGCAUAGCAAGUUAAAAUAAGGCUAGUCCGUU AUCAACUUGAAAAAGUGGCACCGAGUCGGUGCUUUUUUC). In some embodiments, the third segment comprises two sub-segments, which encode for a crRNA and a tracrRNA upon transcription. In some embodiment, the crRNA does not comprise the recognition site (e.g., N20 sequence) plus the extra sequence which can hybridize with tracrRNA. In some embodiments, the crRNA comprises the extra sequence which can hybridize with tracrRNA. In some embodiments, the two sub-segments are independently transcribed. In some embodiments, the two sub-segments are transcribed as a single unit. In some embodiments, the DNA encoding the crRNA comprises $N_{target}$-GTTTTAGAGCTATGCTGTTTG, where $N_{target}$ represents the targeting sequence. In some embodiments, the DNA encoding the tracrRNA comprises the sequence

GGAACCATTCAAAACAGCATAGCAAGTTAAAATAAGGCTAGTCCGTTATCAACTTG

AAAAAGTGGCACCGAGTCGGTGCTTTTTTT.

In some embodiments, a nucleic acid encoding for a gNA (e.g., gRNA) comprises a first segment comprising a regulatory region; a second segment encoding a targeting sequence; and a third segment comprising a nucleic acid encoding a nucleic acid-guided nuclease (e.g., CRISPR/Cas) system protein-binding sequence. In some embodiments, the third segment comprises a DNA sequence, which upon transcription yields a gRNA stem-loop sequence capable of binding a nucleic acid-guided nuclease (e.g., CRISPR/Cas) system protein. In one embodiment, the DNA sequence can be double-stranded. In some embodiments, the third segment double stranded DNA comprises the following DNA sequence on one strand (5'>3',

GTTTTAGAGCTAGAAATAGCAAGTTAAAATAAGGCTAGTCCGTTATCAACTTGAAA AAGTGGCACCGAGTCGGTGCTTTTTTT), and its reverse-complementary DNA on the other strand (5'>3', AAAAAAAGCACCGACTCGGTGCCACTTTTTCAAGTTGATAACGGACTAGCCTTATTT TAACTTGCTATTTCTAGCTCTAAAAC). In some embodiments, the third segment double stranded DNA comprises the following DNA sequence on one strand (5'>3', GTTTTAGAGCTATGCTGGAAACAGCATAGCAAGTTAAAATAAGGCTAGTCCGTTAT CAACTTGAAAAAGTGGCACCGAGTCGGTGCTTTTTTTC), and its reverse-complementary DNA on the other strand (5'>3', GAAAAAAAGCACCGACTCGGTGCCACTTTTTCAAGTTGATAACGGACTAGCCTTATT TTAACTTGCTATGCTGTTTCCAGCATAGCTCTAAAAC). In one embodiment, the DNA sequence can be single-stranded. In some embodiments, the third segment single stranded DNA comprises the following DNA sequence (5'>3', AAAAAAAGCACCGACTCGGTGCCACTTTTTCAAGTTGATAACGGACTAGCCTTATTT TAACTTGCTATTTCTAGCTCTAAAAC), wherein the single-stranded DNA serves as a transcription template. In some embodiments, the third segment single stranded DNA comprises the following DNA sequence (5'>3', GAAAAAAAGCACCGACTCGGTGCCACTTTTTCAAGTTGATAACGGACTAGCCTTATT TTAACTTGCTATGCTGTTTCCAGCATAGCTCTAAAAC), wherein the single-stranded DNA serves as a transcription template. In some embodiments, the third segment comprises a DNA sequence which, upon transcription, yields a first RNA sequence that is capable of forming a hybrid with a second RNA sequence, and which hybrid is capable of CRISPR/Cas system protein binding. In some embodiments, the third segment is double-stranded DNA comprising the DNA sequence on one strand: (5'>3', GTTTTAGAGCTATGCTGTTTG) and its reverse complementary DNA sequence on the other strand: (5'>3', CAAAACAGCATAGCTCTAAAAC). In some embodiments, the third segment is single-stranded DNA comprising the DNA sequence of (5'>3', CAAAACAGCATAGCTCTAAAAC). In some embodiments, the second segment and the third segment together encode for a crRNA sequence. In some embodiments, the second RNA sequence that is capable of forming a hybrid with the first RNA sequence encoded by the third segment of the nucleic acid encoding a gRNA is a tracrRNA. In some embodiments, the tracrRNA comprises the sequence (5'>3', GGAACCAUUCAAAACAGCAUAGCAAGUUAAAAUAAGGCUAGUCCGUUAUCAACU UGAAAAAGUGGCACCGAGUCGGUGCUUUUUUU). In some embodiments, the tracrRNA is encoded by a double-stranded DNA comprising sequence of (5'>3', GGAACCATTCAAAACAGCATAGCAAGTTAAAATAAGGCTAGTCCGTTATCAACTTG AAAAAGTGGCACCGAGTCGGTGCTTTTTTT), and optionally fused with a regulatory sequence at its 5' end. In some embodiments, the regulatory sequence can be bound by a transcription factor. In some embodiments, the regulatory sequence is a promoter. In some embodiments, the regulatory sequence is a T7 promoter, comprising the sequence of (5'>3',

GCCTCGAGCTAATACGACTCACTATAGAG). In some embodiments, the T7 promoter comprises a sequence of 5'-TAATACGACTCACTATAGG-3'. In some embodiments, the T7 promoter comprises a sequence of 5'- TAATACGACTCACTATAGGG-3'.

In some embodiments, a nucleic acid encoding for a gNA (e.g., gRNA) comprises a first segment comprising a regulatory region; a second segment encoding a targeting sequence; and a third segment comprising a nucleic acid encoding a nucleic acid-guided nuclease (e.g., CRISPR/Cas) system protein-binding sequence. In some embodiments, for example those embodiments wherein the CRISPR/Cas system protein is a Cpfl system protein, the first, second and third segments are arranged, from 5' to 3': first segment (regulatory region), third segment (nucleic acid-guided nuclease system protein-binding sequence), and second segment (targeting sequence). In some embodiments, the third segment comprises a single transcribed component, which upon transcription yields a NA (e.g., RNA) stem-loop sequence. In some embodiments, the third segment comprising a single transcribed component that encodes for the gNA (e.g., gRNA) stem-loop sequence is double-stranded, comprises the following DNA sequence on one strand (5'>3', AATTTCTACTGTTGTAGAT), and its reverse-complementary DNA on the other strand (5'>3', ATCTACAACAGTAGAAATT). In some embodiments, the third segment comprising a single transcribed component that encodes for the gNA (e.g., gRNA) stem-loop sequence is single-stranded, and comprises the following DNA sequence: (5'>3', ATCTACAACAGTAGAAATT), wherein the single-stranded DNA serves as a transcription template. In some embodiments, upon transcription from the single transcribed component, the resulting gNA (e.g., gRNA) stem-loop sequence comprises the following RNA sequence: (5'>3', AAUUUCUACUGUUGUAGAU).

In some embodiments, a nucleic acid encoding for a gNA comprises a first segment comprising a regulatory region; a second segment encoding a targeting sequence; and a third segment comprising a nucleic acid encoding a nucleic acid-guided nuclease (e.g., CRISPR/Cas) system protein-binding sequence. In some embodiments, the third segment encodes for a RNA sequence that, upon post-transcriptional cleavage, yields a first RNA segment and a second RNA segment. In some embodiments, the first RNA segment comprises a crRNA and the second RNA segment comprises a tracrRNA, which can form a hybrid and together, provide for nucleic acid-guided nuclease (e.g., CRISPR/Cas) system protein binding. In some embodiments, the third segment further comprises a spacer in between the transcriptional unit for the first RNA segment and the second RNA segment, which spacer comprises an enzyme cleavage site.
In some embodiments, a gNA (e.g., gRNA) comprises a first NA segment comprising a targeting sequence and a second NA segment comprising a nucleic acid-guided nuclease (e.g., CRISPR/Cas) system protein-binding sequence. In some embodiments, the size of the first segment is greater than 30 bp. In some embodiments, the second segment comprises a single segment, which comprises the gRNA stem-loop sequence. In some embodiments, the gRNA stem-loop sequence comprises the following RNA sequence: (5'>3', GUUUUAGAGCUAGAAAUAGCAAGUUAAAAUAAGGCUAGUCCGUUAUCAACUUGA AAAAGUGGCACCGAGUCGGUGCUUUUUUU). In some embodiments, the gRNA stem-loop sequence comprises the following RNA sequence: (5'>3', GUUUUAGAGCUAUGCUGGAAACAGCAUAGCAAGUUAAAAUAAGGCUAGUCCGUU AUCAACUUGAAAAAGUGGCACCGAGUCGGUGCUUUUUUUC). In some embodiments, the second segment comprises two sub-segments: a first RNA sub-segment (crRNA) that forms a hybrid with a second RNA sub-segment (tracrRNA), which together act to direct nucleic acid-guided nuclease (e.g., CRISPR/Cas) system protein binding. In some embodiments, the sequence of the second sub-segment comprises GUUUUAGAGCUAUGCUGUUUUG. In some embodiments, the first RNA segment and the second RNA segment together forms a crRNA sequence. In some embodiments, the other RNA that will form a hybrid with the second RNA segment is a tracrRNA. In some embodiments the tracrRNA comprises the sequence of 5'>3', GGAACCAUUCAAAACAGCAUAGCAAGUUAAAAUAAGGCUAGUCCGUUAUCAACU UGAAAAAGUGGCACCGAGUCGGUGCUUUUUUU.
In some embodiments, a gNA (e.g., gRNA) comprises a first NA segment comprising a targeting sequence and a second NA segment comprising a nucleic acid-guided nuclease (e.g., CRISPR/Cas) system protein-binding sequence. In some embodiments, for example those embodiments wherein the CRISPR/Cas system protein is a Cpfl system protein, the second segment is 5' of the first segment. In some embodiments, the size of the first segment is 20 bp. In some embodiments, the size of the first segment is greater than 20 bp. In some embodiments, the size of the first segment is greater than 30 bp. In some embodiments, the second segment comprises a single segment, which comprises the gRNA stem-loop sequence. In some embodiments, the gRNA stem-loop sequence comprises the following RNA sequence: (5'>3', AAUUUCUACUGUUGUAGAU).

CRISPR/Cas System Nucleic Acid-Guided Nucleases

In some embodiments, CRISPR/Cas system proteins are used in the embodiments provided herein. In some embodiments, CRISPR/Cas system proteins include proteins from CRISPR Type I systems, CRISPR Type II systems, and CRISPR Type III systems.

In some embodiments, CRISPR/Cas system proteins can be from any bacterial or archaeal species.

In some embodiments, the CRISPR/Cas system protein is isolated, recombinantly produced, or synthetic.

In some embodiments, the CRISPR/Cas system proteins are from, or are derived from CRISPR/Cas system proteins from *Streptococcus pyogenes, Staphylococcus aureus, Neisseria meningitidis, Streptococcus thermophiles, Treponema denticola, Francisella tularensis, Pasteurella multocida, Campylobacter jejuni, Campylobacter lari, Mycoplasma gallisepticum, Nitratifractor salsuginis, Parvibaculum lavamentivorans, Roseburia intestinalis, Neisseria cinerea, Gluconacetobacter diazotrophicus, Azospirillum, Sphaerochaeta globus, Flavobacterium columnare, Fluviicola taffensis, Bacteroides coprophilus, Mycoplasma mobile, Lactobacillus farciminis, Streptococcus pasteurianus, Lactobacillus johnsonii, Staphylococcus pseudintermedius, Filifactor alocis, Legionella pneumophila, Suterella wadsworthensis, Corynebacter diphtheria, Acidaminococcus, Lachnospiraceae bacterium* or *Prevotella.*

In some embodiments, examples of CRISPR/Cas system proteins can be naturally occurring or engineered versions.

In some embodiments, naturally occurring CRISPR/Cas system proteins can belong to CAS Class I Type I, III, or IV, or CAS Class II Type II or V, and can include Cas9, Cas3, Cas8a-c, Cas10, Cse1, Csy1, Csn2, Cas4, Csm2, Cmr5, Csf1, C2c2, and Cpfl.

In an exemplary embodiment, the CRISPR/Cas system protein comprises Cas9.

In an exemplary embodiment, the CRISPR/Cas system protein comprises Cpfl.

A "CRISPR/Cas system protein-gNA complex" refers to a complex comprising a CRISPR/Cas system protein and a guide NA (e.g. a gRNA or a gDNA). Where the gNA is a gRNA, the gRNA may be composed of two molecules, i.e., one RNA ("crRNA") which hybridizes to a target and provides sequence specificity, and one RNA, the "tracrRNA", which is capable of hybridizing to the crRNA. Alternatively, the guide RNA may be a single molecule (i.e., a gRNA) that contains crRNA and tracrRNA sequences. Alternatively, the guide RNA may be a single molecule (i.e. a gRNA) that comprises a crRNA sequence.

A CRISPR/Cas system protein may be at least 60% identical (e.g., at least 70%, at least 80%, or 90% identical, at least 95% identical or at least 98% identical or at least 99% identical) to a wild type CRISPR/Cas system protein. The CRISPR/Cas system protein may have all the functions of a wild type CRISPR/Cas system protein, or only one or some of the functions, including binding activity, nuclease activity, and nuclease activity.

The term "CRISPR/Cas system protein-associated guide NA" refers to a guide NA. The CRISPR/Cas system protein -associated guide NA may exist as isolated NA, or as part of a CRISPR/Cas system protein-gNA complex.

Cas9

In some embodiments, the CRISPR/Cas System protein nucleic acid-guided nuclease is or comprises Cas9. The Cas9 can be isolated, recombinantly produced, or synthetic.

Examples of Cas9 proteins that can be used in the embodiments herein can be found in F.A. Ran, L. Cong, W.X. Yan, D. A. Scott, J.S. Gootenberg, A.J. Kriz, B. Zetsche, O. Shalem, X. Wu, K.S. Makarova, E.V. Koonin, P.A. Sharp, and F. Zhang; "In vivo genome editing using Staphylococcus aureus Cas9," Nature 520, 186-191 (09 April 2015) doi:10.1038/nature14299.

In some embodiments, the Cas9 is a Type II CRISPR system derived from *Streptococcus pyogenes, Staphylococcus aureus, Neisseria meningitidis, Streptococcus thermophiles, Treponema denticola, Francisella tularensis, Pasteurella multocida, Campylobacter jejuni, Campylobacter lari, Mycoplasma gallisepticum, Nitratifractor salsuginis, Parvibaculum lavamentivorans, Roseburia intestinalis, Neisseria cinerea, Gluconacetobacter diazotrophicus, Azospirillum, Sphaerochaeta globus, Flavobacterium columnare, Fluviicola taffensis, Bacteroides coprophilus, Mycoplasma mobile, Lactobacillus farciminis, Streptococcus pasteurianus, Lactobacillus johnsonii, Staphylococcus pseudintermedius, Filifactor alocis, Legionella pneumophila, Suterella wadsworthensis,* or *Corynebacter diphtheria.*

In some embodiments, the Cas9 is a Type II CRISPR system derived from S. *pyogenes* and the PAM sequence is NGG located on the immediate 3' end of the target specific guide sequence. The PAM sequences of Type II CRISPR systems from exemplary bacterial species can also include: *Streptococcus pyogenes* (NGG), *Staph aureus* (NNGRRT), *Neisseria meningitidis* (NNNNGA TT), *Streptococcus thermophilus* (NNAGAA) and *Treponema denticola* (NAAAAC) which are all usable without deviating from the present invention.

In one exemplary embodiment, Cas9 sequence can be obtained, for example, from the pX330 plasmid (available from Addgene), re-amplified by PCR then cloned into pET30 (from EMD biosciences) to express in bacteria and purify the recombinant 6His tagged protein.

A "Cas9-gNA complex" refers to a complex comprising a Cas9 protein and a guide NA. A Cas9 protein may be at least 60% identical (e.g., at least 70%, at least 80%, or 90% identical, at least 95% identical or at least 98% identical or at least 99% identical) to a wild type Cas9 protein, e.g., to the *Streptococcus pyogenes* Cas9 protein. The Cas9 protein may have all the functions of a wild type Cas9 protein, or only one or some of the functions, including binding activity, nuclease activity, and nuclease activity.

The term "Cas9-associated guide NA" refers to a guide NA as described above. The Cas9-associated guide NA may exist isolated, or as part of a Cas9-gNA complex.

Non-CRISPR/Cas System Nucleic Acid-Guided Nucleases

In some embodiments, non-CRISPR/Cas system proteins are used in the embodiments provided herein.

In some embodiments, the non-CRISPR/Cas system proteins can be from any bacterial or archaeal species.

In some embodiments, the non-CRISPR /Cas system protein is isolated, recombinantly produced, or synthetic.

In some embodiments, the non-CRISPR /Cas system proteins are from, or are derived from *Aquifex aeolicus, Thermus thermophilus, Streptococcus pyogenes, Staphylococcus aureus, Neisseria meningitidis, Streptococcus thermophiles, Treponema denticola, Francisella tularensis, Pasteurella multocida, Campylobacter jejuni, Campylobacter lari, Mycoplasma gallisepticum, Nitratifractor salsuginis, Parvibaculum lavamentivorans, Roseburia intestinalis, Neisseria cinerea, Gluconacetobacter diazotrophicus, Azospirillum, Sphaerochaeta globus, Flavobacterium columnare, Fluviicola taffensis, Bacteroides coprophilus, Mycoplasma mobile, Lactobacillus farciminis, Streptococcus pasteurianus, Lactobacillus johnsonii, Staphylococcus pseudintermedius, Filifactor alocis, Legionella pneumophila, Suterella wadsworthensis, Natronobacterium gregoryi, or Corynebacter diphtheria.*

In some embodiments, the non-CRISPR /Cas system proteins can be naturally occurring or engineered versions.

In some embodiments, a naturally occurring non-CRISPR /Cas system protein is NgAgo (Argonaute from *Natronobacterium gregoryi*).

A "non-CRISPR /Cas system protein-gNA complex" refers to a complex comprising a non-CRISPR /Cas system protein and a guide NA (e.g. a gRNA or a gDNA). Where the gNA is a gRNA, the gRNA may be composed of two molecules, i.e., one RNA ("crRNA") which hybridizes to a target and provides sequence specificity, and one RNA, the "tracrRNA", which is capable of hybridizing to the crRNA. Alternatively, the guide RNA may be a single molecule (i.e., a gRNA) that contains crRNA and tracrRNA sequences.

A non-CRISPR /Cas system protein may be at least 60% identical (e.g., at least 70%, at least 80%, or 90% identical, at least 95% identical or at least 98% identical or at least 99% identical) to a wild type non-CRISPR /Cas system protein. The non-CRISPR /Cas system protein may have all the functions of a wild type non-CRISPR /Cas system protein, or only one or some of the functions, including binding activity, nuclease activity, and nuclease activity.

The term "non-CRISPR /Cas system protein-associated guide NA" refers to a guide NA. The non-CRISPR /Cas system protein -associated guide NA may exist as isolated NA, or as part of a non-CRISPR /Cas system protein-gNA complex.

**Cpfl**

In some embodiments, the CRISPR/Cas system protein nucleic acid-guided nuclease is or comprises a Cpfl system protein. Cpfl system proteins can be isolated, recombinantly produced, or synthetic.

Cpfl system proteins are Class II, Type V CRISPR system proteins. In some embodiments, the Cpfl protein is isolated or derived from *Francisella tularensis.* In some embodiments, the Cpfl protein is isolated or derived from *Acidaminococcus, Lachnospiraceae bacterium* or *Prevotella.*

Cpfl system proteins bind to a single guide RNA comprising a nucleic acid-guided nuclease system protein-binding sequence (e.g., stem-loop) and a targeting sequence. The Cpfl targeting sequence comprises a sequence located immediately 3' of a Cpfl PAM sequence in a target nucleic acid. Unlike Cas9, the Cpfl nucleic acid-guided nuclease system protein-binding sequence is located 5' of the targeting sequence in the Cpfl gRNA. Cpfl can also produce staggered rather than blunt ended cuts in a target nucleic acid. Following targeting of the Cpfl protein-gRNA protein complex to a target nucleic acid, Francisella derived Cpf1, for example, cleaves the target nucleic acid in a staggered fashion, creating an approximately 5 nucleotide 5' overhang 18-23 bases away from the PAM at the 3' end of the targeting sequence. In contrast, cutting by a wild type Cas9 produces a blunt end 3 nucleotides upstream of the Cas9 PAM.

An exemplary Cpfl gRNA stem-loop sequence comprises the following RNA sequence: (5'>3', AAUUUCUACUGUUGUAGAU).

A "Cpfl protein-gNA complex" refers to a complex comprising a Cpfl protein and a guide NA (e.g. a gRNA). Where the gNA is a gRNA, the gRNA may be composed of a single molecule, i.e., one RNA ("crRNA") which hybridizes to a target and provides sequence specificity.

A Cpfl protein may be at least 60% identical (e.g., at least 70%, at least 80%, or 90% identical, at least 95% identical or at least 98% identical or at least 99% identical) to a wild type Cpfl protein. The Cpfl protein may have all the functions of a wild type Cpfl protein, or only one or some of the functions, including binding activity and nuclease activity.

Cpfl system proteins recognize a variety of PAM sequences. Exemplary PAM sequences recognized by Cpfl system proteins include, but are not limited to TTN, TCN and TGN. Additional Cpfl PAM sequences include, but are not limited to TTTN. One feature of Cpfl PAM sequences is that they have a higher A/T content than the NGG or NAG PAM sequences used by Cas9 proteins. Target nucleic acids, for example, different genomes, differ in their percent G/C content. For example, the genome of the human malaria parasite *Plasmodium falciparum* is known to be A/T rich. Alternatively, protein coding sequences within a genome frequently have a higher G/C content than the genome as a whole. The ratio of A/T to G/C nucleotides in a target genome affects the distribution and frequency of a given PAM sequence in that genome. For example, A/T rich genomes may have fewer NGG or NAG sequences, while G/C rich genomes may have fewer TTN sequences. Cpfl system proteins expand the repertoire of PAM sequences available to the ordinarily skilled artisan, resulting superior flexibility and function of gRNA libraries.

**Catalytically Dead Nucleic Acid-Guided Nucleases**

In some embodiments, engineered examples of nucleic acid-guided nucleases include catalytically dead nucleic acid-guided nucleases (CRISPR/Cas system nucleic acid-guided nucleases or non-CRISPR/Cas system nucleic acid-guided nucleases). The term "catalytically dead" generally refers to a nucleic acid-guided nuclease that has inactivated nucleases, for example inactivated HNH and RuvC nucleases. Such a protein can bind to a target site in any nucleic acid (where the target site is determined by the guide NA), but the protein is unable to cleave or nick the nucleic acid.

Accordingly, the catalytically dead nucleic acid-guided nuclease allows separation of the mixture into unbound nucleic acids and catalytically dead nucleic acid-guided nuclease-bound fragments. In one exemplary embodiment, a dCas9/gRNA complex binds to the targets determined by the gRNA sequence. The dCas9 bound can prevent cutting by Cas9 while other manipulations proceed.

In another embodiment, the catalytically dead nucleic acid-guided nuclease can be fused to another enzyme, such as a transposase, to target that enzyme's activity to a specific site.

In some embodiments, the catalytically dead nucleic acid-guided nuclease is dCas9, dCpf1, dCas3, dCas8a-c, dCas10, dCse1, dCsy1, dCsn2, dCas4, dCsm2, dCm5, dCsf1, dC2C2, or dNgAgo.

In one exemplary embodiment the catalytically dead nucleic acid-guided nuclease protein is a dCas9.

Nucleic Acid-Guided Nuclease Nickases

In some embodiments, engineered examples of nucleic acid-guided nucleases include nucleic acid-guided nuclease nickases (referred to interchangeably as nickase nucleic acid-guided nucleases).

In some embodiments, engineered examples of nucleic acid-guided nucleases include CRISPR/Cas system nickases or non-CRISPR/Cas system nickases, containing a single inactive catalytic domain.

In some embodiments, the nucleic acid-guided nuclease nickase is a Cas9 nickase, Cpfl nickase, Cas3 nickase, Cas8a-c nickase, Cas10 nickase, Cse1 nickase, Csy1 nickase, Csn2 nickase, Cas4 nickase, Csm2 nickase, Cm5 nickase, Csf1 nickase, C2C2 nickase, or a NgAgo nickase.

In one embodiment, the nucleic acid-guided nuclease nickase is a Cas9 nickase.

In some embodiments, a nucleic acid-guided nuclease nickase can be used to bind to target sequence. With only one active nuclease domain, the nucleic acid-guided nuclease nickase cuts only one strand of a target DNA, creating a single-strand break or "nick". Depending on which mutant is used, the guide NA-hybridized strand or the non-hybridized strand may be cleaved. nucleic acid-guided nuclease nickases bound to 2 gNAs that target opposite strands can create a double-strand break in the nucleic acid. This "dual nickase" strategy increases the specificity of cutting because it requires that both nucleic acid-guided nuclease /gNA complexes be specifically bound at a site before a double-strand break is formed.

In exemplary embodiments, a Cas9 nickase can be used to bind to target sequence. The term "Cas9 nickase" refers to a modified version of the Cas9 protein, containing a single inactive catalytic domain, i.e., either the RuvC- or the HNH-domain. With only one active nuclease domain, the Cas9 nickase cuts only one strand of the target DNA, creating a single-strand break or "nick". Depending on which mutant is used, the guide RNA-hybridized strand or the non-hybridized strand may be cleaved. Cas9 nickases bound to 2 gRNAs that target opposite strands will create a double-strand break in the DNA. This "dual nickase" strategy can increase the specificity of cutting because it requires that both Cas9/gRNA complexes be specifically bound at a site before a double-strand break is formed.

Capture of DNA can be carried out using a nucleic acid-guided nuclease nickase. In one exemplary embodiment, a nucleic acid-guided nuclease nickase cuts a single strand of double stranded nucleic acid, wherein the double stranded region comprises methylated nucleotides.

**Dissociable and Thermostable Nucleic Acid-Guided Nucleases**

In some embodiments, thermostable nucleic acid-guided nucleases are used in the methods provided herein (thermostable CRISPR/Cas system nucleic acid-guided nucleases or thermostable non-CRISPR/Cas system nucleic acid-guided nucleases). In such embodiments, the reaction temperature is elevated, inducing dissociation of the protein; the reaction temperature is lowered, allowing for the generation of additional cleaved target sequences. In some embodiments, thermostable nucleic acid-guided nucleases maintain at least 50% activity, at least 55% activity, at least 60% activity, at least 65% activity, at least 70% activity, at least 75% activity, at least 80% activity, at least 85% activity, at least 90% activity, at least 95% activity, at least 96% activity, at least 97% activity, at least 98% activity, at least 99% activity, or 100% activity, when maintained for at least 75°C for at least 1 minute. In some embodiments, thermostable nucleic acid-guided nucleases maintain at least 50% activity, when maintained for at least 1 minute at least at 75°C, at least at 80°C, at least at 85°C, at least at 90°C, at least at 91°C, at least at 92°C, at least at 93°C, at least at 94°C, at least at 95°C, 96°C, at least at 97°C, at least at 98°C, at least at 99°C, or at least at 100°C. In some embodiments, thermostable nucleic acid-guided nucleases maintain at least 50% activity, when maintained at least at 75°C for at least 1 minute, 2 minutes, 3 minutes, 4 minutes, or 5 minutes. In some embodiments, a thermostable nucleic acid-guided nuclease maintains at least 50% activity when the temperature is elevated, lowered to 25°C-50°C. In some embodiments, the temperature is lowered to 25°C, to 30°C, to 35°C, to 40°C, to 45°C, or to 50°C In one exemplary embodiment, a thermostable enzyme retains at least 90% activity after 1 min at 95°C.

In some embodiments, the thermostable nucleic acid-guided nuclease is thermostable Cas9, thermostable Cpfl, thermostable Cas3, thermostable Cas8a-c, thermostable Cas10, thermostable Cse1, thermostable Csy1, thermostable Csn2, thermostable Cas4, thermostable Csm2, thermostable Cm5, thermostable Csf1, thermostable C2C2, or thermostable NgAgo.

In some embodiments, the thermostable CRISPR/Cas system protein is thermostable Cas9.

Thermostable nucleic acid-guided nucleases can be isolated, for example, identified by sequence homology in the genome of thermophilic bacteria Streptococcus thermophilus and Pyrococcus furiosus. Nucleic acid-guided nuclease genes can then be cloned into an expression vector. In one exemplary embodiment, a thermostable Cas9 protein is isolated.

In another embodiment, a thermostable nucleic acid-guided nuclease can be obtained by *in vitro* evolution of a non-thermostable nucleic acid-guided nuclease. The sequence of a nucleic acid-guided nuclease can be mutagenized to improve its thermostability.

### *Methods of Makins Collections of gNAs*

Provided herein are methods that enable the generation of a large number of diverse gRNAs, collections of gNAs, from any source nucleic acid (e.g., DNA). Methods provided herein can employ enzymatic methods including but not limited to digestion, ligation, extension, overhang filling, transcription, reverse transcription, amplification.

Generally, the method can comprise providing a nucleic acid (e.g., DNA); employing a first enzyme (or combinations of first enzymes) that cuts at a part of the PAM sequence in the nucleic acid, in a way that a residual nucleotide sequence from the PAM sequence is left; ligating an adapter that positions a restriction enzyme type IIS site (an enzyme that cuts outside yet near its recognition motif) at a distance to eliminate the PAM sequence; employing a second type IIS enzyme (or combination of second enzymes) to eliminate the PAM sequence together with the adapter; and fusing a sequence that can be recognized by protein members of the nucleic acid-guided nuclease (e.g., CRISPR/Cas) system, for example, a gRNA stem-loop sequence. In some embodiments, the first enzymatic reactions cuts part of the PAM sequence in a way that residual nucleotide sequence from the PAM sequence is left, and that the nucleotide sequence immediately 5' to the PAM sequence can be any purine or pyrimidine, not just those with a cytosine 5' to the PAM sequence, for example, not just those that are C/NGG or C/TAG, etc.

Table 1 shows exemplary strategies/protocols to convert any source nucleic acid (e.g., DNA) into a collection of gNAs (e.g., gRNAs) using different restriction enzymes.

**Table 1**. Exemplary strategies for preparing a collection of guide nucleic acids.

| CRISPR/Cas System Species | PAM Sequence | First Enzyme/ Components | Strategy | 3' Adapter sequence with type IIS enzyme site (provided with only one strand sequence 5'>3') |
|---|---|---|---|---|
| Streptococcus pyogenes (SP); SpCas9 | NGG | CviPII | Nicks immediately 5' of CCD sequence, nicks the other strand with T7 endonuclease I, blunt with T4 DNA polymerase; ligate to adapter; cut with MlyI to remove PAM and adapter; ligate gRNA stem-loop sequence at 3' end | ggGACTCggatccctatagtc |
| Staphylococcus aureus (SA); SaCas9 | NNGRRT or NNGRR(N) | AlwI | Cut, blunt with T4 DNA polymerase; ligate to adapter SA; cut with Mme I or EcoP15I to remove PAM and adapter; blunt end; ligate gRNA stem-loop sequence at 3' end | ttttagcggccgcctgctgCTCt acaaagacgatgacgacaagcgt |
| Neisseria meningitidis (NM) | NNNNGATT | TfiI | Cut, blunt with T4 DNA polymerase; ligate to adapter NM; cut with EcoRI to eliminate unwanted DNA and MmeI or EcoP15I to remove PAM and adapter; blunt end; ligate gRNA stem-loop sequence at 3' end | TCgcggccgcttttattctgctgC TCtacaaagacgatgacgacaa gcgt |
| Streptococcus thermophilus (ST) | NNAGAAW | BsmI | Cut, blunt with T4 DNA polymerase; ligate to adapter ST; cut with MmeI or EcoP15I to remove PAM and adapter; blunt end; ligate gRNA stem-loop sequence at 3' end | ttgcggccgcttttattctgctgCT Ctacaaagacgatgacgacaagc gt |
| Treponema denticola (TD) | NAAAAC | Cly7489II | Cut, blunt with T4 DNA polymerase; ligate to adapter TD; cut with MmeI or EcoP15I to remove PAM and adapter | tttagcggccgcctgctgCTCta caaagacgatgacgacaagcgt |

Table 2 shows additional exemplary strategies/protocols to convert any source nucleic acid (e.g., DNA) into a collection of gNAs (e.g., gRNAs) using different restriction enzymes.

**Table 2.** Additional exemplary strategies for preparing a collection of guide nucleic acids.

| CRISPR/ Cas System Species | PAM Sequence | First Enzyme/ Component | Exemplary Strategy | Adapter oligo sequence (with Inosine overhangs, all in 5'>3' direction) |
|---|---|---|---|---|
| Streptococcus pyogenes (SP); SpCas9 | NGG | CviPII | Nicks immediately 5' of CCD sequence, nicks the other strand with T7 endonuclease I; ligate to adapter; cut with MlyI to remove PAM and 3' adapter; ligate gRNA stem-loop sequence at 3' end | Adapter oligo 1: ggggGACTCggatccctatagtg atacaaagacgatgacgacaagcg <br> Adapter oligo 2: gcctcgagc*t*a*atacgactcact atagggatccaagtccc <br> (* denotes a phosphorothioate backbone linkage) |
| Staphylococcus aureus (SA); SaCas9 | NNGRRT or NNGRR(N) | AlwI | Cut; ligate to adapter SA; cut with MmeI or EcoP15I to remove PAM and 3' adapter; blunt end; ligate gRNA stem-loop sequence at 3' end | Adapter oligo 1: ItttttagcggccgcctgctgCTCtac aaagacgatgacgacaagcgt <br> Adapter oligo 2: gagatcagcttctgcattgatgcGA Gcagcaggcggccgctaaaa |
| Neisseria meningitidis (NM) | NNNNGATT | TfiI | Cut; ligate to adapter NM; cut with Mme I or EcoP15I to remove PAM and 3' adapter; blunt end; ligate gRNA stem-loop sequence at 3' end | Adapter oligo 1: attTCgcggccgcttttattctgctgC TCtacaaagacgatgacgacaagc gt <br> Adapter oligo 2: gagatcagcttctgcattgatgcGA GcagcagaataaaagcggccgcG A |
| Streptococcus thermophilus (ST) | NNAGAAW | BsmI | Cut; ligate to adapter ST; cut with MmeI or EcoP15I to remove PAM and 3' adapter; blunt end; ligate gRNA stem-loop sequence at 3' end | Adapter oligo 1: gcggccgcttttattctgctgCTCta caaagacgatgacgacaagcgt <br> Adapter oligo 2: gagatcagcttctgcattgatgcGA GcagcagaataaaagcggccgcIG |

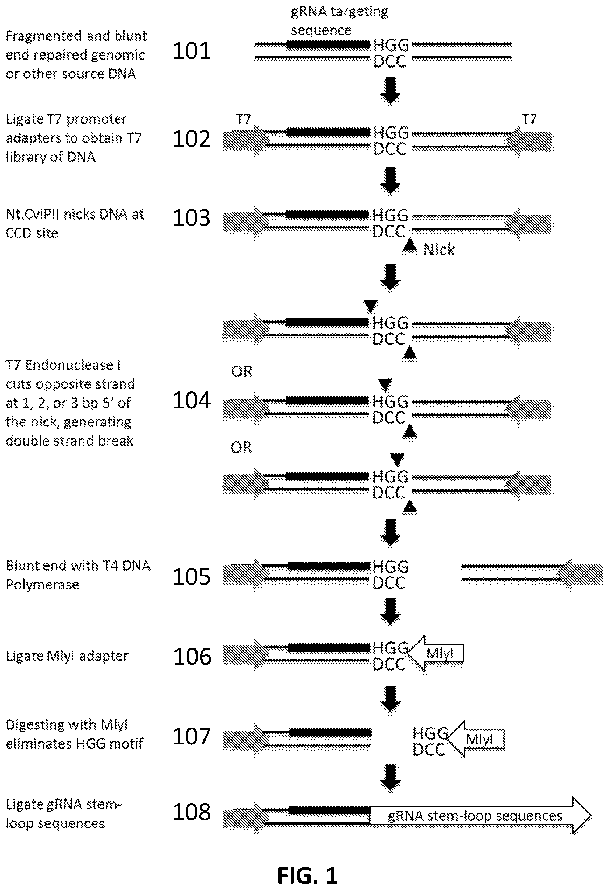

Exemplary applications of the compositions and methods described herein are provided in **FIG. 1, FIG. 2**, **FIG. 3**, **FIG. 4**, **FIG. 5**, and **FIG. 6**. The figures depict non-limiting exemplary embodiments of the present invention that includes a method of constructing a gNA library (e.g., gRNA library) from input nucleic acids (e.g., DNA), such as genomic DNA (e.g., human genomic DNA). Many of the protocols herein are described for example with reference to a PAM site of NGG or HGG, with a complementary sequence ('MAP' site) of CCD. These examples are non-limiting, and other PAM sites for various nucleic acid-guided nucleases are contemplated. Likewise, exemplary restriction enzymes described with the methods herein can be substituted for other restriction enzymes compatible with other PAM sequences.

In **FIG. 1**, the starting material can be fragmented genomic DNA (e.g., human) or other source DNA. These fragments are blunt-ended before constructing the library **101**. T7 promoter adapters are ligated to the blunt-ended DNA fragments **102**, which is then PCR amplified. Nt.CviPII is then used to generate a nick on one strand of the PCR product immediately 5' to the CCD sequence **103**. T7 Endonuclease I cleaves on the opposite strand 1, 2, or 3 bp 5' of the nick **104**. The resulting DNA fragments are blunt-ended with T4 DNA Polymerase, leaving HGG sequence at the end of the DNA fragment **105**. The resulting DNA is cleaned and recovered on beads. An adapter carrying MlyI recognition site is ligated to the blunt-ended DNA fragment immediately 3' of HGG sequence **106**. MlyI generates a blunt-end cleavage immediately 5' to the HGG sequence, removing HGG together with the adapter sequence **107**. The resulting DNA fragments are cleaned and recovered again on beads. A gRNA stem-loop sequence is then ligated to the blunt-end cleaved by MlyI, forming a gRNA library covering the human genome **108**. This library of DNA is then PCR amplified and cleaned on beads, ready for *in vitro* transcription.

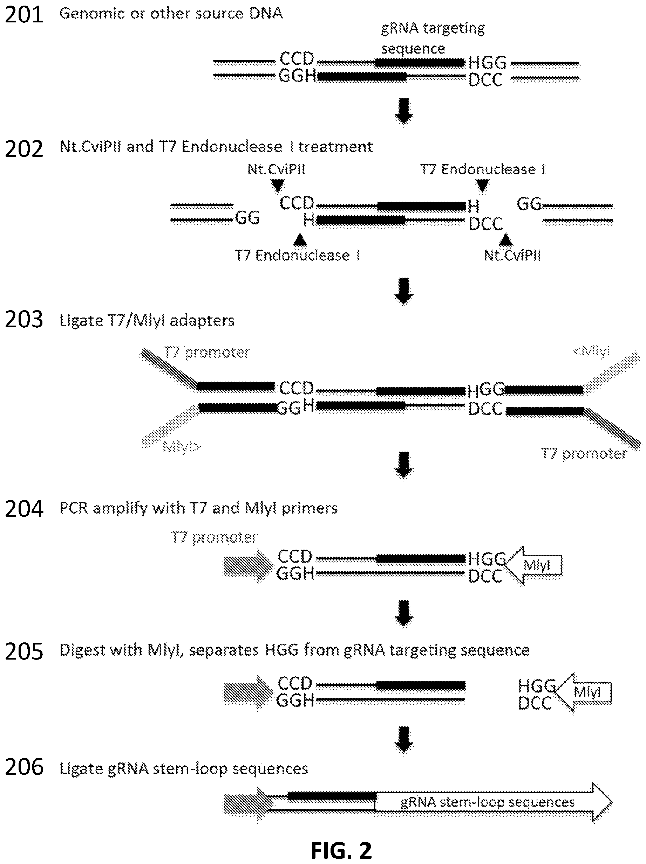

In **FIG. 2**, the starting material can intact genomic DNA (e.g., human) or other source DNA **201**. Nt.CviPII and T7 Endonuclease I are used to generate nicks on each strand of the human genomic DNA, resulting in smaller DNA fragments **202**. DNA fragments of 200-600 bp are size selected on beads, then ligated with Y-shaped adapters carrying a GG overhang on the 5'. One strand of the Y-shaped adapter contains a MlyI recognition site, wherein the other strand contains a mutated MlyI site and a T7 promoter sequence **203**. Because of these features, after PCR amplification, the T7 promoter sequence is at the distal end of the HGG sequence, and the MlyI sequence is at the rear end of HGG **204**. Digestion with MlyI generates a cleavage immediately 5' of HGG sequence **205**. MlyI generates a blunt-end cleavage immediately 5' to the HGG sequence, removing HGG together with the adapter sequence **206**. A gRNA stem-loop sequence is then ligated to the blunt-end cleaved by MlyI, forming a gRNA library covering the human genome. This library of DNA is then PCR amplified and cleaned on beads, ready for *in vitro* transcription.

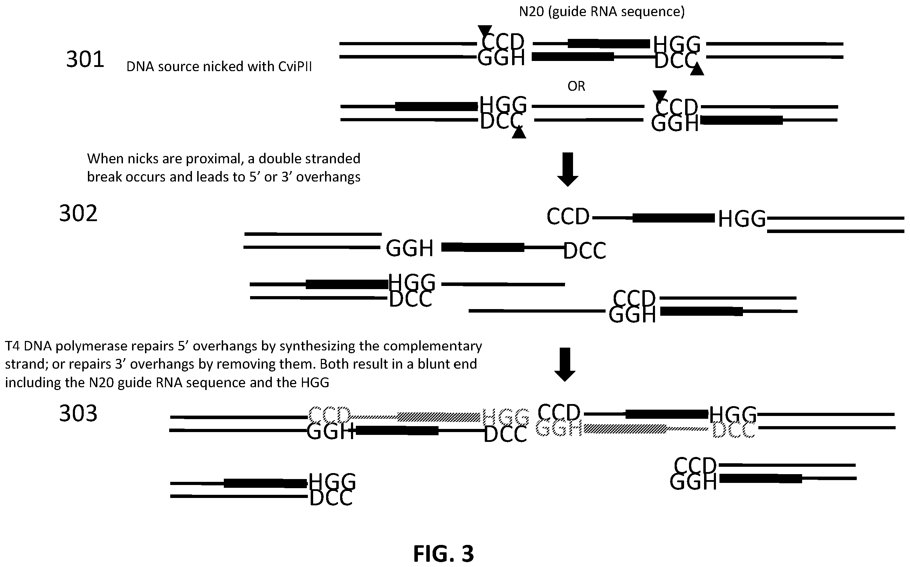

In **FIG. 3**, the source DNA (e.g., genomic DNA) can be nicked **301**, for example with a nicking enzyme. In some cases, the nicking enzyme can have a recognition site that is three or fewer bases in length. In some cases, CviPII is used, which can recognize and nick at a sequence of CCD (where D represents a base other than C). Nicks can be proximal, surrounding a region containing the sequence (represented by the thicker line) which will be used to yield the guide RNA recognition site (e.g., N20 sequence). When nicks are proximal, a double stranded break can occur and lead to 5' or 3' overhangs **302**. These overhangs can be repaired, for example with a polymerase (e.g., T4 polymerase). In some cases, such as with 5' strands, repair can comprise synthesizing a complementary strand. In some case, such as with 3' strands, repair can comprise removing overhangs. Repair can result in a blunt end including the recognition site (e.g., N20 guide sequence) and a sequence complementary to the nick recognition sequence (e.g., HGG, where H represents a base other than G).

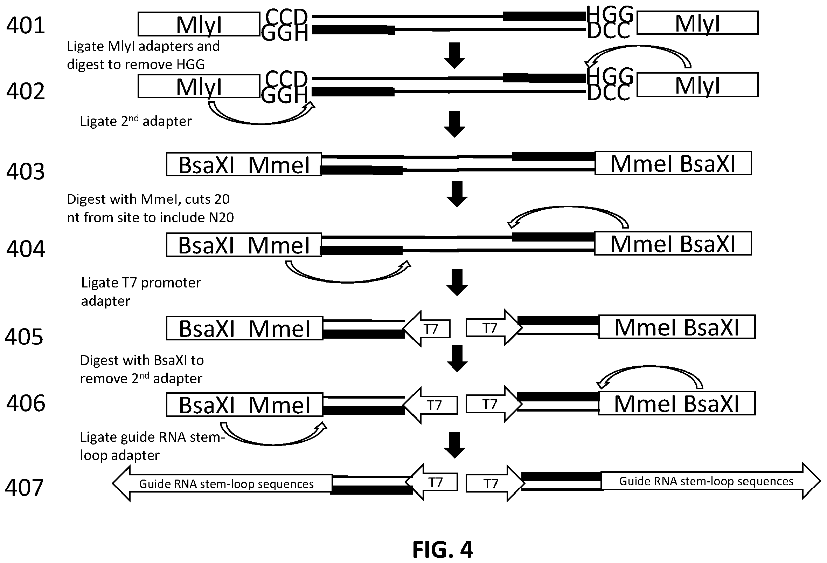

In **FIG. 4**, continuing for example from the end of **FIG. 3**, different combinations of adapters can be ligated to the DNA to allow for the desired cleaving. Adapters with a recognition site for a nuclease enzyme that cuts 3 base pairs from the site (e.g., MlyI) can be ligated **401**, and digestion at that site can be used to remove a left over sequence, such as an HGG sequence **402**. Adapters with a recognition site for a nuclease that cuts 20 base pairs from the site (e.g., MmeI) **403**. These adapters can also include a second recognition site for a nuclease that cuts the proper number of nucleotides from the site to later remove the first recognition site (e.g., BsaXI). The first enzyme can be used to cut 20 nucleotides down, thereby keeping the recognition site (e.g., N20 sequence) **404**. Then, a promoter adapter (e.g., T7) can be ligated next to the recognition site (e.g., N20 sequence) **405**. Then, the nuclease corresponding to the second recognition site (e.g., BsaXI) can be used to remove the adapter for the site that cuts 20 nucleotides away (e.g., MmeI) **406**. Finally, the guide RNA stem-loop sequence adapter can be ligated to the recognition site (e.g., N20 sequence) **407** to prepare for guide RNA production.

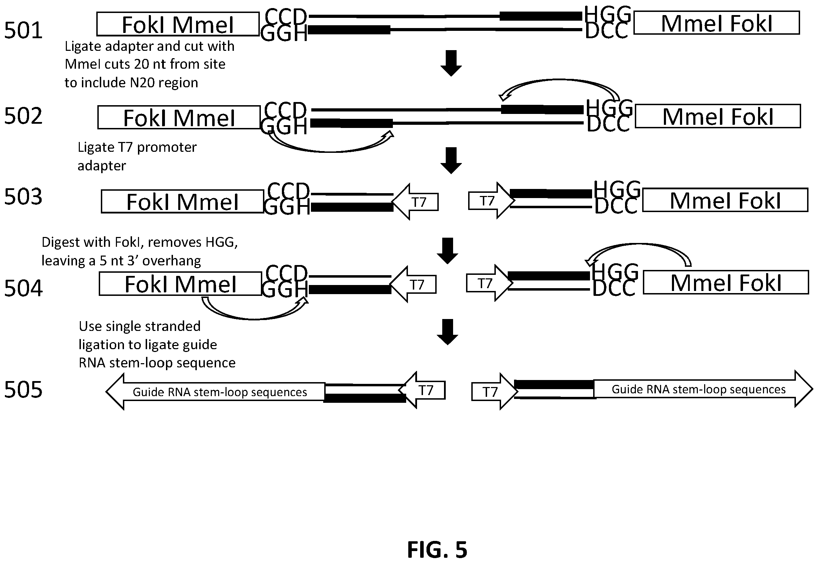

Alternatively, the protocol shown in **FIG. 5** can follow the end of a protocol such as that shown in **FIG. 3**. Adapters with a recognition site for a nuclease enzyme that cleaves 25 nucleotides from the site (e.g., MmeI or EcoP15I) can be ligated to the DNA **501**. These adapters can also include a second recognition site for a nuclease that cuts the proper number of nucleotides (or more) from the site to later remove the first recognition site (e.g., FokI or BaeI) and any other left-over sequence, such as HGG. The enzyme corresponding to the first recognition site (e.g., MmeI or EcoP15I) can then be used to cleave after the recognition site (e.g., N20 sequence) **502**. Then, a promoter adapter (e.g., T7) can be ligated next to the recognition site (e.g., N20 sequence) **503**. The enzyme corresponding to the second recognition site (e.g., FokI or BaeI) can then be used to remove the recognition sites and any residual sequence (e.g., HGG) **504**. Finally, the guide RNA stem-loop sequence adapter can be ligated (e.g., by single strand ligation) to the recognition site (e.g., N20 sequence) **505**.

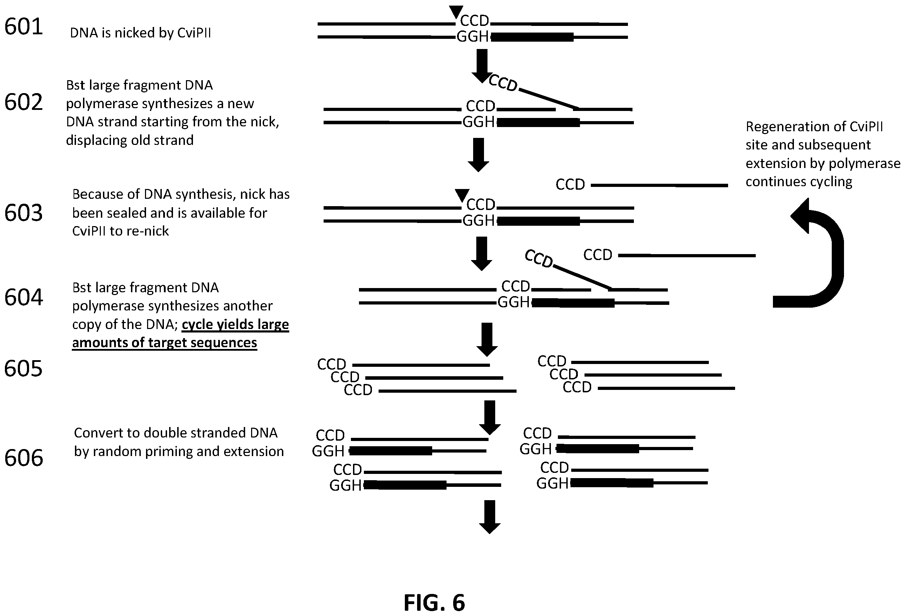

As an alternative to protocols such as that shown in **FIG. 3**, the protocol shown in **FIG. 6** can be used in preparation for protocols such as those shown in **FIG. 4** or **FIG. 5**. A nick can be introduced by a nicking enzyme (e.g., CviPII) **601**. In some cases, the nick recognition site is three or fewer bases in length. In some cases, CviPII is used, which can recognize and nick at a sequence of CCD. A polymerase (e.g., Bst large fragment DNA polymerase) can then be used to synthesize a new DNA strand starting from the nick while displacing the old strand **602**. Because of the DNA synthesis, the nick can be sealed and made available to be nicked again **603**. Subsequent cycles of nicking and synthesis can be used to yield large amounts of target sequences **604**. These single stranded copies of target sequences can be made double stranded, for example by random priming and extension. These double stranded nucleic acids comprising recognition site (e.g., N20 sequences) can then be further processed by methods disclosed herein, such as those shown in **FIG. 4** or **FIG. 5**.

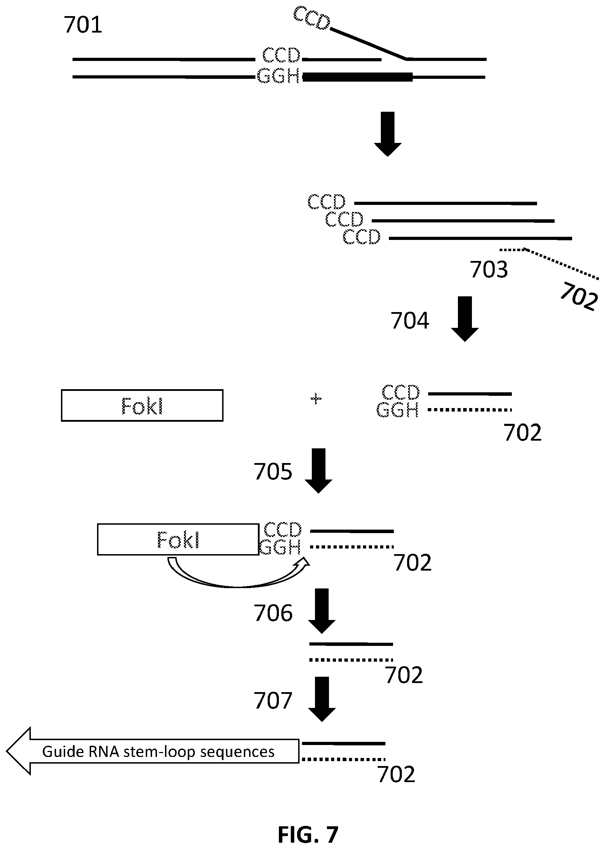

As another alternative to protocols such as that shown in **FIG. 3** or **FIG. 6**, the protocol shown in **FIG. 7** can be used in preparation for protocols such as those shown in **FIG. 4** or **FIG. 5**. A nick can be introduced by a nicking enzyme (e.g., CviPII) **701**. In some cases, the nicking enzyme recognition site is three or fewer bases in length. In some cases, CviPII is used, which can recognize and nick at a sequence of CCD. A polymerase (e.g., Bst large fragment DNA polymerase) can then be used to synthesize a new DNA strand starting from the nick while displacing the old strand (e.g., nicking endonuclease-mediated strand-displacement DNA amplification (NEMDA)). The reaction parameters can be adjusted to control the size of the single stranded DNA produced. For example, the nickase:polymerase ratio (e.g., CviPII:Bts large fragment polymerase ratio) can be adjusted. Reaction temperature can also be adjusted. Next, an oligonucleotide can be added **704** which has (in the 5'>3' direction) a promoter (e.g., T7 promoter) **702** followed by a random n-mer (e.g., random 6-mer, random 8-mer) **703**. The random n-mer region can bind to a region of the single stranded DNA generated previously. For example, binding can be conducted by denaturing at high temperature followed by rapid cool down, which can allow the random n-mer region to bind to the single stranded DNA generated by NEMDA. In some cases, the DNA is denatured at 98 °C for 7 minutes then cooled down rapidly to 10 °C. Extension and/or amplification can be used to produce double-stranded DNA. Blunt ends can be produced, for example enzymatically (e.g., by treatment with DNA polymerase I at 20 °C). This can result in one end ending at the promoter (e.g., T7 promoter) and the other end ending at any nicking enzyme recognition sites (e.g., any CCD sites). These fragments can then be purified, for example by size selection (e.g., by gel purification, capillary electrophoresis, or other fragment separation techniques). In some cases, the target fragments are about 50 base pairs in length (adapter sequence (e.g., T7 adapter) + target recognition (e.g., N20) sequence + nicking enzyme recognition site or complement (e.g., HGG)). Fragments can then be ligated to an adapter comprising a nuclease recognition site for a nuclease that cuts an appropriate distance away to remove the nicking enzyme recognition site **705**. For example, for a three-nucleotide long nicking enzyme recognition site (e.g., CCD for CviPII), BaeI can be used. Restriction enzymes that cut a little farther away from the recognition site can also be used, such as FokI. The appropriate nuclease (e.g., FokI or BaeI) can then be used to remove the nuclease recognition site and the nicking enzyme recognition site **706**. The remaining nucleic acid sequence (e.g., the recognition site) can then be ligated to the final stem-loop sequence for the guide RNA **707**. Amplification (e.g., PCR) can be conducted. Guide RNAs can be produced.

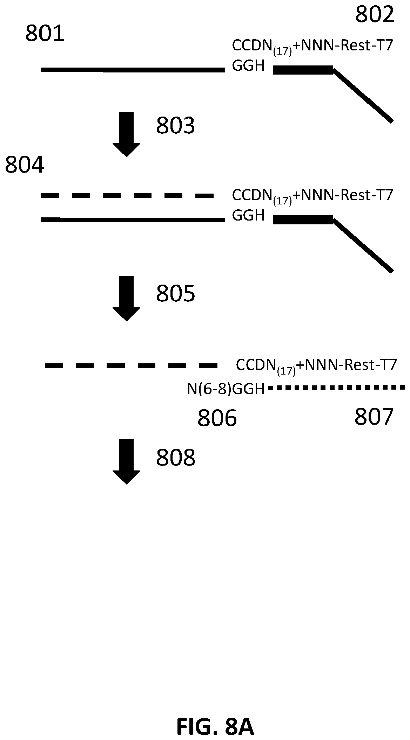

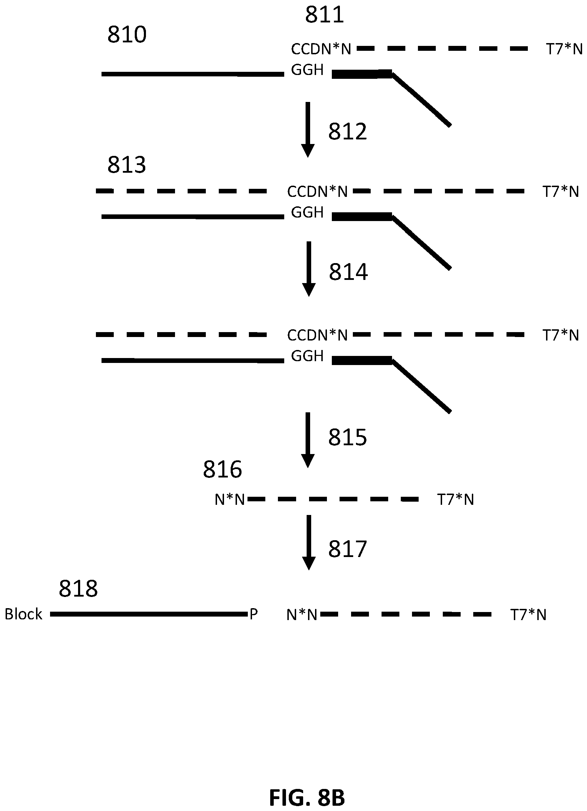

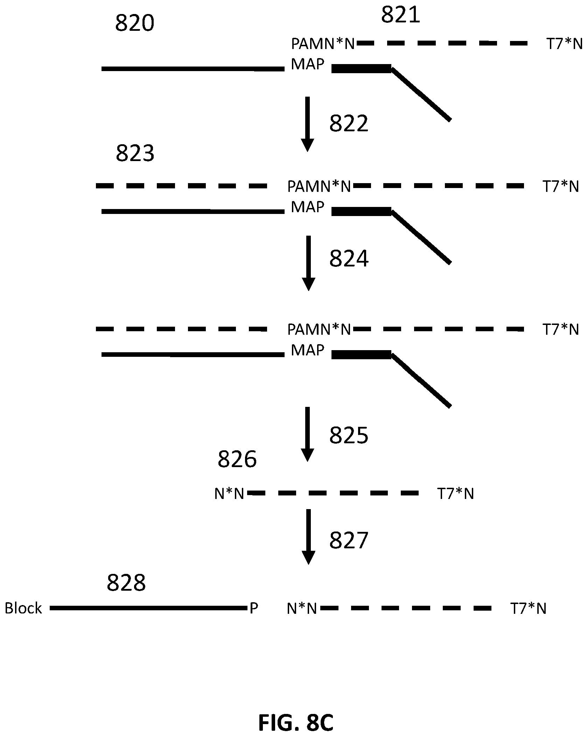

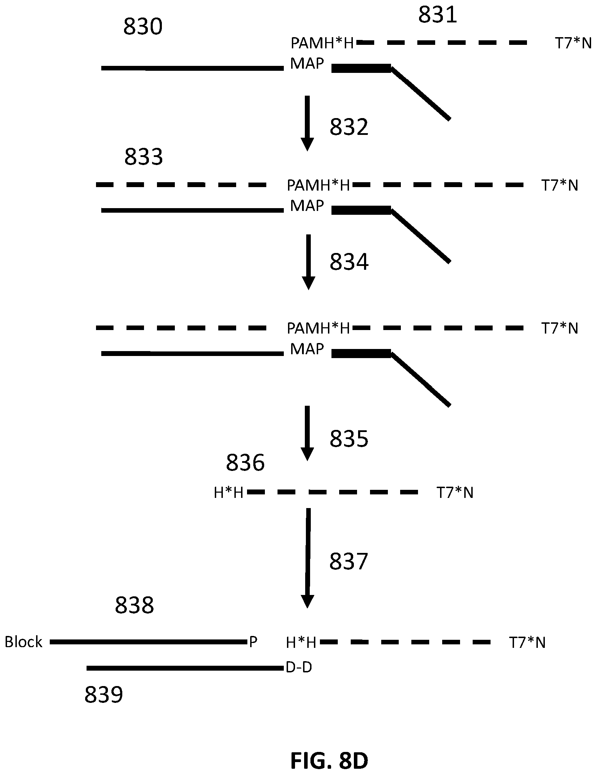

FIG. **8A**, **FIG. 8B**, **FIG. 8C**, and **FIG. 8D** show additional techniques for constructing a gNA library (e.g., gRNA library) from input nucleic acids (e.g., DNA), such as genomic DNA (e.g., human genomic DNA, reverse transcribed cDNA such as from mRNA).

**FIG. 8A** shows a protocol beginning with nucleic acid fragments **801** such as sheared genomic DNA or cDNA reverse-transcribed from mRNA. Primers **802** can then be hybridized to GGH locations or other PAM sites. The primers can comprise the sequence MAP-Recognition-Restriction-Promoter, where MAP represents the complement to a PAM site of a nucleic acid-guided nuclease, Recognition represents a recognition site of a nucleic acid-guided nuclease, Restriction represents a restriction enzyme recognition site, and Promoter represents a promoter site. The recognition site of the nucleic acid-guided nuclease can be an appropriate length for a given nucleic acid-guided nuclease (e.g., between about 15 and about 25 nucleotides, in some cases 20 nucleotides). In an example, primers can comprise the sequence $CCDN_{(17)}$+NNN-Rest-T7 or its complement, where CCD represents a MAP site, + represents a modified nucleic acid bond, Rest represents an appropriate restriction site, and T7 represents a T7 promoter site or other appropriate promoter site. The N nucleic acid sequences can be generated randomly, with each primer hybridizing to nucleic acid fragments which comprise sequences that match their random N-mer segments. The position of the modified nucleic acid can be varied within the primer, and more than one modified nucleic acid site can be used. Modified nucleic acids can comprise locked nucleic acid (LNA), bridged nucleic acid (BNA), peptide nucleic acid (PNA), zip nucleic acid (ZNA), glycol nucleic acid (GNA), threose nucleic acid (TNA), and other modified nucleic acids such as those with increased binding specificity or sensitivity. An extension reaction **803** can then be conducted to extend the primers **804**, incorporating sequence complementary to the nucleic acid fragment. Then reverse priming **805** can be conducted with a strand-displacing polymerase, extending reverse primers **806** to incorporate sequence **807** complementary to the first primers, including for example the restriction enzyme recognition sites and T7 (or other promoter) sites. The reverse primers can comprise the sequence $N_{(6-8)}$GGH. The length of the reverse primer can depend on restriction enzyme (e.g., MmeI) activity at the end of the fragment. These products can then be further processed to produce guide nucleic acids (e.g., gRNAs) as discussed herein, for example as discussed with respect to **FIG. 4** or **FIG. 7.**

**FIG. 8B** shows a protocol beginning with nucleic acid fragments **810** such as sheared genomic DNA or cDNA reverse-transcribed from mRNA. Primers **811** can then be hybridized to GGH locations or other PAM sites. The primers can comprise the sequence MAP-Recognition-Promoter, where MAP represents the complement to a PAM site of a nucleic acid-guided nuclease, Recognition represents a recognition site of a nucleic acid-guided nuclease, and Promoter represents a promoter site. The recognition site of the nucleic acid-guided nuclease can be an appropriate length for a given nucleic acid-guided nuclease (e.g., between about 15 and about 25 nucleotides, in some cases 20 nucleotides). In an example, the primers can comprise the sequence $CCDN*N_{(16)}$+$N_{(3)}$-T7*N or its complement, where + represents a modified nucleic acid bond, * represents a phosphorothioate (PTO) nucleic acid bond, and T7 represents a T7 promoter site or other appropriate promoter site. In an example, the primers can comprise the sequence $CCDH*H_{(4)}N_{(12)}$+$N_{(3)}$-T7*N. Modified nucleic acids can comprise locked nucleic acid (LNA), bridged nucleic acid (BNA), peptide nucleic acid (PNA), zip nucleic acid (ZNA), glycol nucleic acid (GNA), threose nucleic acid (TNA), and other modified nucleic acids such as those with increased binding specificity or sensitivity. Contemplated primer variations can include more modified and/or PTO nucleic acids. Use of PTO can protect products of interest (e.g., guide nucleic acids) from degradation by exonu-

cleaves. The primers can then be extended **812** to incorporate sequence **813** complementary to the nucleic acid fragment. In some cases, the extension can be conducted using labeled nucleotides (e.g., biotinylated uracil) for later purification. Next, the unextended or unbound primers can be removed **814**. In some cases, the primers can be removed by capturing extension products incorporating labels (e.g., using streptavidin to capture biotinylated nucleotides). In some cases, the primers can be removed by size selection (e.g., electrophoresis, solid phase reversible immobilization (SPRI) beads). In some cases, the primers can be removed by a combination of methods, such as capturing and size selection. Next, the nucleic acids can be nicked, such as with CviPII enzymes, and digested **815**, such as with single stranded exonuclease (e.g., both 5' to 3' and 3' to 5' exonuclease). This can leave single stranded products **816**, which can comprise sequence complementary to that adjacent to the GGH site or other PAM site on the nucleic acid fragments, as well as a T7 site or other appropriate promoter site. Next, ligation **817** can be used to ligate a 5' stemloop with a 3' block **818** to the single stranded products. These products can then be transcribed (e.g., using the T7 site or other appropriate promoter site) to produce guide nucleic acids (e.g., gRNAs).

**FIG. 8C** shows a protocol beginning with nucleic acid fragments **820** such as sheared genomic DNA or cDNA reverse-transcribed from mRNA. Primers **821** can then be hybridized to locations complementary to protospacer adjacent motifs (PAMs), indicated in the figure by 'MAP'. The primers can comprise the sequence PAM-Recognition-Promoter, where PAM represents the PAM site of a nucleic acid-guided nuclease, Recognition represents a recognition site of a nucleic acid-guided nuclease, and Promoter represents a promoter site. The recognition site of the nucleic acid-guided nuclease can be an appropriate length for a given nucleic acid-guided nuclease (e.g., between about 15 and about 25 nucleotides, in some cases 20 nucleotides). In an example, the primers can comprise the sequence PAM-$N^*N_{(16)}+N_{(3)}$-T7*N or its complement, where + represents a modified nucleic acid bond, * represents a phosphorothioate (PTO) nucleic acid bond, PAM represents a protospacer adjacent motif, and T7 represents a T7 site or other appropriate promoter site. Modified nucleic acids can comprise locked nucleic acid (LNA), bridged nucleic acid (BNA), peptide nucleic acid (PNA), zip nucleic acid (ZNA), glycol nucleic acid (GNA), threose nucleic acid (TNA), and other modified nucleic acids such as those with increased binding specificity or sensitivity. In an example, the primers can comprise the sequence PAM-$H^*H_{(4)}N_{(12)}+N_{(3)}$-T7*N. The H*H region can also be replaced by non-PAM sequence. Contemplated primer variations can include more modified and/or PTO nucleic acid bonds. Contemplated primer variations also include different lengths of random nucleotides (for example, between about 15 and about 25 nucleotides). The primers can then be extended **822** to incorporate sequence **823** complementary to the nucleic acid fragment. In some cases, the extension can be conducted using labeled nucleotides (e.g., biotinylated uracil) for later purification. Next, the unextended or unbound primers can be removed **824**. In some cases, the primers can be removed by capturing extension products incorporating labels (e.g., using streptavidin to capture biotinylated nucleotides). In some cases, the primers can be removed by size selection (e.g., electrophoresis, solid phase reversible immobilization (SPRI) beads). Next, the nucleic acids can be nicked, such as with CviPII enzymes or with a uracil-specific excision enzyme (e.g., USER or uracil DNA glycosylase (UDG)), and digested **825**, such as with single stranded exonuclease (e.g., both 5' to 3' and 3' to 5' exonuclease). This can leave single stranded products **826**, which can comprise sequence complementary to that adjacent to the GGH site or other PAM site on the nucleic acid fragments, as well as a T7 site or other appropriate promoter site. Next, ligation **827** can be used to ligate a 5' stemloop with a 3' block **828** to the single stranded products. These products can then be transcribed (e.g., using the T7 site or other appropriate promoter site) to produce guide nucleic acids (e.g., gRNAs).

**FIG. 8D** shows a protocol beginning with nucleic acid fragments **830** such as sheared genomic DNA or cDNA reverse-transcribed from mRNA. Primers **831** can then be hybridized to locations complementary to protospacer adjacent motifs (PAMs), indicated in the figure by 'MAP'. The primers can comprise the sequence PAM-Recognition-Promoter, where PAM represents the PAM site of a nucleic acid-guided nuclease, Recognition represents a recognition site of a nucleic acid-guided nuclease, and Promoter represents a promoter site. The recognition site of the nucleic acid-guided nuclease can be an appropriate length for a given nucleic acid-guided nuclease (e.g., between about 15 and about 25 nucleotides, in some cases 20 nucleotides). In an example, the primers can comprise the sequence PAM-$N^*N_{(16)}+N_{(3)}$-T7*N or its complement, where + represents a modified nucleic acid bond, * represents a phosphorothioate (PTO) nucleic acid bond, PAM represents a protospacer adjacent motif, and T7 represents a T7 site or other appropriate promoter site. Modified nucleic acids can comprise locked nucleic acid (LNA), bridged nucleic acid (BNA), peptide nucleic acid (PNA), zip nucleic acid (ZNA), glycol nucleic acid (GNA), threose nucleic acid (TNA), and other modified nucleic acids such as those with increased binding specificity or sensitivity. In an example, the primers can comprise the sequence PAM-$H^*H_{(4)}N_{(12)}+N_{(3)}$-T7*N. Contemplated primer variations can include more modified and/or PTO nucleic acid bonds. The primers can then be extended **832** to incorporate sequence **833** complementary to the nucleic acid fragment. In some cases, the extension can be conducted using labeled nucleotides (e.g., biotinylated uracil) for later purification. Next, the unextended or unbound primers can be removed **834**. In some cases, the primers can be removed by capturing extension products incorporating labels (e.g., using streptavidin to capture biotinylated nucleotides). In some cases, the primers can be removed by size selection (e.g., electrophoresis, solid phase reversible immobilization (SPRI) beads). Next, the nucleic acids can be nicked, such as with CviPII enzymes or with a uracil-specific excision enzyme (e.g., USER or uracil DNA glycosylase (UDG)), and digested **335**, such as with single stranded exonuclease (e.g., both 5' to 3' and 3' to 5' exonuclease). This can leave single stranded products **836**, which can comprise sequence complementary to that adjacent to the GGH site or other PAM site on the nucleic acid fragments, as well as a T7 site or other appropriate promoter site. Next, ligation **837** can be used to ligate a 5' stemloop with a 3' block **828** to the single stranded products. A staggered double stranded stemloop **839** can also be added. The end of the stemloop D-D can comprise sequence complementary to the H*H region or sequence that is complementary to the PAM sequence. These products can then be transcribed (e.g., using the T7 site or other appropriate promoter site) to produce guide nucleic acids (e.g., gRNAs).

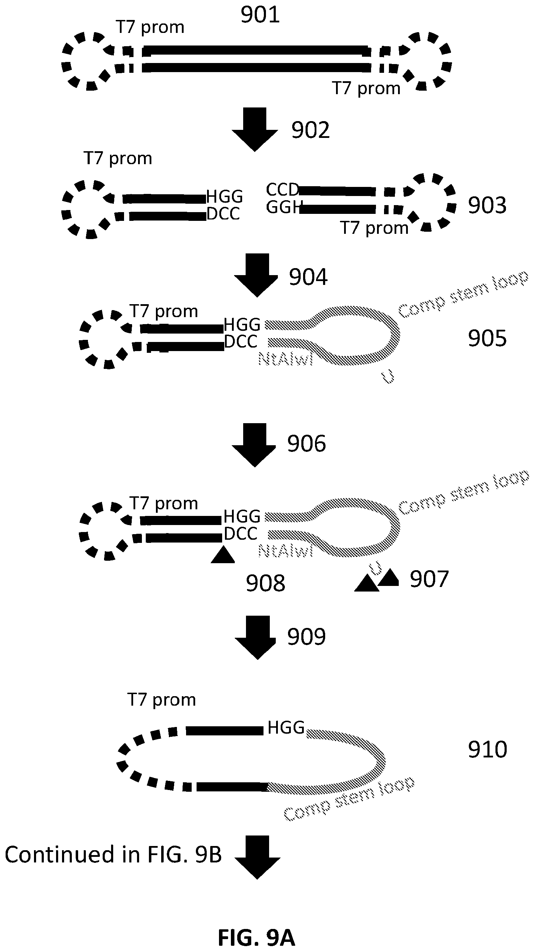

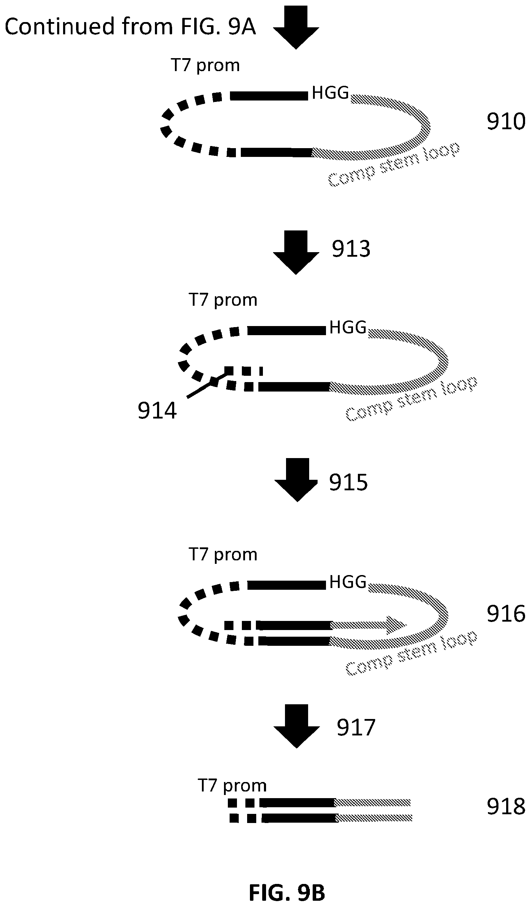

**FIG. 9A** and **FIG. 9B** show an additional technique for constructing a gNA library (e.g., gRNA library) from input nucleic acids (e.g., DNA), such as genomic DNA (e.g., human genomic DNA, reverse transcribed cDNA such as from mRNA). The protocol can begin with nucleic acid fragments **901** such as sheared genomic DNA or cDNA reverse-transcribed from mRNA, with circular adapters ligated onto the ends to form circular nucleic acids. The circular adapters can comprise promoter sites, such as T7 promoter sites or other appropriate promoter sites. The nucleic acids can then be nicked **902**, for example using CviPII. Close nicks can generate double strand breaks, which can then be blunted (e.g., using T4 DNA polymerase). The blunt ends of the products **903** can have HGG/GGH sequences (or other PAM sites) or the complements thereof (e.g., MAP sites). A circular adapter **905** can be ligated **904** to the ends of the products. This circular adapter can comprise a sequence complementary to a guide nucleic acid stem loop sequence, one or more restriction sites (e.g., Nt.AlwI site) and can contain one or more uracil nucleotides. This product can then be treated with uracil-specific excision enzymes such as uracil DNA glycosylase (UDG) and DNA glycosylase-lyase Endonuclease VIII to remove the U residue and create cuts before and after it **907**. Treatment with Nt.AlwI can be used to introduce a nick **908** downstream of the CCD motif or other MAP site. The product can then be ligated **909** (e.g., using a circular ligase such as CircIILigase), such that the sequence complementary to a guide nucleic acid stem loop sequence is ligated immediately upstream of the complement for the recognition region (e.g., N20 region) of the guide nucleic acid **910**. Continuing in **FIG. 9B**, the product can then be primed **913** with a primer **914**, for example at a promoter site (e.g., T7 site or other appropriate promoter site). The product can then be amplified **915**, e.g. using rolling circle amplification. Amplification can be performed with a polymerase such as Phi29 polymerase. Amplification can produce many single stranded concatemers of the promoter site, the recognition site region, and the stem loop region of the guide nucleic acid. The promoter-recognition site-stem loop sequences can be excised **917** from any adjacent sequence, for example using restriction sites located 5' and 3' relative to the sequences. Each of these promoter-recognition site-stem loop sequences **918** can be used as a guide nucleic acid precursor.

In some embodiments, a collection of gNAs (e.g., gRNAs) targeting human mitochondrial DNA (mtDNA) is created, that can be used for directing nucleic acid-guided nuclease (e.g., Cas9) proteins, comprising the nucleic acid-guided nuclease (e.g., Cas9) target sequence. In some embodiments, the targeting sequence of this collection of gNAs (e.g., gRNAs) are encoded by DNA sequences comprising at least the 20 nt sequence provided in the right-most column of Table 3 (e.g., if the NGG sequence is on negative strand). In some embodiments, a collection of gRNA nucleic acids, as provided herein, with specificity for human mitochondrial DNA, comprise a plurality of members, wherein the members comprise a plurality of targeting sequences provided in the right-most column of Table 3.

**Table 3.** Oligonucleotides used with MlyI Adapter.

| Oligo name | Sequence (5'>3') | Modification |
|---|---|---|
| MlyI-Ad1 | gagatcagcttctgcattgatgccagcagcccgagtcag | none |
| MlyI-Ad2 | ctgactcgggctgctgtacaaagacgatgacgacaagcgtta | 5'phosphate |
| BsMm-Ad 1 | gagatcagcttctgcattgatgcGGAGCCGCAGTACACTATCCAAC | none |
| BsMm-Ad2 | GTTGGATAGTGTACTGCGGCTCCtacaaagacgatgacgacaagcg | 5'phosphate |
| T7-Ad1 | gcctcgagctaatacgactcactatagagNN | none |
| T7-Ad2 | Ctctatagtgagtcgtatta | 5'phosphate |
| gR-top | ttagagctagaaatagcaagttaaaataaggctagtccgttatcaacttgaaaaagtggcaccgagtc ggtgctttttt | 5'phosphate |
| gR-bot | aaaaaagcaccgactcggtgccactttttcaagttgataacggactagccttattttaacttgctatttcta gctctaaaac | none |

Provided herein are methods that enable the generation of a large number of diverse gRNAs, collections of gNAs, from any source nucleic acid (e.g., DNA) that can be used with CRISPR/Cas system endonucleases. Some methods for the efficient synthesis of collections of gRNAs with a 3' nucleic acid guided nuclease system protein binding sequence and a 5' targeting sequence may be specific to gNAs with that arrangement of segments. Provided herein are methods for the synthesis of collections of gRNAs with a 5' nucleic acid guided nuclease system protein binding sequence and a 3' targeting sequence. All CRISPR/Cas endonucleases that are compatible with gRNAs with a 5' nucleic acid guided nuclease system protein binding sequence and a 3' targeting sequence are envisaged as within the scope of the methods of the disclosure.

Methods provided herein can employ enzymatic methods including but not limited to digestion, ligation, extension, overhang filling, transcription, reverse transcription, amplification.

Several strategies are employed. A method can comprise providing a nucleic acid (e.g., DNA); employing a first enzyme (or combinations of first enzymes) that cuts at a part of the PAM sequence in the nucleic acid, in a way that a residual nucleotide sequence from the PAM sequence is left; ligating an adapter that positions a restriction enzyme type IIS site (an enzyme that cuts outside yet near its recognition motif) at a distance to eliminate the PAM sequence; employing a second type IIS enzyme (or combination of second enzymes) to eliminate the PAM sequence together with the adapter; and fusing a sequence that can be recognized by protein members of the nucleic acid-guided nuclease (e.g., CRISPR/Cas) system, for example, a gRNA stem-loop sequence. The first enzymatic reactions cut part of the PAM sequence in a way that residual nucleotide sequence from the PAM sequence is left, and that the nucleotide sequence immediately 3' to the PAM sequence can be any purine or pyrimidine. An alternative strategy for fragmenting a provided nucleic acid (e.g. DNA) specifically at the Cpfl PAM sites comprises replacing adenines with inosines, or thymidines with uracils, and then cutting at abasic or mismatched sites, followed by the additional steps outlined above.

As an additional alternative, a provided nucleic acid (e.g. DNA) can be randomly sheared. By random chance, a proportion of the fragmentation sites generated by random shearing will overlap with TTN PAM sequences. The fragments can be ligated either to adapters with complementary overhangs, or to blunt ended adapters that reconstitute functional restriction sites only when ligated to a fragment with a terminal PAM. These strategies allow for the selective processing into gRNAs of only those fragments that were 3' of a PAM sequence in the original nucleic acid provided.

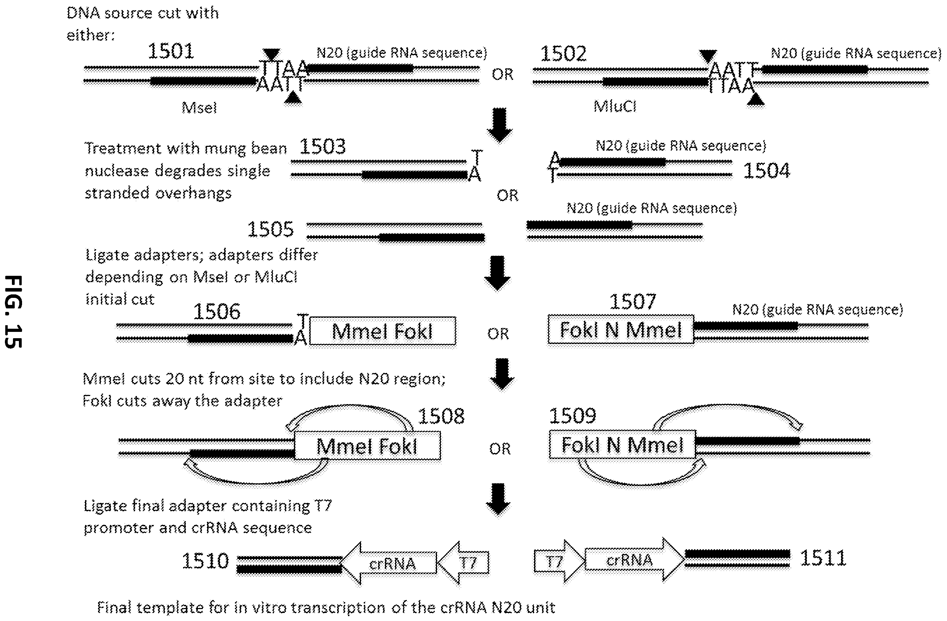

FIG. 15 shows an additional technique for constructing a gNA library (e.g., gRNA library) from input nucleic acids (e.g., DNA), such as genomic DNA (e.g., human genomic DNA, reverse transcribed cDNA such as from mRNA). The protocol can begin with nucleic acid fragments that have been cut with either MseI (1501) or MluCI (1502). MseI cuts within TTAA sites, while MluCI cuts at AATT sites. Both MseI and MluCI recognition sites comprise TTN, which, in certain embodiments, functions as a PAM site. For example, Cpfl proteins isolated from *Francisella tularensis* recognize TTN as a PAM. Starting DNA digested with MseI or MluCI results in a collection of digested fragments such that the ends of the fragments comprise potential PAM sequences. Enzymes other than MseI and MluCI that cut within or adjacent to other PAM sequences are also envisaged as being within the scope of the invention. Exemplary, but non-limiting examples of restriction enzymes that produce digested fragments with terminal PAM sequences are listed in Table 7. MseI or MluCI digested DNA fragments are then treated with mung bean nuclease to degrade the single stranded overhangs (1503, 1504, 1505). Adapters comprising MmeI and FokI restriction sites are then ligated to these DNA fragments. The adapter sequence will depend on whether the starting nucleic acid material was cut with MseI (1506) or MluCI (1507). The MmeI enzyme is then used to cut the DNA fragment 20 bp away from the MmeI site in the adapter sequence, removing unwanted DNA sequence from the 20 nucleotide nucleic acid targeting sequence (N20). Following MmeI digestion, the FokI enzyme is then used to cut adjacent to the adapter liberating the 20 nucleotide nucleic acid targeting sequence (N20) (1508, 1509). An additional adapter comprising a promoter sequence such as a T7 promoter sequence and a nucleic acid guided nuclease system protein binding sequence is then ligated to the DNA fragment comprising the N20 sequence (1510, 1511). This produces the final template for *in vitro* transcription of the crRNA N20 unit to produce a gNA.

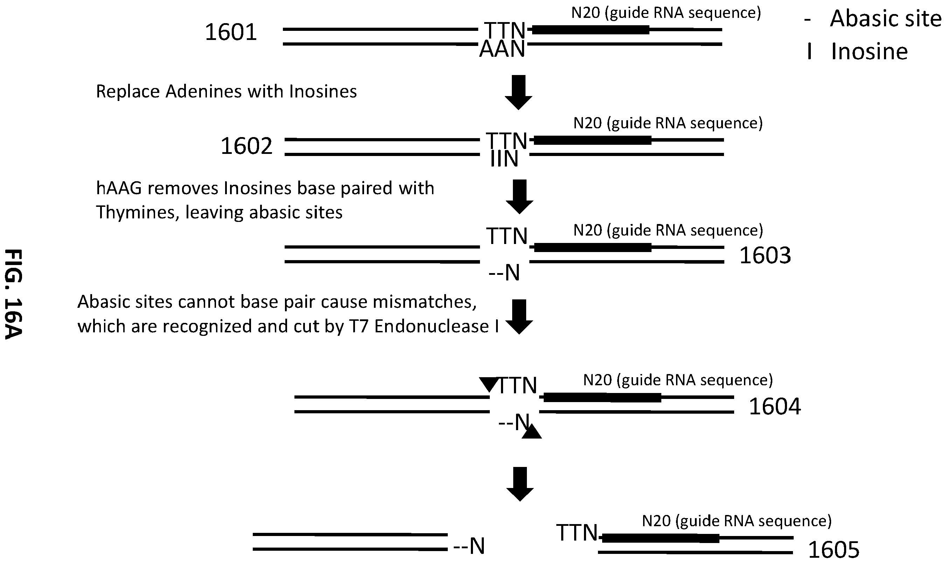

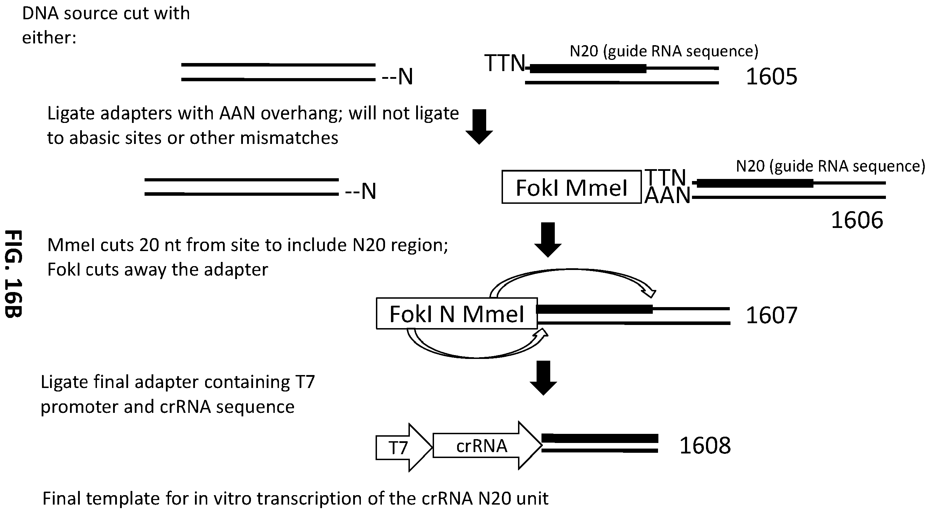

FIG. 16 shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA in which the Adenines have been replaced with Inosines (FIG. 16). When Adenines have been replaced with Inosines (1602), human Alkyladenine DNA Glycosylase (hAAG) is used to remove the Inosines that are based-paired with Thymines, leaving abasic sites (1603). These abasic sites cannot base-pair, which causes mismatches that are recognized and cut by T7 Endonuclease I (1604), resulting in DNA fragments with, for example, a TTN overhang (1605). In certain embodiments, TTN functions as a PAM site. For example, Cpfl proteins isolated from *Francisella tularensis* recognize TTN as a PAM. This TTN overhang can be used to ligate adapters with AAN overhangs. This overhang, in the 5' to 3' direction, is 5'-NAA-3' and is complementary to the TTN overhang of DNA fragments produced by this method (1606). A feature of these AAN overhang containing adapters is that these adapters will not ligate to abasic sites or other mismatches, which leads to adapter ligation specific to those N20 containing fragments that comprise TTN PAM sites as overhangs. DNA fragments, with, for example, a TNN terminal sequence that was cut by the T7 Endonuclease I of this method will fail to ligate to an adapter. This produces a collection of nucleic acid molecules comprising an adapter such as an adapter comprising FokI and MmeI restriction sites, a TTN sequence, and a nucleic acid targeting sequence (N20) (**1606**). The MmeI restriction enzyme is then used to cut 20 bp away from the Mme I site in the adapter sequence, removing unwanted DNA sequence from the 20 nucleotide nucleic acid targeting sequence (N20). Following MmeI digestion, FokI is used to cut adjacent to the adapter, liberating the 20 nucleotide nucleic acid targeting sequence (N20) (**1607**). An additional adapter comprising a promoter sequence such as a T7 promoter sequence and a nucleic acid guided nuclease system protein binding sequence is then ligated to the DNA fragment comprising the N20 sequence (**1608**). This produces the final template for *in vitro* transcription of the crRNA N20 unit to produce a gNA.

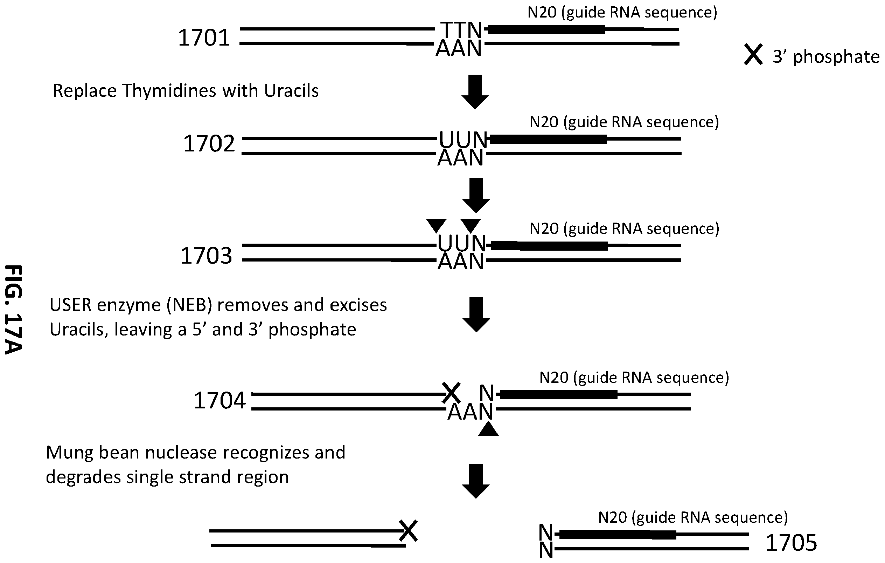

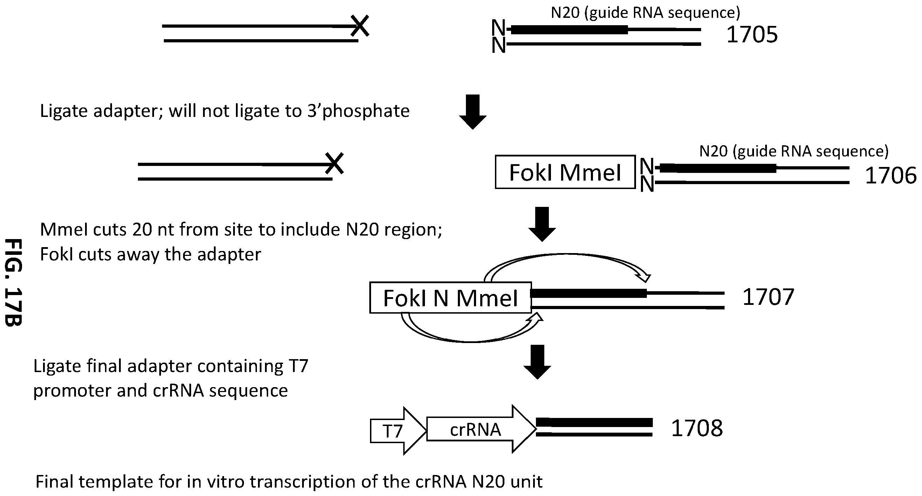

**FIG. 17** shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA in which the Thymidines have been replaced with Uracils (**1702**). The USER Enzyme (Uracil-Specific Excision Reagent, NEB #M5505S) removes and excises the Uracils, leaving a 5' and a 3' phosphate (**1704**). With USER, a Uracil DNA Glycosylase (UDG) catalyzes the excision of a uracil base to generate an abasic site, and Endonuclease VIII breaks the phosphodiester backbone at the 3' and 5' sides of the abasic site.

In certain embodiments of this method, phosphatase treatment removes the 3' phosphate adjacent to the abasic site, followed by a single base pair extension using the dideoxyribonucleic acid ddTTP, prior to treatment with mung bean nuclease. Other DNA repair enzymes that can produce abasic sites are envisioned as within the scope of the invention. For example a DNA glycosylase such as human Oxoguanine glycosylase (hOGG1) can be used to excise mismatched base pairs and generate abasic sites. A feature of this method is that specificity for fragmentation of the starting DNA at TTN sites, rather than, for example TN sites, comes in part from the combination of USER mediated excision and ddTTP extension. For TN sites, the end product is a nick, which makes a poor substrate. For TTN (or greater than two Ts), there is an at least one base pair gap that is more efficiently cleaved. In an alternative embodiment, USER-mediated Uracil excision is followed immediately by mung bean nuclease degradation of the single stranded region. Mung bean nuclease then recognizes and degrades the single stranded region (**1705**). Mung bean nuclease treatment produces a collection of DNA fragments whose 5' end is adjacent to the TT of a TTN site. In certain embodiments, TTN functions as a PAM site. For example, Cpf1 proteins isolated from *Francisella tularensis* recognize TTN as a PAM. Adapters comprising FokI and MmeI sites are ligated to the resulting nucleic acid fragments (**1706**). A feature of these adapters is that these adapters will not ligate to 3' phosphates. The MmeI restriction enzyme is used to cut 20 bp away from the MmeI site in the adapter sequence, removing unwanted DNA sequence from the 20 nucleotide nucleic acid targeting sequence (N20), and FokI is used to cut adjacent to the adapter liberating the 20 nucleotide nucleic acid targeting sequence (N20) (**1707**). An additional adapter comprising a promoter sequence such as a T7 promoter sequence and a nucleic acid guided nuclease system protein binding sequence is then ligated to the DNA fragment comprising the N20 sequence (**1708**). This produces the final template *for in vitro* transcription of the crRNA N20 unit to produce a gNA.

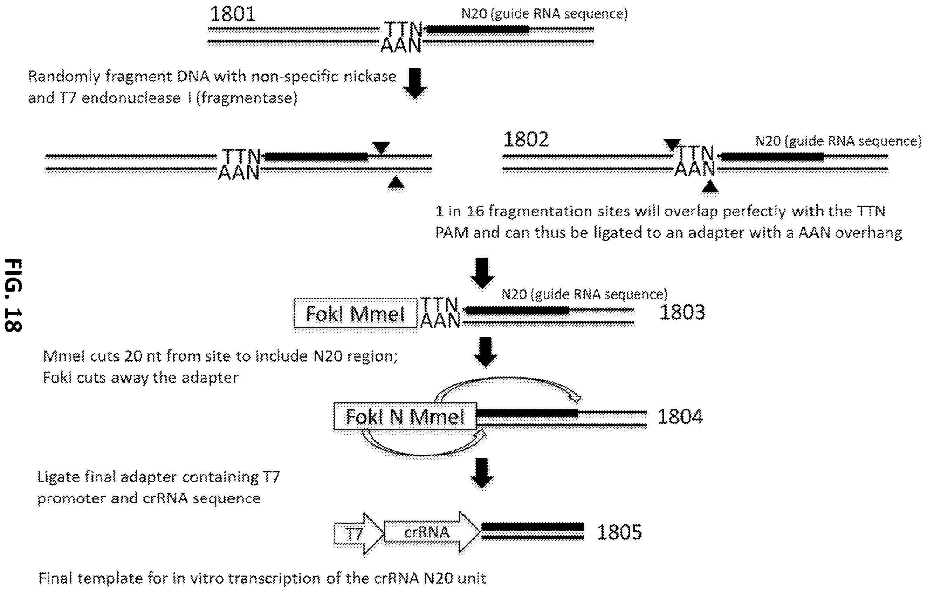

**FIG. 18** shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA which has been randomly fragmented with a non-specific nickase and T7 endonuclease I (fragmentase). In certain embodiments, 1 in 16 fragmentation sites will overlap perfectly with the TTN PAM site (**1802**), producing a TTN overhang that can be ligated to an adapter comprising an AAN overhang. This produces a collection of adapter ligated DNA fragments that comprise an N20 sequence adjacent to a TTN PAM sequence. For example, an adapter comprising FokI and MmeI restriction sites is ligated to the DNA fragments (**1803**). The MmeI enzyme is then used to cut 20 bp away from the MmeI site in the adapter sequence removing unwanted DNA sequence from the 20 nucleotide nucleic acid targeting sequence (N20), and FokI used to cut adjacent to the adapter liberating the 20 nucleotide nucleic acid targeting sequence (N20) (**1804**). An additional adapter comprising a promoter sequence such as a T7 promoter sequence and a nucleic acid guided nuclease system protein binding sequence is then ligated to the DNA fragment comprising the N20 sequence (**1805**). This produces the final template for *in vitro* transcription of the crRNA N20 unit to produce a gNA.

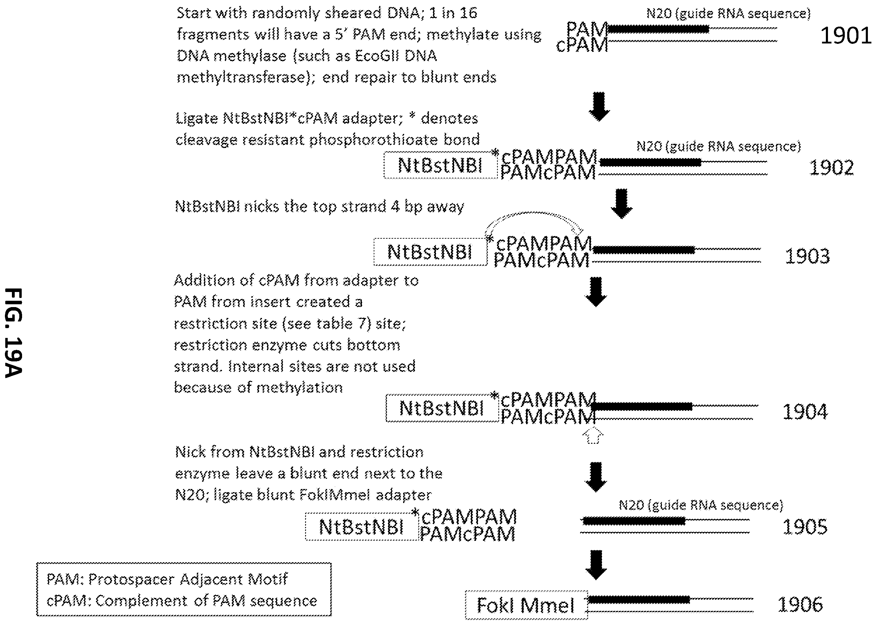

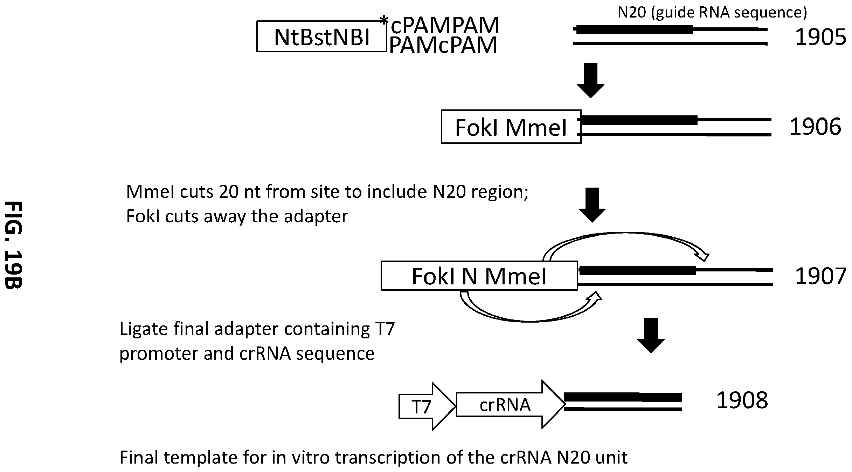

**FIG. 19** shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA which has been randomly sheared. In certain embodiments, 1 in 16 fragments will have a 5' PAM end (**1901**). The 5' end of the randomly sheared DNA fragments can be methylated using a DNA methylase such as EcoGII DNA methyltransferase, and end repaired to produce blunt ends (**1901**). An NtBstNBI*cPAM is ligated to the ends of the sheared, methylated and end repaired DNA fragments comprising the N20 nucleic acid targeting sequence (**1902**). (*) denotes a cleavage resistant phosphorothioate bond, which negates second strand cutting. NtBstNBI (also called Nt.NstNBI) then nicks the top strand of the DNA 4 base pairs away from the phosphorothioate bond (**1903**). In some embodiments, the NtBstNBI*cPAM adapter comprises a sequence such that the addition of the complementary PAM (cPAM) sequence of the adapter to the PAM sequence of the DNA fragment creates a restriction site (see table 7 below for PAMs and the associated sequences and restriction enzymes). This restriction site can be cut by a restriction enzyme such as HaeIII, MluCI, AluI, DpnII or FatI. The creation of the restriction site through the ligation of the NtBstNBI*cPAM adapter (**1903**) to the sheared DNA fragment comprising a PAM site, and the subsequent cleavage of the newly created restriction site (**1903**, **1904**) allows for the selective processing of only those DNA fragments containing a terminal PAM sequence. The cleavage resistant phosphorothioate bond in the adapter negates second strand cutting by the restriction enzyme, and internal sites are not used because of methylation. Using an AATT PAM and MluCI as an example, by nicking the top strand at the PAM site with NtBstNBI producing an AATT(cut) position before cutting with MluCI, which cuts both strands, a blunt ended fragment is produced, as opposed to a nick or a 4 bp overhang. Only a blunt fragment can ligate to the adapter. The NtBstNBI nick (**1903**) and the restriction enzyme cut produce a blunt end next to the N20 sequence (**1905**), to which an adapter comprising a FokI site and an MmeI site is ligated (**1906**). The MmeI enzyme then cuts 20 bp away from the adapter sequence removing unwanted DNA sequence from the 20 nucleotide nucleic acid targetingsequence (N20), and FokI cuts adjacent to the adapter liberating the 20 nucleotide nucleic acid targeting sequence (N20) (**1907**). An additional adapter comprising a promoter sequence such as a T7 promoter and a nucleic acid guided nuclease system protein binding sequence is then ligated to the DNA fragment comprising the N20 sequence (**1908**). This produces the final template for *in vitro* transcription of the crRNA N20 unit to produce a gNA.

**Table 7**.

| Target sequence and PAM | Sequence of initial adapter (PBS= primer binding site) | Restriction enzyme to be utilized to specifically cut terminal PAM sites |
| --- | --- | --- |
| N20-NGG | PBS-GAGTCGG (NtBstNBI Ad)<br><br>Circ-GG (Circ Ad) | HaeIII |
| TTN-N20 | PBS-GAGTCAA (NtBstNBI Ad)<br><br>Circ-AA (Circ Ad) | MluCI |
| N20-NAG | PBS-GAGTCAG (NtBstNBI Ad)<br><br>Circ-AG (Circ Ad) | AluI |
| TCN-N20 | PBS-GAGTCGA (NtBstNBI Ad)<br><br>Circ-GA (Circ Ad) | DpnII |
| TGN-N20 | BS-GAGTCCA (NtBstNBI Ad)<br><br>Circ-CA (Circ Ad) | FatI |

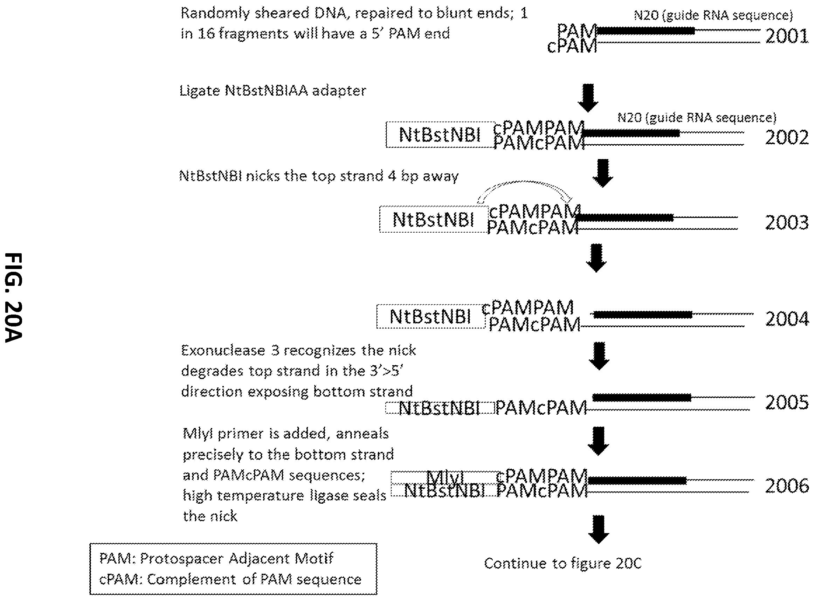

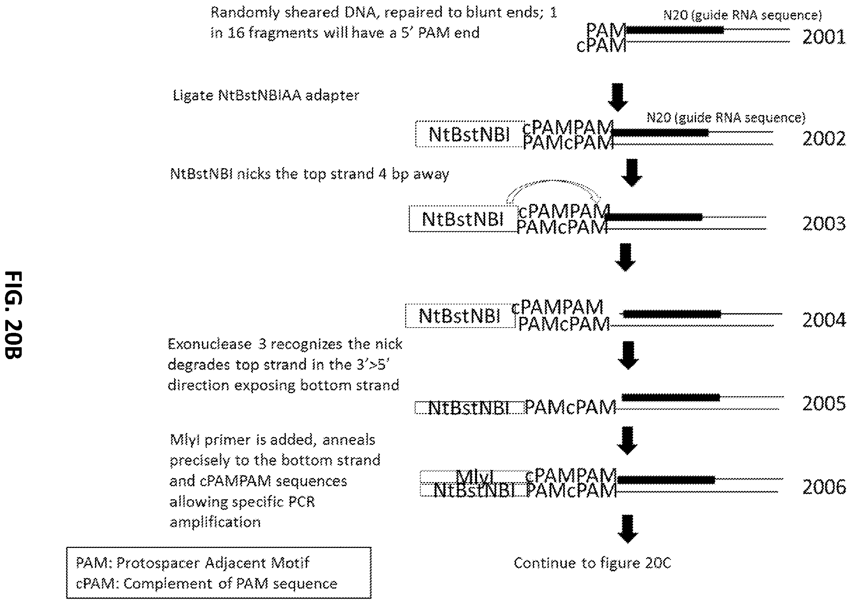

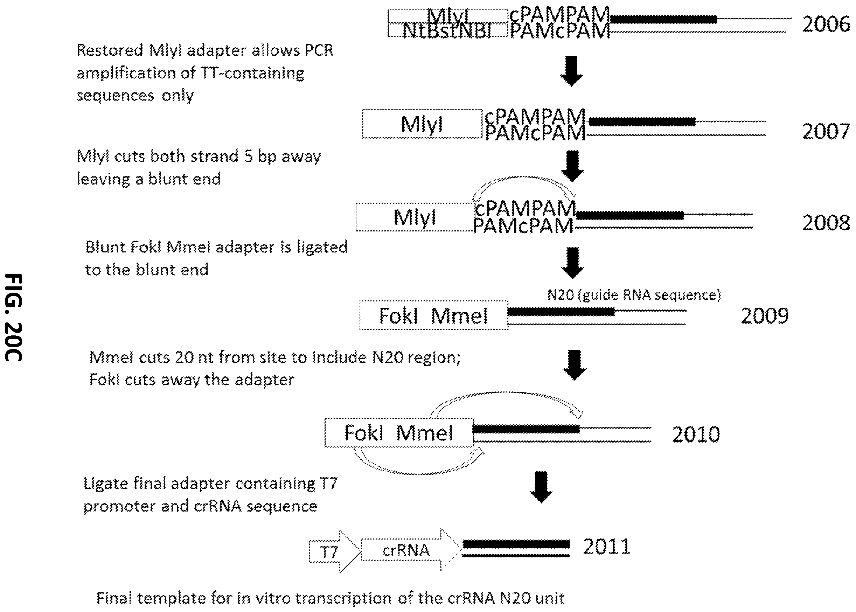

**FIG. 20** shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA which has been randomly sheared and repaired to blunt ends. In certain embodiments, 1 in 16 fragments will have a 5' PAM end (**2001**, PAM and complementary PAM (cPAM) sequences, as indicated). An NtBstNBIAA adapter is ligated to the randomly sheared, blunt ended DNA fragments (**2002**), and NtBstNBI then nicks the top strand 4 base pairs away (**2003**). Exonuclease 3 recognizes the nick (**2004**) and degrades the top strand in the 3' to 5' direction exposing the bottom strand (**2005**). An MlyI primer is added which anneals precisely to the bottom strand and the PAMcPAM sequences. A high temperature ligase seals the nick (**2006**) which creates specificity for only those sheared, blunted DNA fragments comprising a terminal PAM sequence, and which gave rise to an PAMcPAM sequence upon ligation of the NtBstNBI adapter. Only creation of the PAMcPAM sequence allows precise ligation. Any other fragments will have a mismatch near the ligation site and this will negate the activity of the ligase. In some embodiments, the restored MlyI adapter allows for selective PCR amplification of the TT-containing sequences only of **2006** (**FIG. 20B**) producing the MlyI fragments of **2007,** i.e. PCR amplified DNA fragments that contain both an MlyI sequence and PAM adjacent N20 sequences. PCR amplification is carried out with an enzyme without proofreading 3' to 5' exonuclease activity. MlyI then cuts both strands 5 base pairs away, leaving a blunt end and removing the PAMcPAM sequence (**2008**). A blunt adapter comprising FokI and MmeI restriction sites is then ligated to the MlyI digested DNA fragments (**2009**). The MmeI enzyme then cuts 20 bp away from the adapter sequence removing unwanted DNA sequence from the 20 nucleotide nucleic acid targeting sequence (N20), and FokI cuts adjacent to the adapter liberating the 20 nucleotide nucleic acid targeting sequence (N20) (**2010**). An additional adapter containing a promoter sequence such as a T7 promoter sequence and a nucleic acid guided nuclease system protein binding sequence is then ligated to the DNA fragment comprising the N20 sequence (**2011**). This produces the final template for *in vitro* transcription of the crRNA N20 unit to produce a gNA.

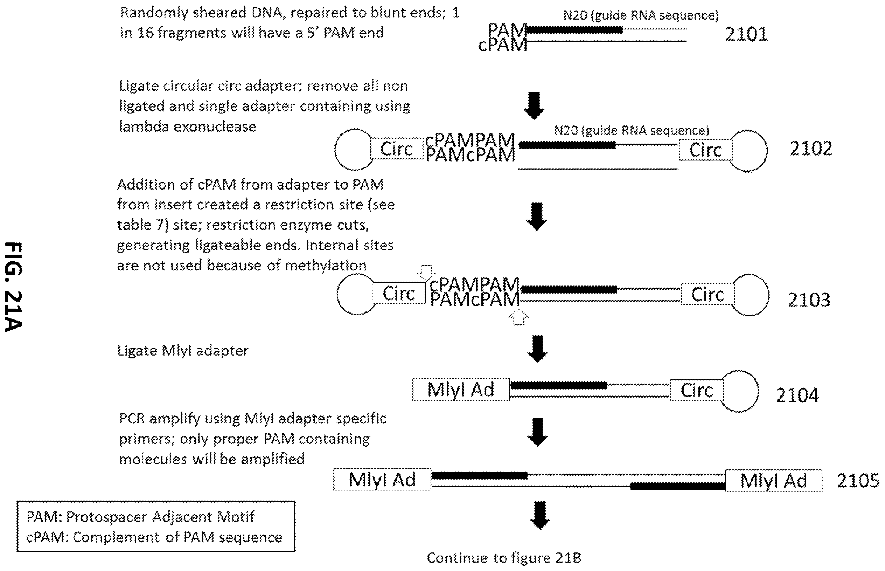

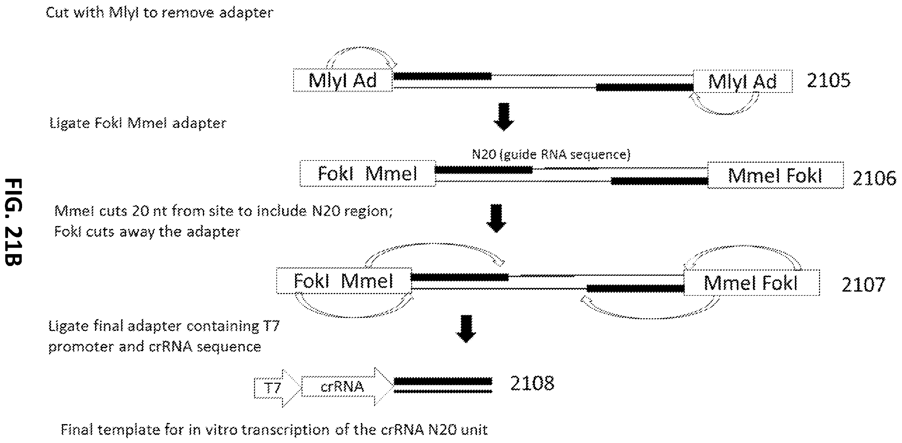

FIG. 21 shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA which has been randomly sheared and repaired to have blunt ends. In certain embodiments, 1 in 16 fragments will have a 5' PAM end (**2101**, PAM and complimentary PAM (cPAM), as indicated). A circular adapter (circ adapter) is ligated to these blunt ended DNA fragments, and fragments without circular adapters at both ends are degraded using lambda exonuclease (**2102**). In some embodiments, the addition of the cPAM sequence from the adapter to the PAM sequence of the DNA fragment creates a restriction site (see Table 7, and **2103**). This restriction site can be cut by a restriction enzyme such as HaeIII, MluCI, AluI, DpnII or FatI. When this site is cut by a restriction enzyme such as HaeIII, MluCI, AluI, DpnII or FatI, it generates ligate-able ends. The creation of the restriction site through the ligation of the circular adapter (**2102** to the sheared DNA fragment comprising a PAM site, and the subsequent cleavage of the newly created restriction site (**2103**) allows for the selective processing of only those DNA fragments containing a terminal PAM sequence. Fragments with adapters that are not ligated at the PAM site will not be cut by the restriction enzyme (e.g. MluCI) at this step, and will thus remain circular. These circular fragments are unavailable for the subsequent rounds of ligation. Only the fragments with adapters ligated at the PAM sites will resist lambda nuclease (**2102**), and then be cut by the restriction enzyme (e.g. MluCI, and **2103**) thus opening them for the subsequent ligation round. Internal restriction sites are not used because of methylation. A methyltransferase such as EcoGII can be used as a pre-treatment. An additional adapter comprising a MlyI sequence is then ligated to the DNA fragments (**2104**). The DNA fragments are PCR amplified using MlyI adapter specific PCR primers (**2105**). Only DNA molecules containing PAM sequences will be amplified. The amplified PCR product is then cut with MlyI to remove the adapter (**FIG. 21B,** 2105), and an adapter comprising FokI and MmeI restriction sites is ligated to the resulting DNA fragment (**2106**). The MmeI enzyme then cuts 20 bp away from the adapter sequence removing unwanted DNA sequence from the 20 nucleotide nucleic acid targeting sequence (N20), and FokI cuts adjacent to the adapter liberating the 20 nucleotide nucleic acid targeting sequence (N20) (**2107**). An additional adapter containing a promoter such as T7 and a nucleic acid guided nuclease system protein binding sequence is then ligated to the DNA fragment comprising the N20 sequence (**2108**). This produces the final template for *in vitro* transcription of the crRNA N20 unit to produce a gNA.

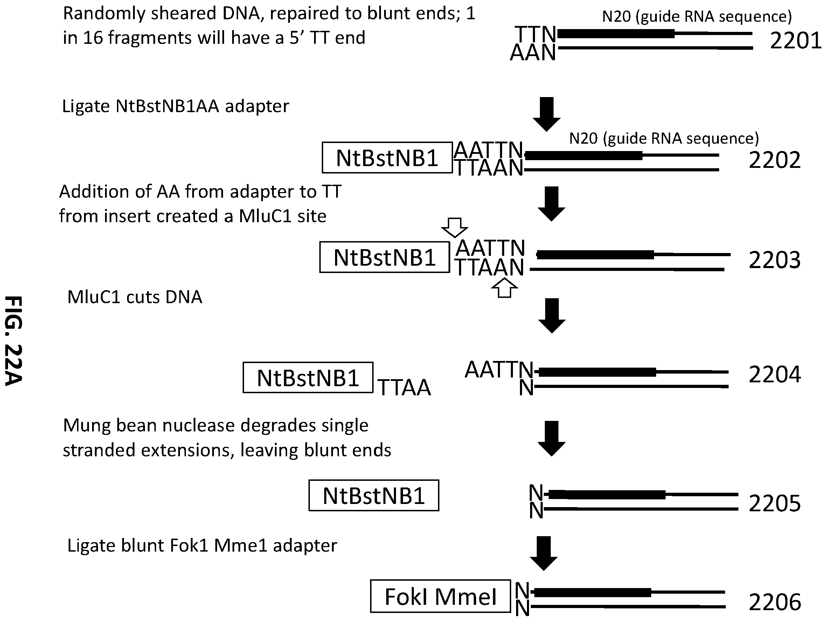

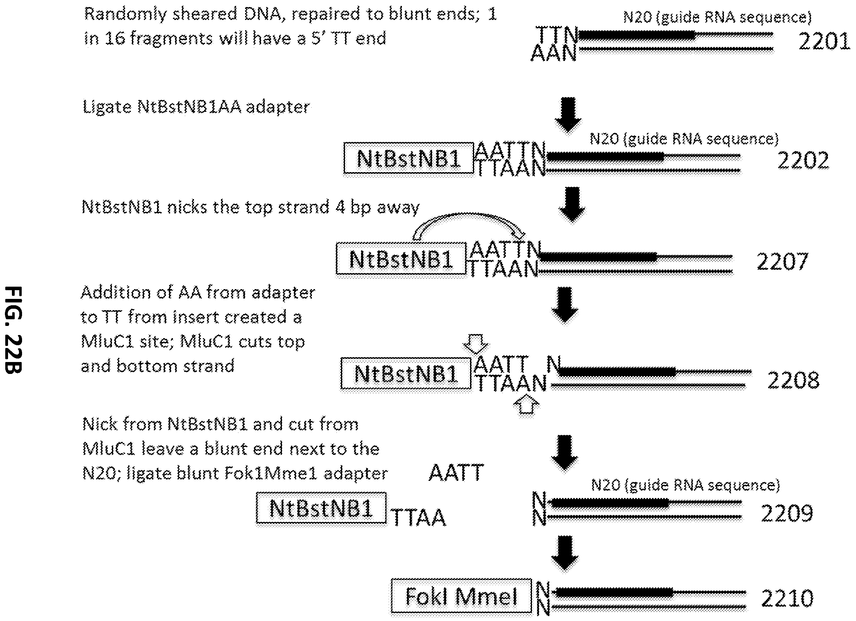

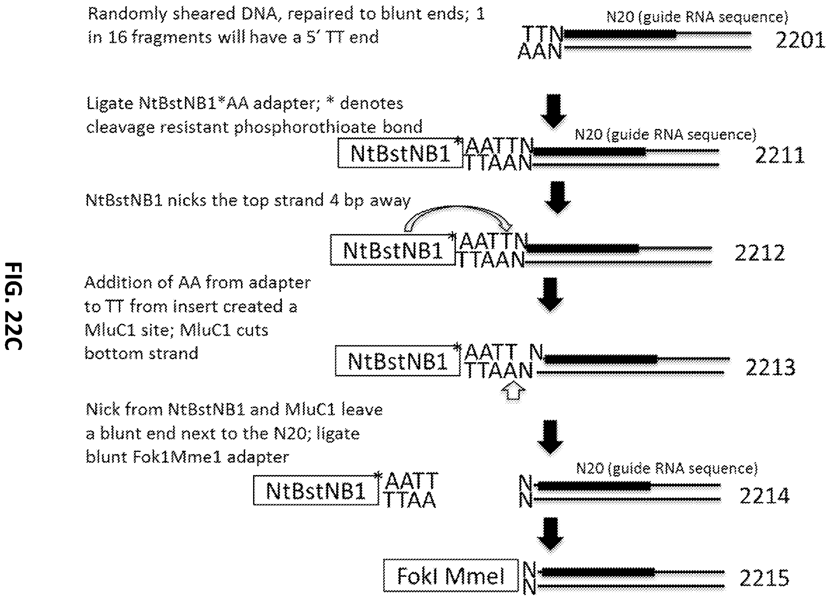

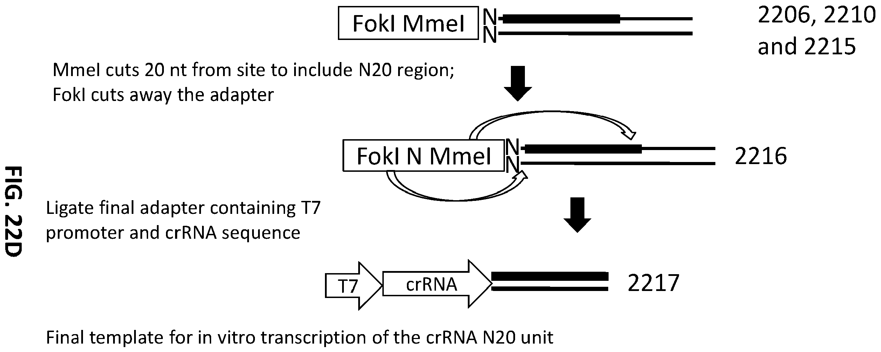

FIG. 22 shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA which has been randomly sheared and repaired to have blunt ends. In certain embodiments, 1 in 16 fragments will have a 5' TT end (**2201**, TTN and AAN, as indicated). In certain embodiments, TTN can be used as a PAM site. For example, TTN is recognized by Cpfl and related family members. A NtBstNBI adapter comprising terminal an AA (NtBstNBIAA) is then ligated to the TT end (**2202**). The addition of 3' terminal AA from the adapter to 5' terminal TT from the DNA fragment creates an MluCI restriction site. MluCI cuts in this newly created site (**2203**), leaving an AATT single stranded overhang (**2204**), which is degraded by mung bean nuclease to leave blunt ended fragments (**2205**). The creation of the AATT MluCI restriction site by the ligation of the NtBstNBI adapter with a terminal AA to sheared DNA fragments with a terminal TT allows for the selective processing of N20 DNA fragments adjacent to a TTN PAM sequence. An adapter comprising FokI and MmeI restriction sites is ligated to the resulting DNA fragment (**2206**).

Alternatively, following ligation of the NtBstNBI adapter, NtBstNBI may be used to nick the top strand 4 base pairs away (**2207**), and MluCI used to cut the top and bottom strand (2208). The nick from the NtBstNBI and the cut from the MluCI produce a blunt end next to the N20 sequence (**2209**), to which a blunt ended adapter comprising FokI and MmeI restriction sites is ligated (**2210**). In certain embodiments, the NtBstNBI adapter may be a NtBstNBI*AA adapter, where (*) denotes a cleavage resistant phosphorothioate bond (**2211**). NtBstNBI is used to nick the top strand 4 base pairs away (**2212**). The addition of AA from the adapter to TT from the DNA fragment creates an MluCI restriction site, and MluCI cuts the bottom strand of this restriction site (**2213**). The nick from NtBstNBI and the cut from the MluCI produce a blunt end next to the N20 sequence (**2214**), to which a blunt ended adapter comprising FokI and MmeI restriction sites is ligated (**2215**). After the blunt ended adapter comprising FokI and MmeI restriction sites has been ligated to the DNA fragments comprising the N20 sequence, the MmeI enzyme then cuts 20 bp away from the adapter sequence removing unwanted DNA sequence from the 20 nucleotide nucleic acid targeting sequence (N20), and FokI cuts adjacent to the adapter liberating the 20 nucleotide nucleic acid targeting sequence (N20) (**2216**). An additional adapter containing a promoter such as T7 and the crRNA sequence is then ligated to the DNA fragment comprising the N20 sequence (**2217**). This produces the final template for in vitro transcription of the crRNA N20 unit.

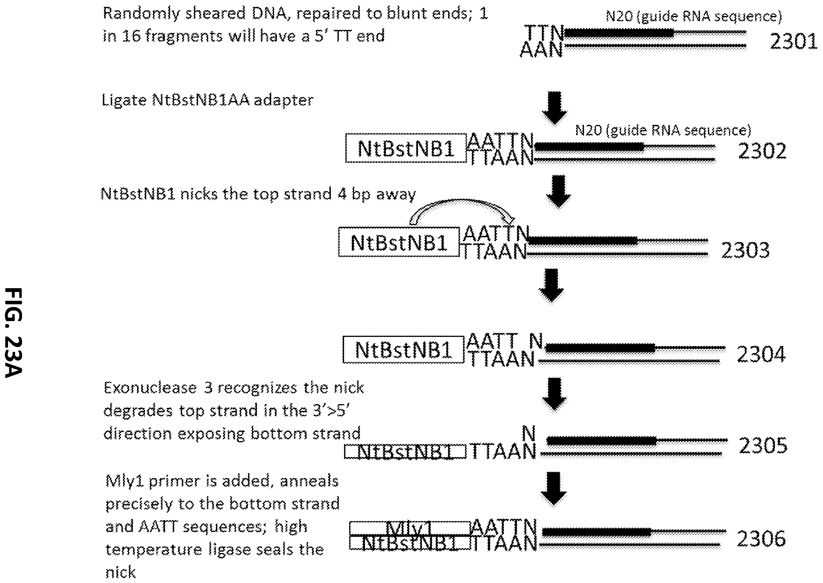

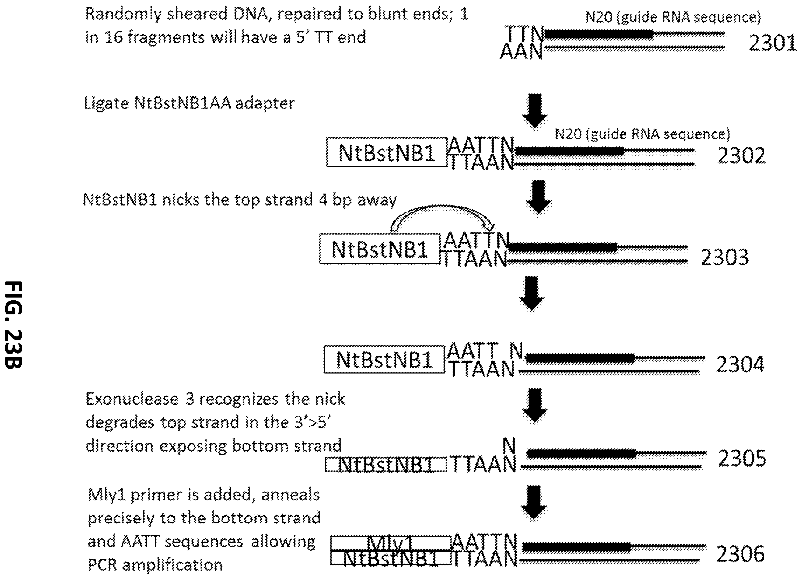

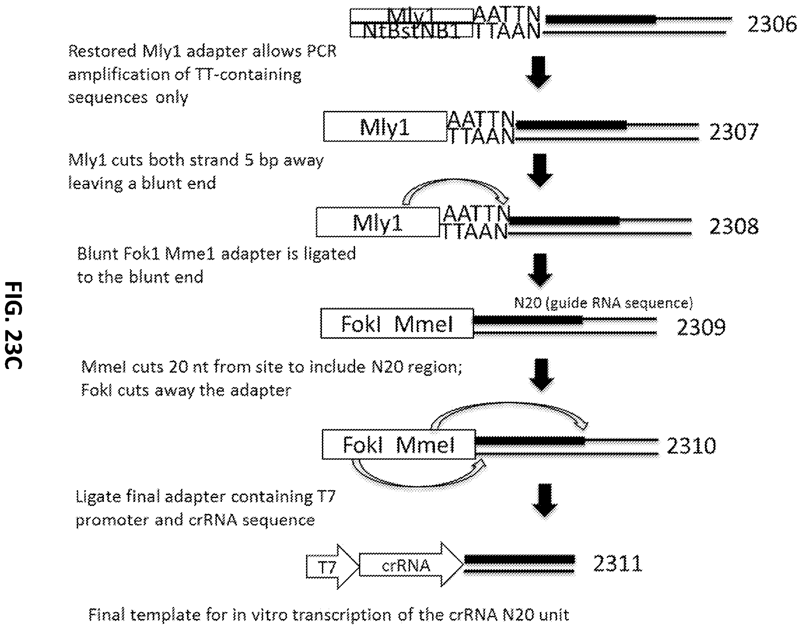

FIG. 23 shows an additional technique for constructing a gNA library (e.g., gRNA library) from input nucleic acids (e.g., DNA), such as genomic DNA (e.g., human genomic DNA, reverse transcribed cDNA such as from mRNA). In certain embodiments, the nucleic acid starting material for constructing a gNA library comprises DNA which has been randomly sheared and repaired to have blunt ends. In certain embodiments, 1 in 16 fragments will have a 5' TT end (**2301**, TTN and AAN, as indicated). In certain embodiments, TTN can be used as a PAM site. For example, Cpfl proteins isolated from *Francisella tularensis* recognize TTN as a PAM. The NtBstNBI adapter comprising a terminal AA (NtBst-NBIAA) is ligated to the end of the sheared, blunted DNA fragment (**2302**). When the sheared blunted DNA fragment comprises a terminal TT, ligation of the NtBstNBI adapter creates an AATT sequence (**2302**). The NtBstNBI enzyme is used to nick the top strand 4 base pairs away (**2303**). Exonuclease 3 recognizes the nick and degrades the top strand in the 3' to 5' direction, exposing the bottom strand (**2305**). An MlyI primer is added which anneals precisely to the bottom strand and the AATT sequence (**2306**). A high temperature ligase seals the nick (**FIG. 23A**, 2306), which creates specificity for only those sheared, blunted DNA fragments comprising a terminal TT sequence, and which gave rise to an AATT sequence upon ligation of the NtBstNBI AA adapter. In some embodiments, the restored MlyI adapter allows PCR selective amplification of the AATT-containing DNA fragments, i.e. those with TTN PAM adjacent N20 sequences(**2307**, **FIG. 23B**). MlyI then cuts both strands 5 base pairs away, leaving a blunt end and removing the AATT sequence (**2308**). A blunt adapter comprising FokI and MmeI restriction sites is then ligated to the MlyI digested DNA fragments (**2309**). The MmeI enzyme then cuts 20 bp away from the adapter sequence removing unwanted DNA sequence from the 20 nucleotide nucleic acid targeting sequence (N20), and FokI cuts adjacent to the adapter, liberating the 20 nucleotide nucleic acid targeting sequence (N20) (**2310**). An additional adapter containing a promoter such as T7 and a nucleic acid guided nuclease system protein binding sequence is then ligated to the DNA fragment comprising the N20 sequence (**2311**). This produces the final template for *in vitro* transcription of the crRNA N20 unit to produce a gNA.

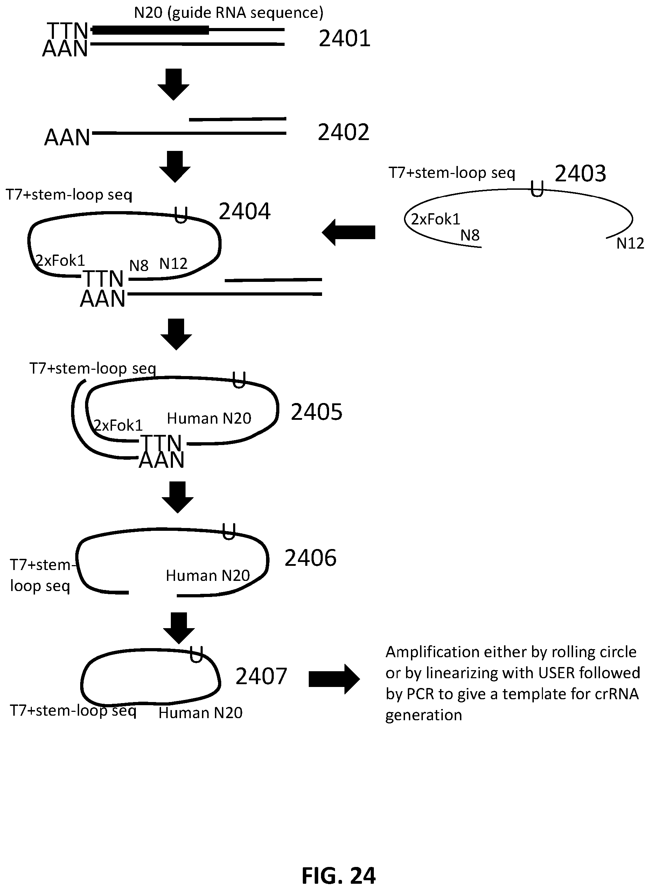

**FIG. 24** shows an additional technique for constructing a gNA library (e.g., gRNA library) from input nucleic acids (e.g., DNA), such as genomic DNA (e.g., human genomic DNA, reverse transcribed cDNA such as from mRNA). A feature of the method is the ligation at high temperature, that results in circularization of the oligo, and converts randomized N20 sequences to N20 repertoires, as well as building a library of crRNA molecules. In certain embodiments, the nucleic acid starting material for constructing a gNA library comprises DNA which has been randomly sheared and repaired to have blunt ends. In certain embodiments, 1 in 16 fragments will have a 5' TT end (**2401**, TTN and AAN, as indicated). The double stranded DNA fragments are treated with T7 exonuclease to expose a single strand (**2402**). Following treatment with T7 exonuclease, a linear oligo comprising a 5' phosphate, a random N12 sequence at the 5' end, a T7+stem-loop sequence, 2 opposed Fok1 sites and a TTN sequence followed by an N8 sequence at the 3'(**2403**) is added, annealed to the exposed single stranded DNA, and ligated using HiFidelity Taq ligase (**2404**). High temperature ligase requires greater than 10 bp perfect homology on either side of the nick to ligate. If there is less homology, gaps or mismatches, it will not ligate. This produces a circularized product, and thus the random nucleotides (N8 + N12) form a library of N20 sequences adjacent to a TTN PAM site (for example, a library of human N20 sequences as shown in FIG. 24). All remaining DNA is degraded using Exonuclease 1 and Exonuclease 3. An oligo complementary to the 2 opposed Fok1 regions is annealed to the circular DNA (**2405**) and the resulting product is cut with Fok1. This excises the (double stranded) opposed FokI sites, producing a collection of linear single stranded DNA fragments. TTN and unwanted sequences between end of stem-loop and N20 are eliminated (**2406**). These DNA fragments are self-circularized using CircLigase (a single stranded DNA ligase, Lucigen) (**2407**). The resulting circular DNAs are then amplification either by rolling circle amplification or by linearizing with USER followed by PCR to give a template for crRNA (gNA) generation.

## *Design and Synthesis*

Collections of guide nucleic acids can be designed (e.g., computationally) and then synthesized for use. Synthesis of gNAs can employ standard oligonucleotide synthesis techniques. In some cases, precursors to the gNAs can be synthesized, from which the gNAs can be produced. In an example, DNA precursors are synthesized and gNAs are transcribed (e.g., via in vitro transcription) from the DNA precursors.

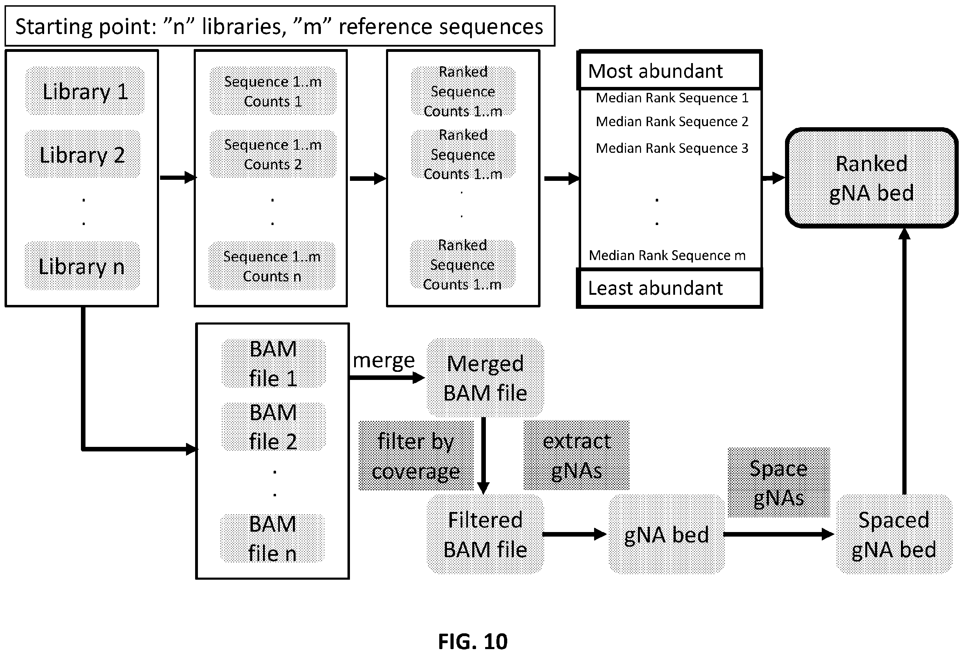

**FIG. 10** illustrates a technique for designing collections of guide nucleic acids. Sequence information for the target nucleic acid sequences (e.g., target genome, target transcriptome) can be obtained. Multiple sequencing libraries can be created that include the target nucleic acid, these libraries can be sequenced to the desired coverage, and raw sequencing read data can be generated. Reads from each sequenced library can be mapped to suitable reference sequence(s). Considering all reads that reliably map to the reference sequence(s), a sequence read alignment file (e.g., binary read alignment or "BAM" file) can be created, and the number of target reads that originated from a given reference sequence (the "abundance") can be calculated. The abundance measures obtained per target sequence can be sorted in decreasing order. Files from multiple sequencing libraries can be merged to create a single file. Regions of the sequence alignment (herein "target regions") that are covered by a minimum number of reads can be identified. Guide nucleic acid sequences (e.g., 20 nucleotides immediately preceding an "NGG" motif or other PAM site on either DNA strand, or 20 nucleotides following a "TTN" motif or other PAM site on either DNA strand) can be extracted from target regions. Next, an additional filtration step can be performed to ensure that gNAs are spaced by a minimum number of nucleotides. Map reads from each sequenced library to suitable reference sequence(s). This approach can give weight to more abundant sequences in the target sequences (e.g., cDNA from more abundant mRNA molecules for a transcriptome). For example, if the sequencing reads are from cDNA, then the number of reads can be correlated with the abundance of the associated transcript.

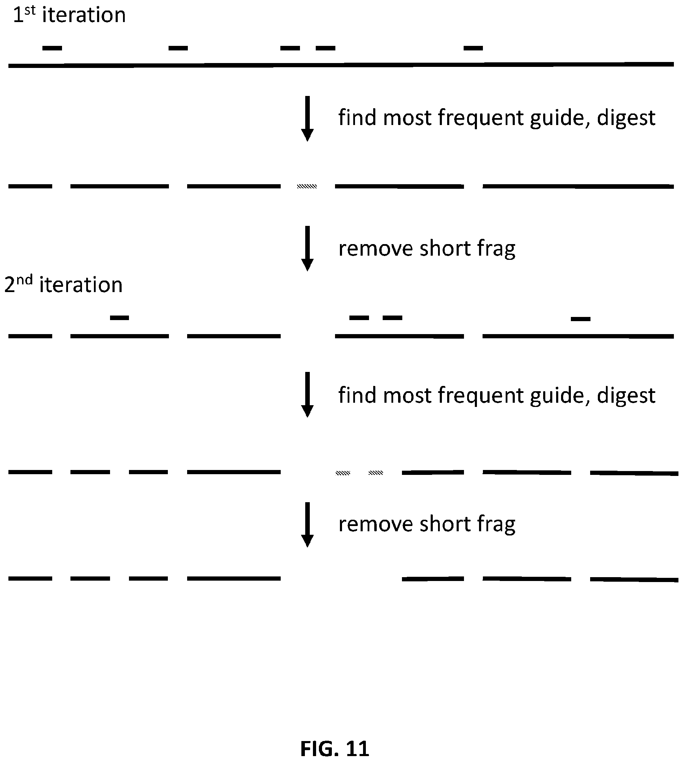

FIG. 11 illustrates a technique for designing collections of guide nucleic acids. Sequence information for the target nucleic acid sequences (e.g., target genome, target transcriptome) can be obtained. The most frequent guide nucleic acid recognition sequence (aka targeting sequence) (e.g., 20 nucleotides (N20) immediately preceding an "NGG" motif or other PAM site on either DNA strand, or 20 nucleotides following a "TTN" motif or other PAM site on either DNA strand) can be extracted from target regions, and a digestion can be conducted or simulated using this most frequent guide. Short fragments can be removed, and the second most frequent guide can be found and used for a digestion. Short fragments can again be removed, and the third most frequent guide can be found and used for a digestion. This process can be iterated until the number of guides matches a preset number (e.g., a preset number determined by the capacity of a synthesis method such as an array), all remaining fragments are short, no guides can be found, or an acceptable amount of digestion or depletion is enabled by the guides found. This process can be conducted computationally, locating guides and simulating digestions on the target nucleic acid sequences. Multiple guides can be found in a given iteration. For example, each iteration can yield fewer potential guides, so in some after a few iterations multiple guides can found in a given iteration. In some cases, rather than determining the most frequent guide in an iteration, the guide identified is that which yields the most fragments below a certain threshold (e.g., short fragments) after cutting. This approach can give weight to more abundant sequences in the target sequences (e.g., cDNA from more abundant mRNA molecules for a transcriptome).

Short fragments can be nucleic acids less than about 10000 bp, 9000 bp, 8000 bp, 7000 bp, 6000 bp, 5000 bp, 4000 bp, 3000 bp, 2000 bp, 1000 bp, 500 bp, 450 bp, 400 bp, 350 bp, 300 bp, 250 bp, 200 bp, 150 bp, 100 bp, 90 bp, 80 bp, 70 bp, 60 bp, 50 bp, 40 bp, 30 bp, 20 bp, or 10 bp. The preset number of guides can be at least about 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, or 10000000. The acceptable amount of depletion can be at least about 10%, 20%, 30%, 40%, 50%, 60%, 70%, 80%, 90%, 95%, 99%, 99.9%, 99.99%, 99.999%, or 100%. The amount of depletion can, in some cases, be the percentage of starting target nucleic acids that are cleaved to short fragments.

### *Applications*

The gNAs (e.g., gRNAs) and collections of gNAs (e.g., gRNAs) provided herein are useful for a variety of applications, including depletion, partitioning, capture, or enrichment of target sequences of interest; genome-wide labeling; genome-wide editing; genome-wide function screens; and genome-wide regulation.

In one embodiment, the gNAs are selective for host nucleic acids in a biological sample from a host, but are not selective for non-host nucleic acids in the sample from a host. In one embodiment, the gNAs are selective for non-host nucleic acids from a biological sample from a host but are not selective for the host nucleic acids in the sample. In one embodiment, the gNAs are selective for both host nucleic acids and a subset of the non-host nucleic acids in a biological sample from a host. For example, where a complex biological sample comprises host nucleic acids and nucleic acids from more than one non-host organisms, the gRNAs may be selective for more than one of the non-host species. The gNAs may be used to serially deplete or partition the sequences that are not of interest. For example, saliva from a human contains human DNA, as well as the DNA of more than one bacterial species, but may also contain the genomic material of an unknown pathogenic organism. gNAs directed at the human DNA and the known bacteria can be used to serially deplete the human DNA, and the DNA of the known bacterial, thus resulting in a sample comprising the genomic material of the unknown pathogenic organism.

In an exemplary embodiment, the gNAs are selective for human host DNA obtained from a biological sample from the host, but do not hybridize with DNA from an unknown pathogen(s) also obtained from the sample.

The gNAs amay be useful for depleting and partitioning of targeted sequences in a sample, enriching a sample for non-host nucleic acids, or serially depleting targeted nucleic acids in a sample comprising: providing nucleic acids extracted from a sample; and contacting the sample with a plurality of complexes comprising (i) any one of the collection of gNAs described herein and (ii) nucleic acid-guided nuclease (e.g., CRISPR/Cas) system proteins.

The gNAs may be useful for method of depletion and partitioning of targeted sequences in a sample comprising: providing nucleic acids extracted from a sample, wherein the extracted nucleic acids comprise sequences of interest and targeted sequences for one of depletion and partitioning; contacting the sample with a plurality of complexes comprising (i) a collection of gNAs provided herein; and (ii) nucleic acid-guided nuclease (e.g., CRISPR/Cas) system proteins, under conditions in which the nucleic acid-guided nuclease system proteins cleave the nucleic acids in the sample.

In some cases, fusion proteins comprising domains from a nucleic acid-guided nuclease system protein (e.g., a CRISPR/Cas system protein) can be used with gNAs. Domains from nucleic acid-guided nuclease system proteins can include guide nucleic acid complexing domains, target nucleic acid recognition and binding domains, nuclease domains, and other domains. Domains can be from different variants of nucleic acid-guided nuclease system proteins, including but not limited to catalytically active variants, nickase variants, catalytically dead variants, and combinations thereof. Other domains in fusion proteins can come from proteins including restriction enzymes, other endonucleases (e.g., FokI), enzymes that modify DNA (e.g., methyltransferases), or tags (e.g., avidin, or fluorescent proteins such as GFP). As an example, nucleic acid-guided nuclease system protein domains for complexing with guide nucleic acids and binding to target nucleic acids can be combined in a fusion protein with nucleic acid cleaving or nicking domains from restriction enzymes. In some cases, the fusion protein comprises a catalytic domain of a restriction enzyme plus a nucleic acid guided nuclease domain. In some cases, the fusion protein comprises a catalytic domain of a restriction enzyme plus a catalytically-dead nucleic acid guided nuclease domain. For example, the catalytic domain of a restriction enzyme can be a catalytic domain of FokI. The nucleic acid guided nuclease domain can be a Cas9 domain, including a catalytically dead Cas9 domain. In some cases, the fusion protein comprises a catalytic domain of a restriction enzyme plus a nucleotide sequence recognition domain. In some cases, the fusion protein comprises a restriction enzyme domain plus a nucleic acid guided nuclease domain. The restriction enzyme domain can be a mutant that lacks a functioning nucleotide sequence recognition domain. For example, the restriction enzyme domain can be FokI, in some cases with a N13Y mutation to inactivate the nucleotide sequence recognition domain. In some cases, the fusion protein comprises a restriction enzyme domain plus a catalytically-dead nucleic acid guided nuclease domain. In some cases, the fusion protein comprises a restriction enzyme domain plus a nucleotide sequence recognition domain. The nucleotide sequence recognition domain can be from a restriction enzyme or a nucleic acid guided nuclease, for example.

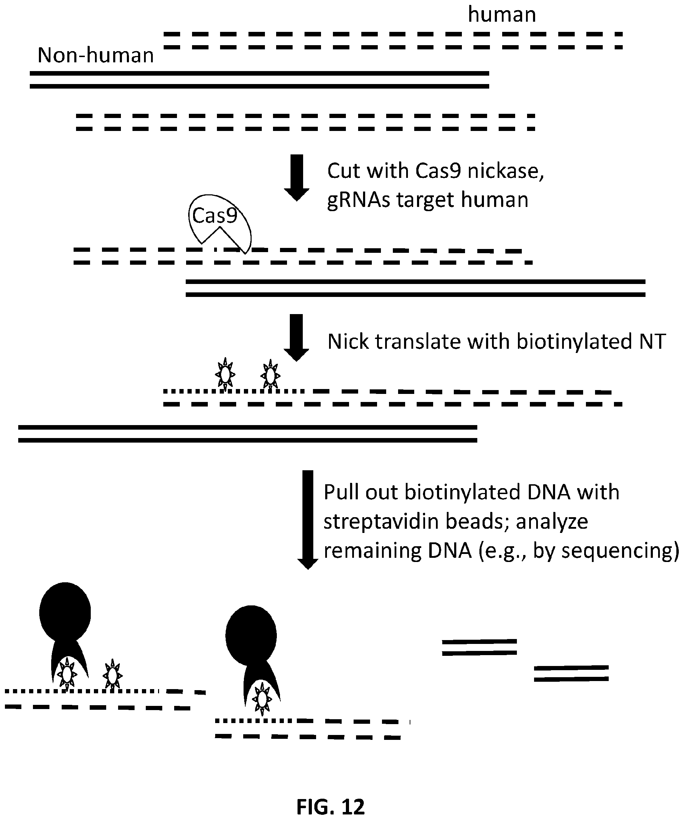

The gNAs amay be useful for depleting, partitioning, or capturing targeted nucleic acids (e.g., host nucleic acids) in a sample. For example, gNAs, comprising targeting sequences directed at the target (e.g., host) nucleic acids, are complexed with nucleic acid guided nickase system proteins and used to nick the target nucleic acids. Nick translation can then be conducted with labeled nucleotides, such as biotinylated nucleotides. The labeled nucleic acid sequences generated by nick translation can be used to bind the targeted sequences, such as with streptavidin. This binding can be used to capture the target nucleic acids. The captured target nucleic acids can then be separated from the non-captured nucleic acids. The non-captured nucleic acids (e.g., non-host nucleic acids) can be further analyzed, such as by sequencing. Alternatively or additionally, the captured target nucleic acids can also be further analyzed. **FIG. 12** shows an exemplary schematic of such a method. In **FIG. 12**, a sample comprising human and non-human nucleic acids is contacted with a nucleic acid guided nuclease nickase (e.g., Cas9 nickase) guided by human-targeted guide nucleic acids (e.g., gRNAs). At the nicked sites, nick translation is performed with labeled nucleotides (e.g., biotinylated nucleotides), and the labeled (e.g., biotinylated) nucleic acids can be captured using the labels (e.g., on a streptavidin substrate). The remaining non-human nucleic acids can then be further analyzed, for example by sequencing or other assay (e.g., hybridization, PCR).

Nucleic acids with hairpin loops (e.g., nanopore sequencing adapters) can also be targeted for depletion. A collection of nucleic acids (e.g., a sequencing library) with loops on one side of the nucleic acids (e.g., sequencing adapters) can be obtained. Then, second loops can be added to the other side of the nucleic acids, making the nucleic acids circular. The second loops can comprise a known restriction site or a particular nucleic acid-guided nuclease site. The collection of circular nucleic acids can then be contacted with target-specific (e.g., host-specific, human-specific) nucleic acid-guided nucleases or nickases. These nucleic acid-guided nucleases or nickases can cut or nick the targeted constituents of the nucleic acid collection while leaving the other nucleic acids in the collection intact. The cut or nicked nucleic acids can then be digested with exonucleases, while the intact nucleic acids remain undigested, thereby depleting the targeted nucleic acids from the collection. Then, the second loops can be removed by digestion at the restriction site or particular nucleic acid-guided nuclease site. The non-depleted nucleic acids (e.g., non-host nucleic acids) can then be further analyzed, such as by sequencing (e.g., sequencing on a nanopore sequencing platform). The adapters, such as the second loops, can also be designed such that any adapter dimers formed would result in a known site (e.g., a restriction enzyme site or a specific nucleic acid-guided nuclease site) in the adapter dimers, which can be digested by the appropriate restriction enzyme or nucleic acid-guided nuclease. Such an approach can also be employed for sequencing libraries for sequencing platforms that do not employ hairpin adapters, such as Illumina libraries, for example by amplifying the library after digesting the second loops.

Nucleic acids targeted for depletion can comprise human ribonucleic acids. In some cases, all human ribonucleic acids can be targeted for depletion.

Nucleic acids targeted for depletion comprise nucleic acids that are common or prevalent in a subject. For example, the depleted nucleic acids can comprise nucleic acids common to all cell types, or more abundant in typical or healthy cells, including but not limited to those associated with immune system factors (e.g., mRNA). Following depletion, the remaining nucleic acids to be analyzed can then comprise less common or less prevalent nucleic acids, such as cell type-specific nucleic acids. These less common nucleic acids can be signals of cell death, including cell death of one or more particular cell types. Such signals can be indicative of infections, cancers, and other diseases. In some cases, the signals are signals of cancer-related apoptosis in a particular tissue or tissues.

The gNAs may be useful for enriching a sample for non-host nucleic acids comprising: providing a sample comprising host nucleic acids and non-host nucleic acids; contacting the sample with a plurality of complexes comprising (i) a collection of gNAs provided herein comprising targeting sequences directed at the host nucleic acids; and (ii) nucleic acid-guided nuclease (e.g., CRISPR/Cas) system proteins, under conditions in which the nucleic acid-guided nuclease system proteins cleave the host nucleic acids in the sample, thereby depleting the sample of host nucleic acids, and allowing for the enrichment of non-host nucleic acids.

The gNAs may be useful for one method for serially depleting targeted nucleic acids in a sample comprising: providing a biological sample from a host comprising host nucleic acids and non-host nucleic acids, wherein the non-host nucleic acids comprise nucleic acids from at least one known non-host organism and nucleic acids from an unknown non-host organism; providing a plurality of complexes comprising (i) a collection of gNAs provided herein, directed at the host nucleic acids; and (ii) nucleic acid-guided nuclease (e.g., CRISPR/Cas) system proteins; mixing the nucleic acids from the biological sample with the gNA-nucleic acid-guided nuclease system protein complexes (e.g., gRNA-CRISPR/Cas system protein complexes) configured to hybridize to targeted sequences in the host nucleic acids, wherein at least a portion of the complexes hybridizes to the targeted sequences in the host nucleic acids, and wherein at least a portion of the host nucleic acids are cleaved; mixing the remaining nucleic acids from the biological sample with the gNA-nucleic acid-guided nuclease system protein complexes configured to hybridize to targeted sequences in the at least one known non-host nucleic acids, wherein at least a portion of the complexes hybridizes to the targeted sequences in the at least one non-host nucleic acids, and wherein at least a portion of the non-host nucleic acids are cleaved; and isolating the remaining nucleic acids from the unknown non-host organism and preparing for further analysis.

The gNAs generated herein may be used to perform genome-wide or targeted functional screens in a population of cells. Libraries of *in* vitro-transcribed gNAs (e.g., gRNAs) or vectors encoding the gNAs can be introduced into a population of cells via transfection or other laboratory techniques known in the art, along with a nucleic acid-guided nuclease (e.g., CRISPR/Cas) system protein, in a way that gNA-directed nucleic acid-guided nuclease system protein editing can be achieved to sequences across the entire genome or to a specific region of the genome. The nucleic acid-guided nuclease system protein can be introduced as a DNA. The nucleic acid-guided nuclease system protein can be introduced as mRNA. The nucleic acid-guided nuclease system protein can be introduced as protein. The nucleic acid-guided nuclease system protein may be Cas9. The nucleic acid-guided nuclease system protein may be Cpfl.

The gNAs generated herein may be used for the selective capture and/or enrichment of nucleic acid sequences of interest. The gNAs generated herein may be used for capturing target nucleic acid sequences comprising: providing a sample comprising a plurality of nucleic acids; and contacting the sample with a plurality of complexes comprising (i) a collection of gNAs provided herein; and (ii) nucleic acid-guided nuclease (e.g., CRISPR/Cas) system proteins. Once the sequences of interest are captured, they can be further ligated to create, for example, a sequencing library.

The gNAs generated herein may be used for introducing labeled nucleotides at targeted sites of interest comprising: (a) providing a sample comprising a plurality of nucleic acid fragments; (b) contacting the sample with a plurality of complexes comprising (i) a collection of gNAs provided herein; and (ii) nucleic acid-guided nuclease (e.g., CRISPR/Cas) system protein-nickases (e.g. Cas9-nickases), wherein the gNAs are complementary to targeted sites of interest in the nucleic acid fragments, thereby generating a plurality of nicked nucleic acid fragments at the targeted sites of interest; and (c) contacting the plurality of nicked nucleic acid fragments with an enzyme capable of initiating nucleic acid synthesis at a nicked site, and labeled nucleotides, thereby generating a plurality of nucleic acid fragments comprising labeled nucleotides in the targeted sites of interest.

The gNAs generated herein may be used for capturing target nucleic acid sequences of interest comprising: (a) providing a sample comprising a plurality of adapter-ligated nucleic acids, wherein the nucleic acids are ligated to a first adapter at one end and are ligated to a second adapter at the other end; and (b) contacting the sample with a collection of gNAs which comprise a plurality of dead nucleic acid-guided nuclease-gNA complexes (e.g., dCas9-gRNA complexes), wherein the dead nucleic acid-guided nuclease (e.g., dCas9) is fused to a transposase, wherein the gNAs are complementary to targeted sites of interest contained in a subset of the nucleic acids, and wherein the dead nucleic acid-guided nuclease-gNA transposase complexes (e.g., dCas9-gRNA transposase complexes) are loaded with a plurality of third adapters, to generate a plurality of nucleic acids fragments comprising either a first or second adapter at one end and a third adapter at the other end. The method may further comprise amplifying the product of step (b) using first or second adapter and third adapter-specific PCR.

The gNAs generated herein may be used to perform genome-wide or targeted activation or repression in a population of cells. Libraries of *in vitro*-transcribed gNAs (e.g., gRNAs) or vectors encoding the gNAs can be introduced into a population of cells via transfection or other laboratory techniques known in the art, along with a catalytically dead nucleic acid-guided nuclease (e.g., CRISPR/Cas) system protein fused to an activator or repressor domain (catalytically dead nucleic acid-guided nuclease system protein-fusion protein), in a way that gNA-directed catalytically dead nucleic acid-guided nuclease system protein-mediated activation or repression can be achieved at sequences across the entire genome or to a specific region of the genome. The catalytically dead nucleic acid-guided nuclease system protein -fusion protein can be introduced as DNA. The catalytically dead nucleic acid-guided nuclease system protein -fusion protein can be introduced as mRNA. The catalytically dead nucleic acid-guided nuclease system protein-fusion protein can be introduced as protein. the collection of gNAs or nucleic acids encoding for gNAs may exhibit specificity for more than one nucleic acid-guided nuclease system protein. The catalytically dead nucleic acid-guided nuclease system protein may be dCas9.

In some embodiments, the collection comprises gRNAs or nucleic acids encoding for gRNAs with specificity for Cas9 and one or more CRISPR/Cas system proteins selected from the group consisting of Cpfl, Cas3, Cas8a-c, Cas10, Cse1, Csy1, Csn2, Cas4, Csm2, and Cm5. In some embodiments, the collection comprises gRNAs or nucleic acids encoding for gRNAs with specificity for various catalytically dead CRISPR/Cas system proteins fused to different fluorophores, for example for use in the labeling and/or visualization of different genomes or portions of genomes, for use in the labeling and/or visualization of different chromosomal regions, or for use in the labeling and/or visualization of the integration of viral genes/genomes into a genome.

In some embodiments, the collection of gNAs (or nucleic acids encoding for gNAs) have specificity for different nucleic acid-guided nuclease (e.g., CRISPR/Cas) system proteins, and target different sequences of interest, for example from different species. For example, a first subset of gNAs from a collection of gNAs (or transcribed from a population of nucleic acids encoding such gNAs) targeting a genome from a first species can be first mixed with a first nucleic acid-guided nuclease system protein member (or an engineered version); and a second subset of gNAs from a collection of gNAs (or transcribed from a population of nucleic acids encoding such gNAs) targeting a genome from a second species can be mixed with a second different nucleic acid-guided nuclease system protein member (or an engineered version). In one embodiment, the nucleic acid-guided nuclease system proteins can be a catalytically dead version (for example dCas9) fused with different fluorophores, so that different targeted sequence of interest, e.g. different species genome, or different chromosomes of one species, can be labeled by different fluorescent labels. For example, different chromosomal regions can be labeled by different gRNA-targeted dCas9-fluorophores, for visualization of genetic translocations. For example, different viral genomes can be labeled by different gRNA-targeted dCas9-fluorophores, for visualization of integration of different viral genomes into the host genome. In another embodiment, the nucleic acid-guided nuclease system protein can be dCas9 fused with either activation or repression domain, so that different targeted sequence of interest, e.g. different chromosomes of a genome, can be differentially regulated. In another embodiment, the nucleic acid-guided nuclease system protein can be dCas9 fused different protein domain which can be recognized by different antibodies, so that different targeted sequence of interest, e.g. different DNA sequences within a sample mixture, can be differentially isolated.

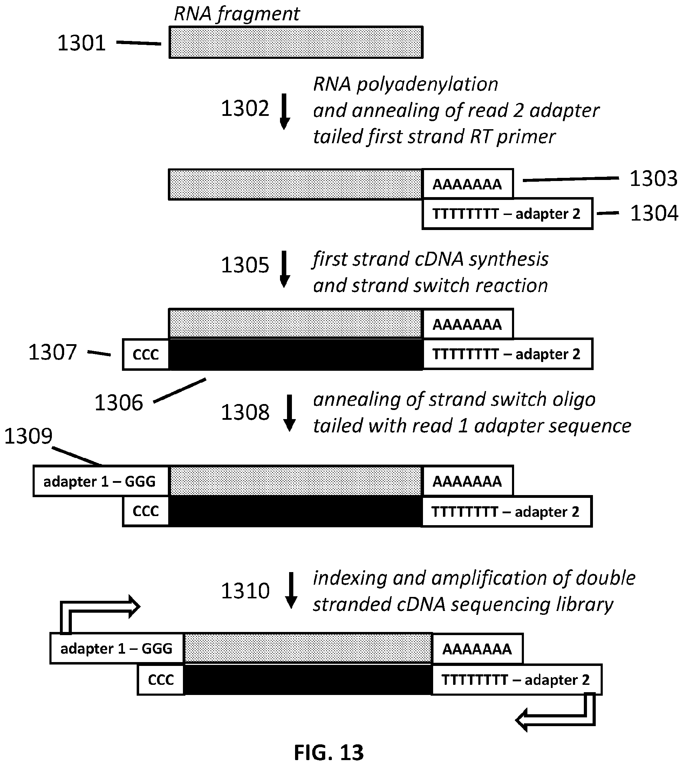

RNA can be prepared for sequencing (e.g., as cDNA) using a strand-switching method. **FIG. 13** shows an exemplary schematic of such a strand-switching method. RNA molecules **1301** can be polyadenylated **1302** or otherwise given a tail (e.g., a poly-A tail) **1303.** An oligonucleotide comprising an adapter (here, "Adapter 2") **1304** can be hybridized to the RNA tail, for example via a poly-T region of the oligonucleotide. Reverse transcription **1305** can then be used to synthesize cDNA **1306.** A region such as a poly-C region **1307** can be added to the cDNA for example by using MMLV as the reverse transcriptase, which can enable strand-switching. A strand-switching oligonucleotide **1309** can then be hybridized to the cDNA tail (e.g., the poly-C tail), for example via a poly-G region of the oligonucleotide. The strand-switching oligonucleotide can comprise an adapter (here, "Adapter 1"). The adapters can then be used for amplification and/or indexing **1310** of a double stranded cDNA sequencing library.

The adapters can comprise sequencing adapters (e.g., Illumina sequencing adapters). The adapters can comprise unique molecular identifier (UMI) sequences. The UMI sequences can comprise a sequence that is unique to each original RNA molecule (e.g., a random sequence). This can allow quantification of RNA amounts, free from sequencing bias. The adapters can comprise "barcode" sequences. The barcode sequences can comprise a barcode sequence that is shared among RNA molecules from a particular source (such as a subject, patient, environmental sample, partition (e.g., droplet, well, bead)). This can allow pooling of sequencing information for subsequent analysis, and can allow detection and elimination of cross-contamination. The adapters can comprise multiple distinct sequences, such as a UMI unique to each RNA molecule, a barcode shared among RNA molecules from a particular source, and a sequencing adapter.

The cDNA library can be further processed according to methods of the present disclosure, such as by targeted digestion or other depletion. For example, cDNA from a host (e.g., a human) can be digested or otherwise depleted, while cDNA from a non-host (e.g., an infectious agent) can remain. The cDNA can be sequenced or otherwise analyzed (e.g., hybridization assay, amplification assay).

Collections of gNAs, nucleic acid-guided nucleases, or complexes thereof can be arranged on one or more surfaces. Arrangement on surfaces can be used to control the amount, timing, and/or order with which a sample encounters the gNAs, nucleic acid-guided nucleases, or complexes thereof. For example, gNAs, nucleic acid-guided nucleases, or complexes thereof can be bound to the surface of a channel into which a sample is flowed; gNAs, nucleic acid-guided nucleases, or complexes thereof bound to the surface closer to the beginning of the channel will be encountered before those bound toward the end of the channel. In some cases, this approach can be used to cause a sample to encounter gNAs, nucleic acid-guided nucleases, or complexes thereof targeted to the most frequent recognition sequences, which can be designed and produced as discussed herein. In some cases, this approach can be used to cause a sample to encounter gNAs, nucleic acid-guided nucleases, or complexes thereof in different amounts or relative amounts, such as in proportion to the frequency of the gNA in the target nucleic acid. In an example, a first gNA-nucleic acid-guided nuclease complex is targeted to a sequence that appears twice as frequently in a target genome compared to a second gNA-nucleic acid-guided nuclease complex, and twice the number of the first complex is bound to a surface compared to the number of the second complex bound to the surface.

Collections of gNAs, nucleic acid-guided nucleases, or complexes thereof can be bound to a variety of surfaces, including but not limited to arrays, flow cells, channels, microfluidic channels, beads, and other substrates.

## Ligation-Free Targeted Sequencing

Targeted sequencing can be conducted without ligation of adapters. This can enable sequencing of otherwise difficult to sequence samples, such as highly degraded samples. Highly degraded DNA, in addition to containing primarily short fragments, often has cross-links to other molecules, making the end-to-end amplification required for sequencing libraries inefficient or impossible. Additionally, existing protocols can require conversion of the entire sample to DNA libraries by ligating adapters, followed by a time-consuming enrichment and multiple PCR amplifications.

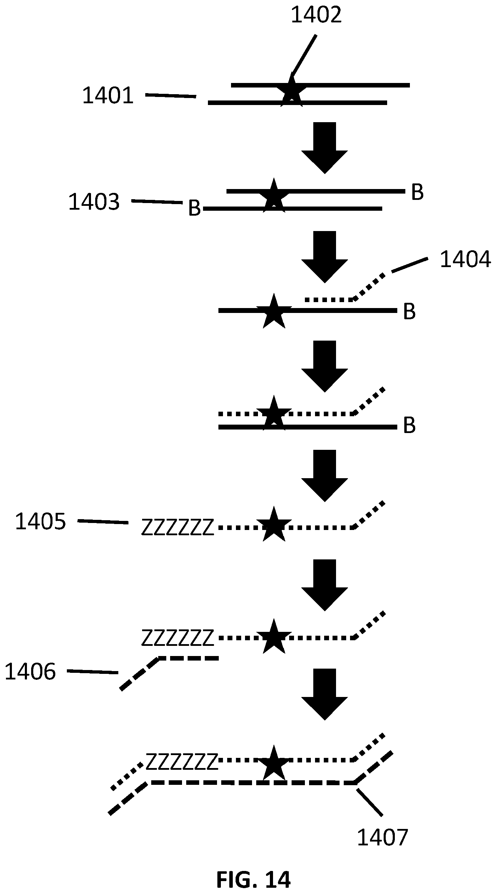

FIG. 14 illustrates a protocol that merges the library generation and enrichment to a single workflow, which can be faster and more efficient at recovering degraded DNA. First, 3' ends of DNA molecules **1401** in the extract are modified, so they are blocked **1403** and will not be extended by any polymerase. Next, a sequencing adapter-tailed primer **1404** is designed to bind near the site of interest **1402** (most often a SNP, but could be miniSTR or other site), and is extended past the site of interest to the end of the DNA fragment. After removing unused primers, a terminal transferase is added and only the extended primers will be given a tail **1405**, since other fragments are blocked. Removal of unused primers can be conducted enzymatically (e.g., by digestion with an exonuclease) or by binding of labeled nucleotides (e.g., biotinylated nucleotides) incorporated in the extension. The tail is used to reverse prime with another adapter-containing primer **1406**, converting the DNA into a library **1407** ready for amplification and sequencing. For higher sensitivity, a linear amplification step can be added by cycling the first extension step prior to removal of un-extended primer.

Primers can also incorporate barcode or unique molecular identifier sequences, enabling tracking of distribution of targeted sites to gain quantitative information, removal of amplification errors, and prevention of cross-contamination from other samples. With 2x 8-mer UMIs more than 4 billion combinations ($4^{16}$) per primer are possible, and as an additional metric the 3' breakpoint for the original molecule is known, making it virtually impossible to encounter the same combination multiple times. With a database of previously used UMIs for each primer, contamination from previously handled samples can be monitored. Importantly, these data can be stored without keeping identifiable information to protect privacy.

Such protocols can be used for forensics or other identification of individuals. For example, targeted sites of interest can include SNPs and other markers in mtDNA and Y chromosome sites for assignment of maternal and paternal haplogroups. MiniSTRs or other identifying regions can be employed. For degraded samples, it is often favorable to look at the mitochondrial DNA due to its high copy number and well-characterized haplogroup tree.

Such protocols can be used for disease diagnostics. For example, targeted sites of interest can include taxonomic markers including clade markers. Targeted sites of interest can include disease trait markers such as pathogenicity, virulence, resistance, strain identification, and other markers.

Sites of interest can be used to determine identity of a subject. In some cases, identity can be determined using identity by state (IBS) or identity-by-decent (IBD). In identifying different genealogical relationships, relationship can be defined as $R = (k_0, k_1, k_2)$, where $k_m$ matches the fraction of the genome where the two individuals share $m$ alleles. Table 4 has expected values for relationships typically relevant in forensics. This can be formulated in Bayesian terms as:

$$R = ((IBD = k_0|Data), (IBD = k_1|Data, P(IBD = k_2|Data).$$

Combining this with the expected values from table 4, we can setup a likelihood ratio test as:

$$LR = \frac{L(H(Data))}{L(H(Expected))} = \prod_{i=0}^{2} \frac{P(IBD = k_i|Data)}{P(IBD = k_i|Expected)}$$

A measure of significance is the obtained by making use of the following asymptotic property:

$$-2\log(LR) \sim \chi_d^2$$

where d is degrees of freedom.

Table 4. Expected allele sharing among related individuals.

| Relationship | $k_0$ | $k_1$ | $k_2$ |
|---|---|---|---|
| Self/mono-zygotic twin | 0 | 0 | 1 |
| Parent-Offspring | 0 | 1 | 0 |
| Full Siblings | 0.25 | 0.5 | 0.25 |
| Niece, nephew, uncle, aunt, grandparent, grandchild, half-sibling | 0.5 | 0.5 | 0 |
| First cousins | 0.75 | 0.25 | 0 |
| Unrelated | 1 | 0 | 0 |

High-throughput sequencing can enable analysis of a huge pool of degraded/trace forensics samples that are refractory to current STR-based genotyping methods. The SNP data generated by HTS also contains information that STR profiles do not, including ancestry and phenotype predictions that can be used to generate investigative leads. As such, the methods disclosed herein can serve as a supplement for samples where partial or no CODIS profile can be generated, and can add additional data for investigative leads in cases where no match is found in the CODIS database. However, for the forensics community to transition to HTS, it needs the tools to collect and analyze SNP data in the most efficient, inexpensive, and targeted way possible. The methods disclosed herein can give a reliable way of testing highly degraded samples, by focusing extraction methods on shorter DNA fragments and targeting sequencing to sites of interest, followed by analysis with a streamlined informatics pipeline backed by strong statistical analyses.

### *Exemplary Compositions*

Provided herein is a composition comprising a nucleic acid fragment, a nickase nucleic acid-guided nuclease-gNA complex, and labeled nucleotides. Provided herein is a composition comprising a nucleic acid fragment, a nickase Cas9-gRNA complex, and labeled nucleotides. The nucleic acid may comprise DNA. The nucleotides can be labeled, for example with biotin. The nucleotides can be part of an antibody-conjugate pair.

Provided herein is a composition comprising a nucleic acid fragment and a catalytically dead nucleic acid-guided nuclease-gNA complex, wherein the catalytically dead nucleic acid-guided nuclease is fused to a transposase. Provided herein is a composition comprising a DNA fragment and a dCas9-gRNA complex, wherein the dCas9 is fused to a transposase.

Provided herein is a composition comprising a nucleic acid fragment comprising methylated nucleotides, a nickase nucleic acid-guided nuclease-gNA complex, and unmethylated nucleotides. Provided herein is a composition comprising a DNA fragment comprising methylated nucleotides, a nickase Cas9-gRNA complex, and unmethylated nucleotides.

Provided herein is a gDNA complexed with a nucleic acid-guided -DNA endonuclease. The nucleic acid-guided -DNA endonuclease may be NgAgo.

Provided herein is a gDNA complexed with a nucleic acid-guided -RNA endonuclease.

Provided herein is a gRNA complexed with a nucleic acid-guided -DNA endonuclease.

Provided herein is a gRNA complexed with a nucleic acid-guided -RNA endonuclease. The nucleic acid-guided -RNA endonuclease may comprise C2c2.

Provided herein is a collection of gNAs produced or designed by the methods of the present disclosure.

### *Kits and Articles of Manufacture*

Provided herein are kits comprising any one or more of the compositions described herein, not limited to adapters, gNAs (e.g., gRNAs), gNA collections (e.g., gRNA collections), nucleic acid molecules encoding the gNA collections, and the like.

The kit may comprise a collection of DNA molecules capable of transcribing into a library of gRNAs wherein the gRNAs are targeted to human genomic or other sources of DNA sequences.

The kit may comprise a collection of gNAs wherein the gNAs are targeted to human genomic or other sources of DNA sequences.

Provided herein are kits comprising any of the collection of nucleic acids encoding gNAs, as described herein. Provided herein are kits comprising any of the collection of gNAs, as described herein.

Also provided are all essential reagents and instructions for carrying out the methods of making the gNAs and the collection of nucleic acids encoding gNAs, as described herein. Provided herein are kits that comprise all essential reagents and instructions for carrying out the methods of making individual gNAs and collections of gNAs as described herein.

Also provided herein is computer software monitoring the information before and after contacting a sample with a gNA collection produced herein. The software can compute and report the abundance of non-target sequence in the sample before and after providing gNA collection to ensure no off-target targeting occurs, and wherein the software can check the efficacy of targeted-depletion/enrichment/capture/partitioning/labeling/regulation/editing by comparing the abundance of the target sequence before and after providing gNA collection to the sample.

The following examples are included for illustrative purposes and are not intended to limit the scope of the invention.

## EXAMPLES

### Example 1: Construction of a gRNA library from a T7 promoter human DNA library

#### T7 promoter library construction

Human genomic DNA (400 ng) was fragmented using an S2 Covaris sonicator (Covaris) for 8 cycles, to yield fragments of 200-300 bp in length. Fragmented DNA was repaired using the NEBNext End Repair Module (NEB) and incubated at 25 °C for 30 min, then heat inactivated at 75 °C for 20 min. To make T7 promoter adapters, oligos T7-1 (5'GCCTCGAGC*T*A*ATACGACTCACTATAGAG3', * denotes a phosphorothioate backbone linkage) and T7-2 (sequence 5'Phos-CTCTATAGTGAGTCGTATTA3') were admixed at 15 $\mu$M, heated to 98 °C for 3 min then cooled slowly (0.1 °C/min) to 30 °C. T7 promoter blunt adapters (15 pmol total) were then added to the blunt-ended human genomic DNA fragments, and incubated with Blunt/TA Ligase Master Mix (NEB) at 25 °C for 30 min ((2) in FIG. 1). Ligations were amplified with 2 $\mu$M oligo T7-1, using Hi-Fidelity 2X Master Mix (NEB) for 10 cycles of PCR (98 °C for 20 s, 63 °C for 20 s, 72 °C for 35 s). Amplification was verified by running a small aliquot on agarose gel electrophoresis. PCR amplified products were recovered using 0.6X AxyPrep beads (Axygen) according to the manufacturer's instructions, and resuspended in 15 $\mu$L of 10 mM Tris-HCl pH 8. Other appropriate promoter sites besides T7 can also be used.

#### Digestion of DNA

PCR amplified T7 promoter DNA (2 $\mu$g total per digestion) was digested with 0.1 $\mu$L ofNt.CviPII (NEB) in 10 $\mu$L of NEB buffer 2 (50 mM NaCl, 10 mM Tris-HCl pH 7.9, 10 mM MgCl$_2$, 100 $\mu$g/mL BSA) for 10 min at 37 °C ((3) in FIG. 1), then heat inactivated at 75 °C for 20 min. An additional 10 $\mu$L of NEB buffer 2 with 1 $\mu$L of T7 Endonuclease I (NEB) was added to the reaction, and incubated at 37 °C for 20 min ((4) in FIG. 1). Enzymatic digestion of DNA was verified by agarose gel electrophoresis. Digested DNA was recovered by adding 0.6X AxyPrep beads (Axygen), according to the manufacturer's instructions, and resuspended in 15 $\mu$L of 10 mM Tris-HCl pH 8.

#### Ligation of adapters and removal of HGG

DNA was then blunted using T4 DNA Polymerase (NEB) for 20 min at 25 °C, followed by heat inactivation at 75 °C for 20 min ((5) in FIG. 1).

To make MlyI adapters, oligos MlyI-1 (sequence 5'>3', 5'Phos-GGGACTCGGATCCCTATAGTGATACAAAGACGATGACGACAAGCG) and MlyI-2 (sequence 5'>3', TCACTATAGGGATCCGAGTCCC) were admixed at 15 $\mu$M, heated to 98 °C for 3 min then cooled slowly (0.1 °C/min) to 30 °C. MlyI adapters (15 pmol total) were then added to T4 DNA Polymerase-blunted DNA, and incubated with Blunt/TA Ligase Master Mix (NEB) at 25 °C for 30 min ((6) in FIG. 1). Ligations were heat inactivated at 75 °C for 20 min, then digested with MlyI and XhoI (NEB) for 1 hr at 37 °C, so that HGG motifs are eliminated ((7) in FIG. 1). Digests were then cleaned using 0.8X AxyPrep beads (Axygen), and DNA was resuspended in 10 $\mu$L of 10 mM Tris-Cl pH 8.

To make StlgR adapters, oligos stlgR (sequence 5'>3', 5'Phos-GTTTTAGAGCTAGAAATAGCAAGTTAAAATAAGGCTAGTCCGTTATCAACTTGAAA AAGTGGCACCGAGTCGGTGCTTTTTTTGGATCCGATGC) and stlgRev (sequence 5'>3', GGATCCAAAAAAAGCACCGACTCGGTGCCACTTTTTCAAGTTGATAACGGACTAGC CTTATTTTAACTTGCTATTTCTAGCTCTAAAAC) were admixed at 15 $\mu$M, heated to 98 °C for 3 min then cooled slowly (0.1 °C/min) to 60 °C. StlgR adapters (5 pmol total) were added to HGG-removed DNA fragments, and incubated with Blunt/TA Ligase Master Mix (NEB) at 25 °C for 30 min ((8) in **FIG. 1**). Ligations were then incubated with Hi-Fidelity 2X Master Mix (NEB), using 2 μM of both oligos T7-1 and gRU (sequence 5'>3', AAAAAAAGCACCGACTCGGTG), and amplified using 20 cycles of PCR (98 °C for 20 s, 60 °C for 20 s, 72 °C for 35 s). Amplification was verified by running a small aliquot on agarose gel electrophoresis. PCR amplified products were recovered using 0.6X AxyPrep beads (Axygen) according to the manufacturer's instructions, and resuspended in 15 μL of 10mM Tris-HCl pH 8.

### *In vitro transcription*

The T7/gRU amplified library of PCR products was then used as template for *in vitro* transcription, using the HiScribe T7 *In Vitro* Transcription Kit (NEB). 500-1000 ng of template was incubated overnight at 37 °C according to the manufacturer's instructions. To transcribe the guide libraries into gRNAs, the following *in vitro* transcription reaction mixture was assembled: 10 μL of purified library (~500 ng), 6.5 μL of $H_2O$, 2.25 μL of ATP, 2.25 μL of CTP, 2.25 μL of GTP, 2.25 μE of UTP, 2.25 μL of 10X reaction buffer (NEB) and 2.25 μL of T7 RNA Polymerase mix. The reaction was incubated at 37 °C for 24 hr, then purified using the RNA cleanup kit (Life Technologies), eluted with 100 μL of RNase-free water, quantified and stored at -20 °C until use.

### Example 2: Construction of gRNA library from intact human genomic DNA *Digestion of DNA*

Human genomic DNA ((1) in **FIG. 2**; 20 μg total per digestion) was digested with 0.1 μL of Nt.CviPII (NEB) in 40 μL of NEB buffer 2 (50 mM NaCl, 10 mM Tris-HCl pH 7.9, 10 mM $MgCl_2$, 100 μg/mL BSA) for 10 min at 37 °C, then heat inactivated at 75 °C for 20 min. An additional 40 μL of NEB buffer 2 and 1 μL of T7 Endonuclease I (NEB) was added to the reaction, with 20 min incubation at 37 °C (e.g., (2) in **FIG. 2**). Fragmentation of genomic DNA was verified with a small aliquot by agarose gel electrophoresis. DNA fragments between 200 and 600 bp were recovered by adding 0.3X AxyPrep beads (Axygen), incubating at 25 °C for 5 min, capturing beads on a magnetic stand and transferring the supernatant to a new tube. DNA fragments below 600 bp do not bind to beads at this bead/DNA ratio and remain in the supernatant. 0.7X AxyPrep beads (Axygen) were then added to the supernatant (this will bind all DNA molecules longer than 200 bp), allowed to bind for 5 min. Beads were captured on a magnetic stand and washed twice with 80% ethanol, air dried. DNA was then resuspended in 15 μL of 10 mM Tris-HCl pH 8. DNA concentration was determined using a Qbit assay (Life Technologies).

### *Ligation of adapters*

To make T7/MlyI adapters, oligos MlyI-1 (sequence 5'>3', 5'Phos-GGGGGACTCGGATCCCTATAGTGATACAAAGACGATGACGACAAGCG) and T7-7 (sequence 5'>3', GCCTCGAGC*T*A*ATACGACTCACTATAGGGATCCAAGTCCC, * denotes a phosphorothioate backbone linkage) were admixed at 15 μM, heated to 98 °C for 3 min then cooled slowly (0.1 °C/min) to 30 °C. The purified, Nt.CviPII/T7 Endonuclease I digested DNA (100 ng) was then ligated to 15 pmol of T7/MlyI adapters using Blunt/TA Ligase Master Mix (NEB) at 25 °C for 30 min ((3) in FIG. 2). Ligations were then amplified by 10 cycles of PCR (98 °C for 20 s, 60 °C for 20 s, 72 °C for 35 s) using Hi-Fidelity 2X Master Mix (NEB), and 2 μM of both oligos T7-17 (GCCTCGAGC*T*A*ATACGACTCACTATAGGG * denotes a phosphorothioate backbone linkage) and Flag (sequence 5'>3', CGCTTGTCGTCATCGTCTTTGTA). PCR amplification increases the yield of DNA and, given the nature of the Y-shaped adapters we used, always resulted in T7 promoter being added distal to the HGG site and MlyI site being added next to the HGG motif ((4) in **FIG. 2**).

PCR products were then digested with MlyI and XhoI (NEB) for 1 hr at 37 °C, and heat inactivated at 75 °C for 20 min ((5) in FIG. 2). Following that, 5 pmol of adapter StlgR (in Example 1) was ligated using Blunt/TA Ligase Master Mix (NEB) at 25 °C for 30 min ((6) in **FIG. 2**). Ligations were then amplified by PCR using Hi-Fidelity 2X Master Mix (NEB), 2 μM of both oligos T7-7 and gRU (in Example 1) and 20 cycles of PCR (98 °C for 20 s, 60 °C for 20 s, 72 °C for 35 s). Amplification was verified by running a small aliquot on agarose gel electrophoresis. PCR amplified products were recovered using 0.6X AxyPrep beads (Axygen) according to the manufacturer's instructions, and resuspended in 15 μL of 10 mM Tris-HCl pH 8.

Samples were then used as templates for *in vitro* transcription reaction as described in Example 1.

### Example 3: Direct Cutting with CviPII

30 μg of human genomic DNA was digested with 2 units of NtCviPII (New England Biolabs) for 1 hour at 37 °C, followed by heat inactivation at 75 °C for 20 minutes. The size of the fragments was verified to be 200-1,000 base pairs using a fragment analyzer instrument (Advanced Analytical). The 5' or 3' protruding ends (as shown, for example, in **FIG. 3**) were converted to blunt ends by adding 100 units of T4 DNA polymerase (New England Biolabs), 100 $\mu$M dNTPs and incubating at 12 °C for 30 minutes. DNA was then recovered using a PCR cleanup kit (Zymo) and eluted in 20 $\mu$L elution buffer. The DNA was then ligated to MlyI adapter (see, for example, Example 4) or FokI/MmeI or BaeI/EcoP15I adapters (see, for example, Example 5).

### Example 4: Use of MlyI Adapter

Adapter MlyI was made by combining 2 $\mu$moles of MlyI Ad1 and MlyAd2 in 40 $\mu$L water. Adapter BsaXI/MmeI was made by combining 2 $\mu$moles oligo BsMm-Ad1 and 2 $\mu$moles oligo BsMm-Ad2 in 40 $\mu$L water. T7 adapter was made by combining 1.5 $\mu$moles of T7-Ad1 and T7-Ad2 oligos in 100 $\mu$L water. Stem-loop adapter was made by combining 1.5 $\mu$moles of gR-top and gR-bot oligos in 100 $\mu$L water. In all cases, after mixing adapters were heated to 98 °C for 3 min then cooled to room temperature at a cooling rate of 1 °C/min in a thermal cycler. Other appropriate promoter sites besides T7 can also be used.

The DNA containing the CCD blunt ends (from earlier section) was then ligated to 50 pmoles of adapter MlyI, using the blunt/TA ligation master mix (New England Biolabs) at room temperature for 30 minutes. The DNA was then recovered by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4 (50 mM potassium acetate, 20 mM Tris-acetate, 10 mM magnesium acetate, 100 $\mu$g/mL BSA, pH 7.9). These steps eliminate small (<100 nucleotides) DNA and MlyI adapter dimers.

Purified DNA was then digested by adding 20 units of MlyI (New England Biolabs) and incubating at 37 °C for 1 hour to eliminate both the adapter derived sequences and the CCD (and complementary HGG) motifs. DNA was recovered from the digest by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 30 $\mu$L buffer 4.

The purified DNA was then ligated to 50 pmoles of adapter BsaXI/MmeI, using the blunt/TA ligation master mix (New England Biolabs) at room temperature for 30 minutes. The DNA was then recovered by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4 (50 mM potassium acetate, 20 mM Tris-acetate, 10 mM magnesium acetate, 100 $\mu$g/mL BSA, pH 7.9). DNA was then digested by addition of 20 units MmeI (New England Biolabs) and 40 pmol/$\mu$L SAM (S-adenosyl methionine) at 37 °C for 1 hour, followed by heat inactivation at 75 °C for 20 minutes. DNA was then ligated to 30 pmoles T7 adapter using the blunt/TA ligation master mix (New England Biolabs) at room temperature for 30 minutes. DNA was then recovered using a PCR cleanup kit (Zymo) and eluted in 20 $\mu$L buffer 4, then digested with 20 units of BsaXI for 1 hour at 37 °C. The guide RNA stem-loop sequences were added by adding 15 pmoles stem-loop adapter and using the blunt/TA ligation master mix (New England Biolabs) at room temperature for 30 min. DNA was then recovered using a PCR cleanup kit (Zymo), eluted in 20 $\mu$L elution buffer and PCR amplified using HiFidelity 2X master mix (New England Biolabs). Primers T7-Ad1 and gRU (sequence 5'>3' AAAAAAGCACCGACTCGGTG) were used to amplify with the following settings (98 °C 3 min; 98 °C for 20 sec, 60 °C for 30 secs, 72 °C for 20 sec, 30 cycles). The PCR amplicon was cleaned up using the PCR cleanup kit and verified by DNA sequencing, then used as template for an in vitro transcription reaction to generate guide RNAs.

### Example 5: Use of BaeI/EcoP15I Adapter

Adapter BaeI/EcoP15I was made by combining 2 $\mu$moles of BE Ad1 and BE Ad2 in 40 $\mu$L water. T7-E adapter was made by combining 1.5 $\mu$moles of T7-Ad3 and T7-Ad4 oligos in 100 $\mu$L water. In all cases, after mixing adapters were heated to 98 °C for 3 min then cooled to room temperature at a cooling rate of 1 °C/min in a thermal cycler. Other appropriate promoter sites besides T7 can also be used.

**Table 5.** Oligonucleotides used with BaeI/EcoP15I Adapter.

| Oligo name | Sequence (5'>3') | Modification |
|---|---|---|
| BE Ad1 | ActgctgacACAAgtatcTTTTTTTTTTTgtttaaac TTTTTTTTTTgatacACAAgtcagcagA | 5'phosphate |
| Be Ad2 | TctgctgacTTGTgtatcAAAAAAAAAAgtttaaac AAAAAAAAAAgatacTTGTgtcagcagT | 5'phosphate |
| T7-Ad3 | gcctcgagctaatacgactcactatagag | none |

(continued)

| Oligo name | Sequence (5'>3') | Modification |
|---|---|---|
| T7-Ad4 | NNctctatagtgagtcgtatta | 5'phosphate |
| stlgR | ttagagctagaaatagcaagttaaaataaggctagtccgttatc aacttgaaaaagtggcaccgagtcggtgctttttt | 5'adenylation |

The DNA containing the CCD blunt ends (from earlier section) was then ligated to 50 pmoles of adapter BaeI/EcoP15I, using the blunt/TA ligation master mix (New England Biolabs) at room temperature for 30 minutes. The DNA was then recovered by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 50 μL buffer 4 (50 mM potassium acetate 20 mM Tris-acetate, 10 mM magnesium acetate, 100 μg/mL BSA, pH 7.9). Recovered DNA was then digested with 20 units PmeI for 30 min at 37 °C; DNA was then recovered by incubating with 1.2X Kapa SPRI beads (Kapa Biosystems) for 5 minutes, capturing the beads with a magnetic rack, washing twice with 80% ethanol, air drying the beads for 5 minutes and finally resuspending the DNA in 50 μL buffer 4. These steps eliminate small (<100 nucleotides) DNA and BaeI/EcoP15I adapter multimers.

DNA was then digested by addition of 20 units EcoP15I (New England Biolabs) and 1 mM ATP at 37 °C for 1 hour, followed by heat inactivation at 75 °C for 20 minutes. DNA was then ligated to 30 pmoles T7-E adapter using the blunt/TA ligation master mix (New England Biolabs) at room temperature for 30 minutes. DNA was then recovered using a PCR cleanup kit (Zymo) and eluted in 20 μL buffer 4.

Purified DNA was then digested by adding 20 units of BaeI (New England Biolabs), 40 pmol/μL SAM (S-adenosyl methionine) and incubating at 37 °C for 1 hour to eliminate both the adapter derived sequences and the CCD (and complementary HGG) motifs. DNA was then recovered using a PCR cleanup kit (Zymo) and eluted in 20 μL elution buffer.

Recovered DNA was then ligated to the stlgR oligo using Thermostable 5' AppDNA/RNA Ligase

(New England Biolabs) by adding 20 units ligase, 20 pmol stlgR oligo, in 20 μL ss ligation buffer (10 mM Bis-Tris-Propane-HCl, 10 mM MgCl$_2$, 1 mM DTT, 2.5 mM MnCl$_2$, pH 7 @ 25 °C) and incubating at 65 °C for 1 hour followed by heat inactivation at 90 °C for 5 min. DNA product was then PCR amplified using HiFidelity 2X master mix (New England Biolabs). Primers T7-Ad3 and gRU (sequence 5'>3' AAAAAAGCACCGACTCGGTG) were used to amplify with the following settings (98 °C 3 min; 98 °C for 20 sec, 60 °C for 30 secs, 72 °C for 20 sec, 30 cycles). The PCR amplicon was cleaned up using the PCR cleanup kit and verified by DNA sequencing, then used as template for an in vitro transcription reaction to generate the guide RNAs.

**Example 6: Use of FokI/MmeI Adapter**

Adapter FokI/MmeI was made by diluting 2 μmoles of circMF oligo in 40 μL water. T7-E adapter was made by combining 1.5 μmoles of T7-Ad3 and T7-Ad4 oligos in 100 μL water. N4stlgR adapter was made by combining 1.5 μmoles of N4UstlgR and MNA oligos in 100 μL water. In all cases, after mixing adapters were heated to 98 °C for 3 min then cooled to room temperature at a cooling rate of 1 °C/min in a thermal cycler. Other appropriate promoter sites besides T7 can also be used.

**Table 6**. Oligonucleotides used with FokI/MmeI Adapter.

| Oligo name | Sequence (5'>3') | Modification |
|---|---|---|
| circMF | TtggatcatcctgtgAAGCTTTTTCTTTTTCTTTTCACTGCGCG AATCTGCATTcacaggatgatccaA | 5'phosphate |
| T7-Ad3 | gcctcgagctaatacgactcactatagagNN | none |
| T7-Ad4 | ctctatagtgagtcgtatta | 5'phosphate |
| N4UstlgR | NNNNGUTTTAGAGCTAGAAATAGCAAGTTAAAATAA GGCTAGTCCGTTATCAACTTGAAAAAGTGGCACCGAG TCGGTGCTTTTTTT | 5'phosphate |

(continued)

| Oligo name | Sequence (5'>3') | Modification |
|---|---|---|
| MNA | GAGATCAGCTTCTGCATTGATGCGGCCG CTTATTTTAACTTGCTATTTCTAGCTCTAAAAC | none |

The DNA containing the CCD blunt ends (from earlier section) was then ligated to 50 pmoles of adapter Fokl/Mmel, using the blunt/TA ligation master mix (New England Biolabs) at room temperature for 20 minutes. Ligation reaction were then terminated by adding 50 ul buffer 4 (50 mM potassium acetate 20 mM Tris-acetate, 10 mM magnesium acetate, 100 $\mu$g/mL BSA, pH 7.9) supplemented with 10 units Mfel and 10 units lambda exonuclease (New England Biolabs) and incubated at 37 °C for 30 min. The DNA was then recovered by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4. Recovered DNA was then digested with 20 units Pmel for 30 min at 37 °C; DNA was then recovered by incubating with 1.2X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4. These steps eliminate non-ligated DNA, non-ligated PokI/Mmel adapters, FokI/Mmel adapter multimer and partially ligated DNA.

DNA was then digested by addition of 20 units Mmel (New England Biolabs) and 0.05 mM SAM (S-adenosyl methionine) at 37 °C for 45 min, followed by heat inactivation at 75 °C for 20 minutes. DNA was then ligated to 30 pmoles T7-E adapter using the blunt/TA ligation master mix (New England Biolabs) at room temperature for 30 minutes. DNA was then recovered by incubating with 1.6X Kapa SPRI beads (Kapa Biosystems) for 5 minutes, capturing the beads with a magnetic rack, washing twice with 80% ethanol, air drying the beads for 5 minutes and finally resuspending the DNA in 20 $\mu$L buffer 4.

Purified DNA was then digested by adding 20 units of FokI (New England Biolabs) and incubating at 37 °C for 20 min to eliminate both the adapter derived sequences and the CCD (and complementary HGG) motifs. DNA was then ligated to 10 pmoles N4stlgR adapter using the Quick ligation kit (New England Biolabs) at room temperature for 20 min. Reaction was then heat inactivated at 75 °C for 20 min.

Ligated DNA was then treated with USER enzyme (New England Biolabs), which excises Uracil (U) residues, to eliminate N4stlgR adapter dimers. DNA was then recovered by incubating with 1.6X Kapa SPRI beads (Kapa Biosystems) for 5 minutes, capturing the beads with a magnetic rack, washing twice with 80% ethanol, air drying the beads for 5 minutes and finally resuspending the DNA in 20 $\mu$L buffer 4.

DNA product was then PCR amplified using HiFidelity 2X master mix (New England Biolabs). Primers T7-Ad3 and gRU (sequence 5'>3' AAAAAAGCACCGACTCGGTG) were used to amplify with the following settings (98 °C 3 min; 98 °C for 20 sec, 60 °C for 30 secs, 72 °C for 20 sec, 30 cycles). The PCR amplicon was cleaned up using the PCR cleanup kit and verified by DNA sequencing, then used as template for an in vitro transcription reaction to generate the guide RNAs.

## Example 7: NEMDA Method (Bael/EcoP15I)

NEMDA (Nicking Endonuclease Mediated DNA Amplification) was performed using 50 ng of human genomic DNA. The DNA was incubated in 100 $\mu$L thermo polymerase buffer (20 mM Tris-HCl, 10 mM $(NH_4)_2SO_4$, 10 mM KCl, 6 mM $MgSO_4$, 0.1% Triton® X-100, pH 8.8) supplemented with 0.3 mM dNTPs, 40 units of Bst large fragment DNA polymerase, and 0.1 units of NtCviPII (New England Biolabs) at 55 °C for 45 min, followed by 65 °C for 30 min and finally 80 °C for 20 min in a thermal cycler.

The DNA was then diluted with 300 $\mu$L of buffer 4 supplemented with 200 pmoles of T7-RND8 oligo (sequence 5'>3' gcctcgagctaatacgactcactatagagnnnnnnnn) and boiled at 98 °C for 10 min followed by rapid cooling to 10 °C for 5 min. Other appropriate promoter sites besides T7 can also be used. The reaction was then supplemented with 40 units of E. coli DNA polymerase I and 0.1 mM dNTPs (New England Biolabs) and incubated at room temperature for 20 min followed by heat inactivation at 75 °C for 20 min. DNA was then recovered using a PCR cleanup kit (Zymo) and eluted in 30 $\mu$L elution buffer.

DNA was then ligated to 50 pmoles of adapter Bael/EcoP15I, using the blunt/TA ligation master mix (New England Biolabs) at room temperature for 30 minutes. The DNA was then recovered by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4 (50 mM potassium acetate, 20 mM Tris-acetate, 10 mM magnesium acetate, 100 $\mu$g/mL BSA, pH 7.9). Recovered DNA was then digested with 20 units Pmel for 30 min at 37 °C; DNA was then recovered by incubating with 1.2X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4. These steps eliminate small (<100 nucleotides) DNA and BaeI/EcoP15I adapter multimers.

Purified DNA was then digested by adding 20 units of BaeI (New England Biolabs), 40 pmol/$\mu$L SAM (S-adenosyl methionine) and incubating at 37 °C for 1 hour to eliminate both the adapter derived sequences and the CCD (and complementary HGG) motifs. DNA was then recovered using a PCR cleanup kit (Zymo) and eluted in 20 $\mu$L elution buffer.

Recovered DNA was then ligated to the stlgR oligo using Thermostable 5' AppDNA/RNA Ligase (New England Biolabs) by adding 20 units ligase, 20 pmol stlgR oligo, in 20 $\mu$L ss ligation buffer (10 mM Bis-Tris-Propane-HCl, 10 mM MgCl$_2$, 1 mM DTT, 2.5 mM MnCl$_2$, pH 7 @ 25 °C) and incubating at 65 °C for 1 hour followed by heat inactivation at 90 °C for 5 min. DNA product was then PCR amplified using HiFidelity 2X master mix (New England Biolabs). Primers T7-Ad3 (sequnce 5'>3' gcctcgagctaatacgactcactatagag ) and gRU (sequence 5'>3' AAAAAAGCACCGACTCGGTG) were used to amplify with the following settings (98 °C for 3 min; 98 °C for 20 sec, 60°C for 30 secs, 72 °C for 20 sec, 30 cycles). The PCR amplicon was cleaned up using the PCR cleanup kit and verified by DNA sequencing, then used as template for an in vitro transcription reaction to generate the guide RNAs.

### Example 8: NEMDA Method (FokI/MmeI)

NEMDA (Nicking Endonuclease Mediated DNA Amplification) was performed using 50 ng of human genomic DNA. The DNA was incubated in 100 $\mu$L thermo polymerase buffer (20 mM Tris-HCl, 10 mM (NH$_4$)$_2$SO$_4$, 10 mM KCl, 6 mM MgSO$_4$, 0.1% Triton$^®$ X-100, pH 8.8) supplemented with 0.3 mM dNTPs, 40 units of Bst large fragment DNA polymerase, and 0.1 units of NtCviPII (New England Biolabs) at 55 °C for 45 min, followed by 65 °C for 30 min and finally 80 °C for 20 min in a thermal cycler.

The DNA was then diluted with 300 $\mu$L of buffer 4 supplemented with 200 pmoles of T7-RND8 oligo (sequence 5'>3' gcctcgagctaatacgactcactatagagnnnnnnnn) and boiled at 98 °C for 10 min followed by rapid cooling to 10 °C for 5 min. Other appropriate promoter sites besides T7 can also be used. The reaction was then supplemented with 40 units of *E. coli* DNA polymerase I and 0.1 mM dNTPs (New England Biolabs) and incubated at room temperature for 20 min followed by heat inactivation at 75 °C for 20 min. DNA was then recovered using a PCR cleanup kit (Zymo) and eluted in 30 $\mu$L elution buffer.

DNA was then ligated to 50 pmoles of adapter FokI/MmeI, using the blunt/TA ligation master mix (New England Biolabs) at room temperature for 30 minutes. Ligation reaction were then terminated by adding 50 ul buffer 4 (50 mM potassium acetate 20 mM Tris-acetate, 10 mM magnesium acetate, 100 $\mu$g/mL BSA, pH 7.9) supplemented with 10 units MfeI and 10 units lambda exonuclease (New England Biolabs) and incubated at 37 °C for 30 min. The DNA was then recovered by incubating with 0.6X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4. Recovered DNA was then digested with 20 units PmeI for 30 min at 37 °C; DNA was then recovered by incubating with 1.2X Kapa SPRI beads (Kapa Biosystems) for 5 minutes, capturing the beads with a magnetic rack, washing twice with 80% ethanol, air drying the beads for 5 minutes and finally resuspending the DNA in 50 $\mu$L buffer 4. These steps eliminate non-ligated DNA, non-ligated FokI/MmeI adapters, FokI/MmeI adapter multimer and partially ligated DNA.

Purified DNA was then digested by adding 20 units of FokI (New England Biolabs) and incubating at 37 °C for 20 min to eliminate both the adapter derived sequences and the CCD (and complementary HGG) motifs. DNA was then ligated to 10 pmoles N4stlgR adapter using the Quick ligation kit (New England Biolabs) at room temperature for 20 min. Reaction was then heat inactivated at 75 °C for 20 min.

Ligated DNA was then treated with USER enzyme (New England Biolabs), which excises Uracil (U) residues, to eliminate N4stlgR adapter dimers. DNA was then recovered by incubating with 1.6X Kapa SPRI beads (Kapa Biosystems) for 5 minutes, capturing the beads with a magnetic rack, washing twice with 80% ethanol, air drying the beads for 5 minutes and finally resuspending the DNA in 20 $\mu$L buffer 4. DNA product was then PCR amplified using HiFidelity 2X master mix (New England Biolabs). Primers T7-Ad3 (sequence 5'>3' gcctcgagctaatacgactcactatagag ) and gRU (sequence 5'>3' AAAAAAGCACCGACTCGGTG) were used to amplify with the following settings (98 °C for 3 min; 98 °C for 20 sec, 60°C for 30 secs, 72 °C for 20 sec, 30 cycles). The PCR amplicon was cleaned up using the PCR cleanup kit and verified by DNA sequencing, then used as template for an in vitro transcription reaction to generate the guide RNAs.

### Claims

**1.** A method of making a collection of nucleic acids, comprising:

a. obtaining target nucleic acids, each comprising a PAM site of a nucleic acid-guided nuclease;

b. hybridizing first primers to the PAM sites of the target nucleic acids, wherein the first primers comprise (i) a MAP site that is complementary to the PAM site, (ii) a complementary recognition site that is complementary to a recognition site of the nucleic acid guided nuclease, and (iii) a complementary promoter site that is complementary to a promoter site;

c. extending the first primers using the target nucleic acids as template, thereby producing first extension products comprising sequence of the first primer and sequence complementary to the target nucleic acids;

d. hybridizing second primers to the first extension products; and

e. extending the second primers using the first extension products as template, thereby producing second extension products comprising the PAM site, the recognition site, and the promoter site.

2. The method of claim 1, wherein the second primers comprise (i) a PAM site of the nucleic acid-guided nuclease and (ii) a random sequence, optionally wherein the random sequence is between about 6 and about 8 bases long.

3. The method of claim 1, wherein the first primers further comprise a restriction enzyme site of a restriction enzyme.

4. The method of claim 1, further comprising:

f. ligating an adapter to the second extension products, wherein the adapter comprises a restriction enzyme site of a restriction enzyme; and

g. cutting the second extension products with the restriction enzyme such that the PAM site and the restriction site are cleaved from the recognition site.

5. The method of claim 3 or claim 4, wherein the restriction enzyme comprises MmeI, FokI or MlyI.

6. The method of claim 1, further comprising removing unbound first primers or unbound second primers.

7. The method of claim 1, wherein the extending the first primers or the extending the second primers is conducted with labeled nucleotides, optionally wherein the labeled nucleotides comprise biotinylated nucleotides.

8. The method of claim 1, wherein the recognition site is about 20 nucleotides in length or from about 15 to about 25 nucleotides in length.

9. The method of claim 1, wherein the nucleic acid-guided nuclease comprises a Cas system protein, optionally wherein the nucleic acid-guided nuclease comprises a Cas9 system protein.

10. The method of claim 1, wherein the target nucleic acids comprise genomic DNA, cDNA, human DNA, host DNA or eukaryotic DNA.

11. The method of claim 1, wherein the complementary recognition site comprises at least one modified nucleic acid bond, optionally wherein the modified nucleic acid bond is selected from the group consisting of locked nucleic acid (LNA), bridged nucleic acid (BNA), peptide nucleic acid (PNA), zip nucleic acid (ZNA), glycol nucleic acid (GNA), threose nucleic acid (TNA), and phosphorothioate (PTO).

12. The method of claim 1, further comprising transcribing the second extension products using the promoter site.

13. The method of any one of claims 1 to 12, wherein:

a) the PAM site comprises NGG or NAG;

b) the collection of nucleic acids comprises at least $10^5$ unique nucleic acids; and/or

c) the target nucleic acids are spaced at least every 10,000 bp across a genome of interest.

14. The method of any one of claims 1-13, wherein the target nucleic acids comprise one or more of ribosomal DNA, centromeric DNA, Alu DNA, long interspersed nuclear elements (LINE) DNA, and short interspersed nuclear elements (SINE) DNA.

**Patentansprüche**

1. Verfahren zur Herstellung einer Sammlung von Nukleinsäuren, umfassend:

   a. Erhalten von Zielnukleinsäuren, die jeweils eine PAM-Stelle einer Nukleinsäure-gesteuerten Nuklease umfassen;

   b. Hybridisieren erster Primer an die PAM-Stellen der Zielnukleinsäuren, wobei die ersten Primer (i) eine MAP-Stelle, die komplementär zu der PAM-Stelle ist, (ii) eine komplementäre Erkennungsstelle, die komplementär zu einer Erkennungsstelle der Nukleinsäure-gesteuerten Nuklease ist, und (iii) eine komplementäre Promotorstelle, die komplementär zu einer Promotorstelle ist, umfassen

   c. Verlängern der ersten Primer unter Verwendung der Zielnukleinsäuren als Matrize, wodurch erste Verlängerungsprodukte erzeugt werden, die eine Sequenz des ersten Primers und eine zu den Zielnukleinsäuren komplementäre Sequenz umfassen;

   d. Hybridisieren zweiter Primer mit den ersten Verlängerungsprodukten; und

   e. Verlängern der zweiten Primer unter Verwendung der ersten Verlängerungsprodukte als Matrize, wodurch zweite Verlängerungsprodukte erzeugt werden, die die PAM-Stelle, die Erkennungsstelle und die Promotorstelle umfassen.

2. Verfahren nach Anspruch 1, wobei die zweiten Primer (i) eine PAM-Stelle der Nukleinsäure-gesteuerten Nuklease und (ii) eine zufällige Sequenz umfassen, wobei die zufällige Sequenz optional zwischen etwa 6 und etwa 8 Basen lang ist.

3. Verfahren nach Anspruch 1, wobei die ersten Primer ferner eine Restriktionsenzymstelle eines Restriktionsenzyms umfassen.

4. Verfahren nach Anspruch 1, ferner umfassend:

   f. Ligieren eines Adapters an die zweiten Verlängerungsprodukte, wobei der Adapter eine Restriktionsenzymstelle eines Restriktionsenzyms umfasst; und

   g. Schneiden der zweiten Verlängerungsprodukte mit dem Restriktionsenzym, so dass die PAM-Stelle und die Restriktionsstelle von der Erkennungsstelle abgespalten werden.

5. Verfahren nach Anspruch 3 oder Anspruch 4, wobei das Restriktionsenzym Mmel, Fokl oder Mlyl umfasst.

6. Verfahren nach Anspruch 1, ferner umfassend das Entfernen ungebundener erster Primer oder ungebundener zweiter Primer.

7. Verfahren nach Anspruch 1, wobei die Verlängerung der ersten Primer oder die Verlängerung der zweiten Primer mit markierten Nukleotiden durchgeführt wird, wobei die markierten Nukleotide optional biotinylierte Nukleotide umfassen.

8. Verfahren nach Anspruch 1, wobei die Erkennungsstelle eine Länge von etwa 20 Nukleotiden oder eine Länge von etwa 15 bis etwa 25 Nukleotiden aufweist.

9. Verfahren nach Anspruch 1, wobei die Nukleinsäure-gesteuerte Nuklease ein Cas-Systemprotein umfasst, wobei die Nukleinsäure-gesteuerte Nuklease optional ein Cas9-Systemprotein umfasst.

10. Verfahren nach Anspruch 1, wobei die Zielnukleinsäuren genomische DNA, cDNA, menschliche DNA, Wirts-DNA oder eukaryotische DNA umfassen.

11. Verfahren nach Anspruch 1, wobei die komplementäre Erkennungsstelle mindestens eine modifizierte Nukleinsäurebindung umfasst, wobei die modifizierte Nukleinsäurebindung optional aus der Gruppe ausgewählt ist, die aus geschlossener Nukleinsäure (LNA), verbrückter Nukleinsäure (BNA), Peptidnukleinsäure (PNA), Zip-Nukleinsäure (ZNA), Glykolnukleinsäure (GNA), Threose-Nukleinsäure (TNA) und Phosphorothioat (PTO) besteht.

12. Verfahren nach Anspruch 1, ferner umfassend das Transkribieren der zweiten Verlängerungsprodukte unter Verwendung der Promotorstelle.

**13.** Verfahren nach einem der Ansprüche 1 bis 12, wobei:

> a) die PAM-Stelle NGG oder NAG umfasst;
> b) die Sammlung von Nukleinsäuren mindestens $10^5$ einzigartige Nukleinsäuren umfasst; und/oder
> c) die Zielnukleinsäuren mindestens alle 10.000 bp über ein Genom von Interesse verteilt sind.

**14.** Verfahren nach einem der Ansprüche 1-13, wobei die Zielnukleinsäuren eine oder mehrere der folgenden umfassen: ribosomale DNA, zentromerische DNA, Alu-DNA, DNA mit langen durchsetzten Kernelementen (LINE) und DNA mit kurzen durchsetzten Kernelementen (SINE).

**Revendications**

**1.** Procédé de fabrication d'une collection d'acides nucléiques, comprenant :

> a. l'obtention d'acides nucléiques cibles, comprenant chacun un site PAM d'une nucléase guidée par acide nucléique ;
> b. l'hybridation de premières amorces aux sites PAM des acides nucléiques cibles, dans lequel les premières amorces comprennent (i) un site MAP qui est complémentaire du site PAM, (ii) un site de reconnaissance complémentaire qui est complémentaire d'un site de reconnaissance de la nucléase guidée par acide nucléique, et (iii) un site promoteur complémentaire qui est complémentaire d'un site promoteur ;
> c. l'extension des premières amorces en utilisant les acides nucléiques cibles comme modèles, produisant ainsi des premiers produits d'extension comprenant une séquence de la première amorce et une séquence complémentaire des acides nucléiques cibles ;
> d. l'hybridation de secondes amorces aux premiers produits d'extension ; et
> e. l'extension des secondes amorces en utilisant les premiers produits d'extension comme modèles, produisant ainsi des seconds produits d'extension comprenant le site PAM, le site de reconnaissance et le site promoteur.

**2.** Procédé selon la revendication 1, dans lequel les secondes amorces comprennent (i) un site PAM de la nucléase guidée par acide nucléique et (ii) une séquence aléatoire, facultativement dans lequel la séquence aléatoire est longue d'entre environ 6 et environ 8 bases.

**3.** Procédé selon la revendication 1, dans lequel les premières amorces comprennent en outre un site d'enzyme de restriction d'une enzyme de restriction.

**4.** Procédé selon la revendication 1, comprenant en outre :

> f. la ligature d'un adaptateur aux seconds produits d'extension, dans lequel l'adaptateur comprend un site d'enzyme de restriction d'une enzyme de restriction ; et
> g. la découpe des seconds produits d'extension avec l'enzyme de restriction de sorte que le site PAM et le site de restriction soient clivés à partir du site de reconnaissance.

**5.** Procédé selon la revendication 3 ou la revendication 4, dans lequel l'enzyme de restriction comprend MmeI, FokI ou MlyI.

**6.** Procédé selon la revendication 1, comprenant en outre l'élimination de premières amorces non liées ou de secondes amorces non liées.

**7.** Procédé selon la revendication 1, dans lequel l'extension des premières amorces ou l'extension des secondes amorces est réalisée avec des nucléotides marqués, facultativement dans lequel les nucléotides marqués comprennent des nucléotides biotinylés.

**8.** Procédé selon la revendication 1, dans lequel le site de reconnaissance a une longueur d'environ 20 nucléotides ou une longueur d'environ 15 à environ 25 nucléotides.

**9.** Procédé selon la revendication 1, dans lequel la nucléase guidée par acide nucléique comprend une protéine de système Cas, facultativement dans lequel la nucléase guidée par acide nucléique comprend une protéine de système Cas9.

**10.** Procédé selon la revendication 1, dans lequel les acides nucléiques cibles comprennent l'ADN génomique, l'ADNc, l'ADN humain, l'ADN hôte ou l'ADN eucaryote.

**11.** Procédé selon la revendication 1, dans lequel le site de reconnaissance complémentaire comprend au moins une liaison d'acide nucléique modifiée, facultativement dans lequel la liaison d'acide nucléique modifiée est choisie dans le groupe consistant en un acide nucléique verrouillé (LNA), un acide nucléique ponté (BNA), un acide peptidonucléique (APN), un acide nucléique zip (ZNA), un acide glycol nucléique (ANG), un acide thréose nucléique (ANT) et un phosphorothioate (PTO).

**12.** Procédé selon la revendication 1, comprenant en outre la transcription des seconds produits d'extension en utilisant le site promoteur.

**13.** Procédé selon quelconque des revendications 1 à 12, dans lequel :

    a) le site PAM comprend NGG ou NAG ;
    b) la collection d'acides nucléiques comprend au moins $10^5$ acides nucléiques uniques ; et/ou
    c) les acides nucléiques cibles sont espacés au moins toutes les 10 000 pb dans un génome d'intérêt.

**14.** Procédé selon l'une quelconque des revendications 1 à 13, dans lequel les acides nucléiques cibles comprennent un ou plusieurs parmi l'ADN ribosomique, l'ADN centromérique, l'ADN Alu, l'ADN d'éléments nucléaires dispersés longs (LINE), et l'ADN d'éléments nucléaires dispersés courts (SINE).

Fragmented and blunt
end repaired genomic
or other source DNA

101

gRNA targeting
sequence
HGG
DCC

Ligate T7 promoter
adapters to obtain T7
library of DNA

102

T7          HGG          T7
            DCC

Nt.CviPII nicks DNA at
CCD site

103

HGG
DCC
▲ Nick

T7 Endonuclease I
cuts opposite strand
at 1, 2, or 3 bp 5' of
the nick, generating
double strand break

104

OR

HGG
DCC
▲

OR

HGG
DCC
▲

HGG
DCC
▲

Blunt end with T4 DNA
Polymerase

105

HGG
DCC

Ligate MlyI adapter

106

HGG
DCC   MlyI

Digesting with MlyI
eliminates HGG motif

107

HGG
DCC   MlyI

Ligate gRNA stem-
loop sequences

108

gRNA stem-loop sequences

FIG. 1

**FIG. 2**

**FIG. 3**

301    DNA source nicked with CviPII

N20 (guide RNA sequence)

OR

When nicks are proximal, a double stranded break occurs and leads to 5' or 3' overhangs

302

T4 DNA polymerase repairs 5' overhangs by synthesizing the complementary strand; or repairs 3' overhangs by removing them. Both result in a blunt end including the N20 guide RNA sequence and the HGG

303

EP 3 635 114 B1

401 | Mlyl | CCD GGH ======= HGG DCC | Mlyl |

Ligate Mlyl adapters and
digest to remove HGG

402 | Mlyl | CCD GGH ======= HGG DCC | Mlyl |

Ligate 2nd adapter

403 | BsaXl Mmel | ======= | Mmel BsaXl |

Digest with Mmel, cuts 20
nt from site to include N20

404 | BsaXl Mmel | ======= | Mmel BsaXl |

Ligate T7 promoter
adapter

405 | BsaXl Mmel | ===< T7 | T7 >=== | Mmel BsaXl |

Digest with BsaXl to
remove 2nd adapter

406 | BsaXl Mmel | ===< T7 | T7 >=== | Mmel BsaXl |

Ligate guide RNA stem-
loop adapter

407 < Guide RNA stem-loop sequences ===< T7 | T7 >=== Guide RNA stem-loop sequences >

**FIG. 4**

501 | FokI MmeI | CCD / GGH ▬▬▬▬▬▬▬ | HGG / DCC | MmeI FokI

Ligate adapter and cut with
MmeI cuts 20 nt from site
to include N20 region

502 | FokI MmeI | CCD / GGH ▬▬▬▬▬▬▬ | HGG / DCC | MmeI FokI

Ligate T7 promoter
adapter

503 | FokI MmeI | CCD / GGH ▬▬ < T7 | T7 > ▬▬ HGG / DCC | MmeI FokI

Digest with FokI, removes HGG,
leaving a 5 nt 3' overhang

504 | FokI MmeI | CCD / GGH ▬▬ < T7 | T7 > ▬▬ HGG / DCC | MmeI FokI

Use single stranded
ligation to ligate guide
RNA stem-loop sequence

505 | ◁ Guide RNA stem-loop sequences ▬▬ < T7 | T7 > ▬▬ Guide RNA stem-loop sequences ▷

**FIG. 5**

**601** DNA is nicked by CviPII

**602** Bst large fragment DNA polymerase synthesizes a new DNA strand starting from the nick, displacing old strand

**603** Because of DNA synthesis, nick has been sealed and is available for CviPII to re-nick

**604** Bst large fragment DNA polymerase synthesizes another copy of the DNA; **cycle yields large amounts of target sequences**

**605**

**606** Convert to double stranded DNA by random priming and extension

Regeneration of CviPII site and subsequent extension by polymerase continues cycling

**FIG. 6**

EP 3 635 114 B1

701

CCD

CCD
GGH

703

702

704

Fokl    +    CCD
             GGH

702

705

Fokl CCD
     GGH

702

706

702

707

Guide RNA stem-loop sequences

702

**FIG. 7**

**801**

**802**

CCDN$_{(17)}$+NNN-Rest-T7

GGH

**803**

**804**

CCDN$_{(17)}$+NNN-Rest-T7

GGH

**805**

CCDN$_{(17)}$+NNN-Rest-T7

N(6-8)GGH

**806**          **807**

**808**

**FIG. 8A**

FIG. 8B

**FIG. 8C**

FIG. 8D

**FIG. 9A**

Continued from FIG. 9A

T7 prom

HGG

*Comp stem loop*

910

913

T7 prom

HGG

914

*Comp stem loop*

915

T7 prom

HGG

916

*Comp stem loop*

917

T7 prom

918

**FIG. 9B**

**Starting point: "n" libraries, "m" reference sequences**

Library 1 → Sequence 1..m Counts 1 → Ranked Sequence Counts 1..m

Library 2 → Sequence 1..m Counts 2 → Ranked Sequence Counts 1..m

. . .

Library n → Sequence 1..m Counts n → Ranked Sequence Counts 1..m

**Most abundant**
Median Rank Sequence 1
Median Rank Sequence 2
Median Rank Sequence 3
. . .
Median Rank Sequence m
**Least abundant**

→ Ranked gNA bed

BAM file 1 / BAM file 2 . . . BAM file n → merge → Merged BAM file

filter by coverage → Filtered BAM file → extract gNAs → gNA bed → Space gNAs → Spaced gNA bed → Ranked gNA bed

**FIG. 10**

EP 3 635 114 B1

1st iteration

find most frequent guide, digest

remove short frag

2nd iteration

find most frequent guide, digest

remove short frag

**FIG. 11**

human

Non-human

Cut with Cas9 nickase,
gRNAs target human

Cas9

Nick translate with biotinylated NT

Pull out biotinylated DNA with
streptavidin beads; analyze
remaining DNA (e.g., by sequencing)

**FIG. 12**

RNA fragment

1301 ——

RNA polyadenylation
1302 ↓ and annealing of read 2 adapter
tailed first strand RT primer

AAAAAAA —— 1303

TTTTTTTT – adapter 2 — 1304

1305 ↓ first strand cDNA synthesis
and strand switch reaction

AAAAAAA

1307 — CCC      TTTTTTTT – adapter 2

1306

1308 ↓ annealing of strand switch oligo
tailed with read 1 adapter sequence

1309

adapter 1 – GGG      AAAAAAA

CCC      TTTTTTTT – adapter 2

1310 ↓ indexing and amplification of double
stranded cDNA sequencing library

adapter 1 – GGG      AAAAAAA

CCC      TTTTTTTT – adapter 2

**FIG. 13**

**FIG. 14**

Final template for in vitro transcription of the crRNA N20 unit

**FIG. 16A**

1601

N20 (guide RNA sequence)

TTN
AAN

- Abasic site
I Inosine

Replace Adenines with Inosines

1602

N20 (guide RNA sequence)

TTN
IIN

hAAG removes Inosines base paired with
Thymines, leaving abasic sites

TTN

N20 (guide RNA sequence)

1603

--N

Abasic sites cannot base pair cause mismatches,
which are recognized and cut by T7 Endonuclease I

▼TTN

N20 (guide RNA sequence)

1604

--N▲

TTN

N20 (guide RNA sequence)

--N

1605

**FIG. 16B**

DNA source cut with either:

—————————
—————————--N

TTN————N20 (guide RNA sequence)——— 1605

Ligate adapters with AAN overhang; will not ligate to abasic sites or other mismatches

—————————
—————————--N

| FokI MmeI | TTN——N20 (guide RNA sequence)———
|           | AAN——————————————— 1606

Mmel cuts 20 nt from site to include N20 region; FokI cuts away the adapter

| FokI N MmeI | ————————————— 1607

Ligate final adapter containing T7 promoter and crRNA sequence

T7 > crRNA > ————————— 1608

Final template for in vitro transcription of the crRNA N20 unit

**FIG. 17A**

N20 (guide RNA sequence)

1705

Ligate adapter; will not ligate to 3'phosphate

N20 (guide RNA sequence)

1706

FokI Mmel

Mmel cuts 20 nt from site to include N20 region;
FokI cuts away the adapter

1707

FokI N Mmel

Ligate final adapter containing T7
promoter and crRNA sequence

1708

T7  crRNA

Final template for in vitro transcription of the crRNA N20 unit

**FIG. 17B**

FIG. 18

1801          N20 (guide RNA sequence)

TTN
AAN

Randomly fragment DNA with non-specific nickase
and T7 endonuclease I (fragmentase)

1802          N20 (guide RNA sequence)

TTN
AAN

TTN
AAN

1 in 16 fragmentation sites will overlap perfectly with the TTN
PAM and can thus be ligated to an adapter with a AAN overhang

N20 (guide RNA sequence)

FokI Mmel TTN
AAN 1803

Mmel cuts 20 nt from site to include N20 region;
FokI cuts away the adapter

FokI N Mmel 1804

Ligate final adapter containing T7
promoter and crRNA sequence

T7 crRNA 1805

Final template for in vitro transcription of the crRNA N20 unit

EP 3 635 114 B1

Start with randomly sheared DNA; 1 in 16 fragments will have a 5' PAM end; methylate using DNA methylase (such as EcoGII DNA methyltransferase); end repair to blunt ends

Ligate NtBstNBI*cPAM adapter; * denotes cleavage resistant phosphorothicate bond

NtBstNBI nicks the top strand 4 bp away

Addition of cPAM from adapter to PAM from insert created a restriction site (see table 7) site; restriction enzyme cuts bottom strand. Internal sites are not used because of methylation

Nick from NtBstNBI and restriction enzyme leave a blunt end next to the N20; ligate blunt FokIMmeI adapter

N20 (guide RNA sequence)

PAM
cPAM

1901

NtBstNBI *cPAMPAM PAMcPAM

N20 (guide RNA sequence)

1902

NtBstNBI *cPAMPAM PAMcPAM

1903

NtBstNBI *cPAMPAM PAMcPAM

N20 (guide RNA sequence)

1904

NtBstNBI *cPAMPAM PAMcPAM

N20 (guide RNA sequence)

1905

FokI MmeI

1906

PAM: Protospacer Adjacent Motif
cPAM: Complement of PAM sequence

FIG. 19A

FIG. 19B

Final template for in vitro transcription of the crRNA N20 unit

EP 3 635 114 B1

Randomly sheared DNA, repaired to blunt ends; 1 in 16 fragments will have a 5' PAM end

N20 (guide RNA sequence)

PAM
cPAM ———————— 2001

Ligate NtBstNBIAA adapter

NtBstNBI cPAMPAM
PAMcPAM

N20 (guide RNA sequence)

2002

NtBstNBI nicks the top strand 4 bp away

NtBstNBI cPAMPAM
PAMcPAM

2003

NtBstNBI cPAMPAM
PAMcPAM

2004

Exonuclease 3 recognizes the nick degrades top strand in the 3'>5' direction exposing bottom strand

NtBstNBI PAMcPAM

2005

MlyI primer is added, anneals precisely to the bottom strand and PAMcPAM sequences; high temperature ligase seals the nick

MlyI cPAMPAM
NtBstNBI PAMcPAM

2006

PAM: Protospacer Adjacent Motif
cPAM: Complement of PAM sequence

Continue to figure 20C

81

FIG. 20B

Randomly sheared DNA, repaired to blunt ends; 1 in 16 fragments will have a 5' PAM end

N20 (guide RNA sequence)

PAM
cPAM                                    2001

Ligate NtBstNBIAA adapter

N20 (guide RNA sequence)

NtBstNBI   cPAMPAM
           PAMcPAM                      2002

NtBstNBI nicks the top strand 4 bp away

NtBstNBI   cPAMPAM
           PAMcPAM                      2003

NtBstNBI   cPAMPAM
           PAMcPAM                      2004

Exonuclease 3 recognizes the nick degrades top strand in the 3'>5' direction exposing bottom strand

NtBstNBI   PAMcPAM                      2005

MlyI primer is added, anneals precisely to the bottom strand and cPAMPAM sequences allowing specific PCR amplification

MlyI       cPAMPAM
NtBstNBI   PAMcPAM                      2006

Continue to figure 20C

PAM: Protospacer Adjacent Motif
cPAM: Complement of PAM sequence

EP 3 635 114 B1

**FIG. 20C**

FIG. 21A

Cut with MlyI to remove adapter

MlyI Ad ▰▰▰▰ ▬▬▬▬ MlyI Ad    2105

Ligate FokI Mmel adapter

N20 (guide RNA sequence)

FokI  Mmel ▰▰▰▰ ▬▬▬▬ Mmel FokI    2106

Mmel cuts 20 nt from site to include N20 region;
FokI cuts away the adapter

FokI  Mmel ▰▰▰▰ ▬▬▬▬ Mmel FokI    2107

Ligate final adapter containing T7
promoter and crRNA sequence

T7 > crRNA > ▰▰▰▰    2108

Final template for in vitro transcription of the crRNA N20 unit

**FIG. 21B**

EP 3 635 114 B1

**FIG. 22A**

Randomly sheared DNA, repaired to blunt ends; 1
in 16 fragments will have a 5' TT end

N20 (guide RNA sequence)

TTN
AAN
2201

Ligate NtBstNB1AA adapter

N20 (guide RNA sequence)

NtBstNB1 | AATTN
TTAAN
2202

Addition of AA from adapter to TT
from insert created a MluC1 site

NtBstNB1 | AATTN
TTAAN
2203

MluC1 cuts DNA

NtBstNB1 | TTAA

AATTN
N
2204

Mung bean nuclease degrades single
stranded extensions, leaving blunt ends

NtBstNB1

N
N
2205

Ligate blunt Fok1 Mme1 adapter

FokI MmeI | N
N
2206

FIG. 22B

Randomly sheared DNA, repaired to blunt ends; 1 in 16 fragments will have a 5' TT end

N20 (guide RNA sequence)

TTN
AAN

2201

Ligate NtBstNB1AA adapter

N20 (guide RNA sequence)

NtBstNB1 AATTN
TTAAN

2202

NtBstNB1 nicks the top strand 4 bp away

NtBstNB1 AATTN
TTAAN

2207

Addition of AA from adapter to TT from insert created a MluC1 site; MluC1 cuts top and bottom strand

NtBstNB1 AATT N
TTAAN

2208

Nick from NtBstNB1 and cut from MluC1 leave a blunt end next to the N20; ligate blunt Fok1Mme1 adapter

AATT

NtBstNB1 TTAA

N20 (guide RNA sequence)

N
N

2209

FokI MmeI N
N

2210

EP 3 635 114 B1

Randomly sheared DNA, repaired to blunt ends; 1
in 16 fragments will have a 5′ TT end

TTN
AAN ══════════════ **2201**

N20 (guide RNA sequence)

Ligate NtBstNB1*AA adapter; * denotes
cleavage resistant phosphorothioate bond

| NtBstNB1 |*AATTN
              TTAAN ══════════════ **2211**

N20 (guide RNA sequence)

NtBstNB1 nicks the top strand 4 bp away

| NtBstNB1 |*AATTN
              TTAAN ══════════════ **2212**

Addition of AA from adapter
to TT from insert created a
MluC1 site; MluC1 cuts
bottom strand

| NtBstNB1 |*AATT N
              TTAAN ══════════════ **2213**

Nick from NtBstNB1 and MluC1 leave
a blunt end next to the N20; ligate
blunt Fok1Mme1 adapter

| NtBstNB1 |*AATT
              TTAA

N
N ══════════════ **2214**

N20 (guide RNA sequence)

| FokI MmeI |N
              N ══════════════ **2215**

FIG. 22C

2206, 2210 and 2215

2216

2217

FokI MmeI

Mmel cuts 20 nt from site to include N20 region;
Fokl cuts away the adapter

FokI N MmeI

Ligate final adapter containing T7 promoter and crRNA sequence

T7 crRNA

Final template for in vitro transcription of the crRNA N20 unit

**FIG. 22D**

FIG. 23A

Randomly sheared DNA, repaired to blunt ends; 1
in 16 fragments will have a 5' TT end

N20 (guide RNA sequence)

TTN
AAN
2301

Ligate NtBstNB1AA adapter

N20 (guide RNA sequence)

NtBstNB1 | AATTN
TTAAN
2302

NtBstNB1 nicks the top strand 4 bp away

NtBstNB1 | AATTN
TTAAN
2303

NtBstNB1 | AATT N
TTAAN
2304

Exonuclease 3 recognizes the nick
degrades top strand in the 3'>5'
direction exposing bottom strand

N
NtBstNB1 TTAAN
2305

Mly1 primer is added, anneals
precisely to the bottom strand
and AATT sequences allowing
PCR amplification

Mly1 | AATTN
NtBstNB1 TTAAN
2306

FIG. 23B

EP 3 635 114 B1

Restored Mly1 adapter allows PCR amplification of TT-containing sequences only — 2306

Mly1 cuts both strand 5 bp away leaving a blunt end — 2307

Blunt Fok1 Mme1 adapter is ligated to the blunt end — 2308

Mme1 cuts 20 nt from site to include N20 region; Fok1 cuts away the adapter — 2309 — N20 (guide RNA sequence)

Ligate final adapter containing T7 promoter and crRNA sequence — 2310

Final template for in vitro transcription of the crRNA N20 unit — 2311 — T7 — crRNA

**FIG. 23C**

92

**FIG. 24**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62516619
- US 62548036
- WO 2017081097 A
- WO 2009133466 A
- WO 2017031360 A
- US 5948902 A
- US 20050233340 A

**Non-patent literature cited in the description**

- **AUSUBEL et al.** Short Protocols in Molecular Biology. Wiley & Sons, 1995
- **SAMBROOK et al.** Molecular Cloning: A Laboratory Manual. Cold Spring Harbor, 2001
- **F.A. RAN ; L. CONG ; W.X. YAN ; D. A. SCOTT ; J.S. GOOTENBERG ; A.J. KRIZ ; B. ZETSCHE ; O. SHALEM ; X. WU ; K.S. MAKAROVA.** In vivo genome editing using Staphylococcus aureus Cas9. *Nature,* 09 April 2015, vol. 520, 186-191